(19) Europäisches Patentamt
European Patent Office
Office européen des brevets

(11) EP 4 130 006 A1

(12) # EUROPEAN PATENT APPLICATION
published in accordance with Art. 153(4) EPC

(43) Date of publication:
**08.02.2023 Bulletin 2023/06**

(21) Application number: **21775717.8**

(22) Date of filing: **25.03.2021**

(51) International Patent Classification (IPC):
***C07D 491/22*** *(1974.07)* ***A61K 31/506*** *(2000.01)*
***A61K 47/68*** *(2017.01)* ***A61K 39/395*** *(1980.01)*
***A61P 35/00*** *(2000.01)*

(52) Cooperative Patent Classification (CPC):
**A61K 31/506; A61K 39/395; A61K 47/68; A61P 35/00; C07D 491/22**

(86) International application number:
**PCT/CN2021/082857**

(87) International publication number:
**WO 2021/190583 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 CN 202010218297**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YING, Hua**
  **Shanghai 200245 (CN)**
- **ZHANG, Xiaomin**
  **Shanghai 200245 (CN)**
- **YANG, Xiaoying**
  **Shanghai 200245 (CN)**
- **TAO, Weikang**
  **Shanghai 200245 (CN)**

(74) Representative: **Sonzogni, Laura Gabriella et al**
**Dragotti & Associati S.r.l.**
**Via Nino Bixio, 7**
**20129 Milano (IT)**

Remarks:
The complete document including Reference Table(s) and the Sequence Listing(s) can be downloaded from the EPO website

(54) **ANTI-PSMA ANTIBODY-EXATECAN ANALOGUE CONJUGATE AND MEDICAL USE THEREOF**

(57) Provided are an anti-PSMA antibody-Exatecan analogue conjugate and medical use thereof. Specifically, provided is an anti-PSMA antibody-drug conjugate represented by general formula (Pc-L-Y-D), wherein Pc is an anti-PSMA antibody or an antigen-binding fragment thereof.

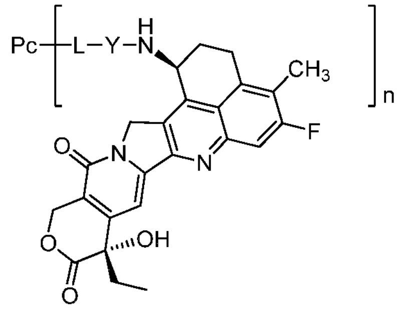

(Pc-L-Y-D)

EP 4 130 006 A1

## Description

The present application claims priority to Chinese Patent Application (Application No. 202010218297.4) filed on March 25, 2020.

## TECHNICAL FIELD

The present disclosure relates to an anti-PSMA antibody and an anti-PSMA antibody-exatecan analog conjugate, a preparation method for the same, a pharmaceutical composition comprising the same, and use of the same in preparing a medicament for the treatment of a PSMA-mediated disease or disorder, particularly in preparing an anticancer medicament.

## BACKGROUND

The statement herein merely provides background information related to the present disclosure and may not necessarily constitute the prior art.

Statistics from the American Cancer Society in 2019 showed that prostate cancer ranked first among new cancer cases in men in the United States, with 174,650 cases (CA CANCER J CLIN 2019; 69: 7-34). For clinical local diseases, surgery and radiotherapy are usually the options. For locally advanced or metastatic diseases, surgical or chemical androgen deprivation therapy (ADT) is usually the first treatment. Sipuleucel-T cell immunotherapy can be selected for the early stage of castration-resistant prostate cancer (CRPC), and drug therapies such as androgen inhibitors, androgen receptor antagonists, radiotherapy drugs for bone metastasis, and chemotherapy drugs acting on microtubules can be selected for the patients with metastatic castration-resistant prostate cancer (mCRPC) as appropriate. However, each of the therapies can only prolong the survival by a few months, so it is necessary to seek effective therapies (European Urology, 66 (6); 1190-1193; Journal of Nuclear Medicine, 59 (2); 177-182).

In addition to prostate epithelial cells, prostate specific membrane antigen (PSMA) can also be expressed by non-prostate tissues, such as the small intestine, proximal renal tubules and salivary glands, but at levels much lower than in the prostate tissue. PSMA is highly expressed in prostate cancer cells, particularly in metastatic diseases, hormone refractory diseases and high-grade lesions. In addition, PSMA is also highly expressed in endothelial cells of neovasculature of all solid tumors, but not in normal vasculature, so it is a target for the treatment of solid tumors (Clinical Cancer Research, Vol. 3, 81-85, January 1997; Urologic Oncology: Seminars and Original Investigations, 1(1), 18-28.; Clin Cancer Res., 2010 November 15; 16(22): 5414-5423). The androgen deprivation therapy and androgen receptor antagonist therapy can both upregulate the expression of prostate specific membrane antigen (PSMA) (J Nucl Med, 2017; 58: 81-84), which provides the basis for targeted therapy in combination with traditional hormone therapy.

PSMA belongs to glutamate carboxypeptidase II (GCPII), which acts as NAALDase in the nervous system to hydrolyze NAAG to obtain glutamate, and is involved in the neurotransmission of glutamate. It acts as FOLH1 in the small intestine to decompose folyl-poly-$\gamma$-glutamate (FPGn) to obtain folic acid, and is involved in the metabolism of folic acid (Curr Med Chem., 2012; 19(6): 856-870.). Moreover, it acts as PSMA in the prostate, expressed on epithelial cells, and is involved in the development of prostate cancer. PSMA consists of 750 amino acids, including 19 intracellular amino acids, 24 transmembrane amino acids, and 707 extracellular amino acids. The crystallization results showed that the extracellular part consisted of 3 domains, namely the protease-like domain, the apical domain and the C-terminal domain. Those three domains all participate in the binding to substrates, with the first two directly binding to the substrate and the C-terminal domain playing a role in dimerizing PSMA (The EMBO Journal (2006) 25, 1375-1384). Glutamate carboxypeptidase II (GCPII) and its splice variants and paralogs have been studied only to a limited extent. The splice variants, represented by PSM', are mostly present intracellularly in normal prostate tissue cells. The most deeply studied GCPII homolog GCPIII or NAALADase II, as an effective drug target by itself, can compensate for the lack of normal GCPII enzymatic activity, and it has 68% sequence similarity with GCPII (Current Medicinal Chemistry, 2012, 19, 1316-1322; Frontiers in Bioscience, Landmark, 24, 648-687, March 1, 2019).

Antibody-drug conjugate (ADC) links a monoclonal antibody or an antibody fragment to a biologically active cytotoxin via a linker compound, making full use of the binding specificity of the antibody to surface antigens of normal cells and tumor cells and the high-efficiency of the cytotoxic substance, and also avoiding defects such as poor therapeutic effect of the antibody and serious toxic side effects of the toxic substance. This means that the antibody-drug conjugate can kill tumor cells more precisely and has a reduced effect on normal cells compared to conventional chemotherapeutic drugs in the past.

A variety of ADC drugs have been used in clinical or clinical studies, such as Kadcyla, which is an ADC drug formed by Her2-targeted Trastuzumab and DM1. Meanwhile, there are also PSMA-targeted ADC drugs for clinical therapeutic studies. PSMA-ADC from Cytogen was in phase II clinical stage, and MEDI-3726 from MedImmune and MLN-2704 from BZL Biologics Inc. were discontinued at the end of phase I clinical stage due to poor efficacy. The

development of new ADC drugs by adopting different strategies has wide prospects.

## SUMMARY

The present disclosure relates to an anti-PSMA antibody-ADC and use thereof, and provides an ADC drug in which an anti-PSMA antibody or an antigen-binding fragment is conjugated with an exatecan analog, a cytotoxic substance.

The present disclosure provides an antibody-drug conjugate of general formula (Pc-L-Y-D) or a pharmaceutically acceptable salt thereof:

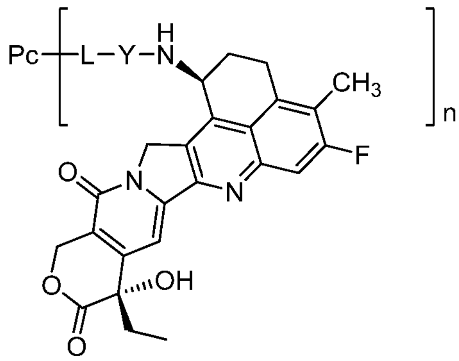

(Pc-L-Y-D)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)- and -S-$(CR^aR^b)_m$-$CR^1R^2$-C(O)-;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with carbon atoms connected thereto, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;

or, $R^a$ and $R^2$, together with carbon atoms connected thereto, form cycloalkyl or heterocyclyl;

m is an integer from 0 to 4; for example, a non-limiting example is that m is selected from the group consisting of 0, 1, 2, 3 and 4;

n is a decimal or an integer from 1 to 10; preferably, n is from 1 to 8, more preferably from 3 to 8, even more preferably from 3 to 7, and most preferably from 6 to 7;

L is a linker unit;

Pc is an anti-PSMA antibody or an antigen-binding fragment thereof, wherein preferably the anti-PSMA antibody or the antigen-binding fragment thereof specifically binds to the extracellular domain of PSMA.

In some embodiments, in the antibody-drug conjugate of general formula (Pc-L-Y-D) or the pharmaceutically acceptable salt thereof as described above, the anti-PSMA antibody or the antigen-binding fragment thereof comprises a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 1, and the light chain variable region comprises an LCDR1, an LCDR2 and an LCDR3 having sequences identical to those of an LCDR1, an LCDR2 and an LCDR3 of a light chain variable region set forth in SEQ ID NO: 2.

In some embodiments, in the antibody-drug conjugate of general formula (Pc-L-Y-D) or the pharmaceutically acceptable salt thereof as described above, the anti-PSMA antibody or the antigen-binding fragment thereof comprises a heavy chain variable region and a light chain variable region, wherein the heavy chain variable region comprises an HCDR1, an HCDR2 and an HCDR3 set forth in SEQ ID NO: 3, SEQ ID NO: 4 and SEQ ID NO: 5, respectively, and the light chain variable region comprises an LCDR1, an LCDR2 and an LCDR3 set forth in SEQ ID NO: 6, SEQ ID NO: 7 and SEQ ID NO: 8, respectively.

In some embodiments, in the antibody-drug conjugate of general formula (Pc-L-Y-D) or the pharmaceutically acceptable salt thereof

acceptable salt thereof as described above, the anti-PSMA antibody is a murine antibody, a chimeric antibody, a humanized antibody or a human antibody.

In some embodiments, in the antibody-drug conjugate of general formula (Pc-L-Y-D) or the pharmaceutically acceptable salt thereof as described above, the anti-PSMA antibody or the antigen-binding fragment thereof comprises a heavy chain variable region and a light chain variable region, wherein the heavy chain variable region has an amino acid sequence set forth in SEQ ID NO: 1 or having at least 90%-100%, including but not limited to, at least 91%, at least 92%, at least 93%, at least 94%, at least 95%, at least 96%, at least 97%, at least 98%, at least 99% or at least 100%, sequence identity thereto; and the light chain variable region has an amino acid sequence set forth in SEQ ID NO: 2 or having at least 90%-100%, including but not limited to, at least 91%, at least 92%, at least 93%, at least 94%, at least 95%, at least 96%, at least 97%, at least 98%, at least 99% or at least 100%, sequence identity thereto.

In some embodiments, in the antibody-drug conjugate of general formula (Pc-L-Y-D) or the pharmaceutically acceptable salt thereof as described above, the anti-PSMA antibody or the antigen-binding fragment thereof comprises a heavy chain variable region set forth in SEQ ID NO: 1 and a light chain variable region set forth in SEQ ID NO: 2.

In some embodiments, in the antibody-drug conjugate of general formula (Pc-L-Y-D) or the pharmaceutically acceptable salt thereof as described above, the anti-PSMA antibody comprises a heavy chain constant region and a light chain constant region; preferably, the heavy chain constant region is selected from the group consisting of constant regions of human IgG1, IgG2, IgG3 and IgG4 and conventional variants thereof, and the light chain constant region is selected from the group consisting of constant regions of human antibody κ and λ chains and conventional variants thereof.

In some embodiments, in the antibody-drug conjugate of general formula (Pc-L-Y-D) or the pharmaceutically acceptable salt thereof as described above, the anti-PSMA antibody comprises a heavy chain set forth in SEQ ID NO: 9 and a light chain set forth in SEQ ID NO: 10.

In some embodiments, in the antibody-drug conjugate of general formula (Pc-L-Y-D) or the pharmaceutically acceptable salt thereof as described above, n is a decimal or an integer from 1 to 8, preferably from 3 to 8, and more preferably from 3 to 7.

In some embodiments, in the antibody-drug conjugate of general formula (Pc-L-Y-D) or the pharmaceutically acceptable salt thereof as described above,

Y is $-O-(CR^aR^b)_m-CR^1R^2-C(O)-$;
$R^a$ and $R^b$ are identical or different and are each independently selected from the group consisting of hydrogen, deuterium, halogen and $C_{1-6}$ alkyl;
$R^1$ is haloalkyl or $C_{3-6}$ cycloalkyl;
$R^2$ is selected from the group consisting of hydrogen, haloalkyl and $C_{3-6}$ cycloalkyl;
or, $R^1$ and $R^2$, together with carbon atoms connected thereto, form $C_{3-6}$ cycloalkyl;
m is 0 or 1.

In some embodiments, in the antibody-drug conjugate of general formula (Pc-L-Y-D) or the pharmaceutically acceptable salt thereof as described above, Y is selected from the group consisting of:

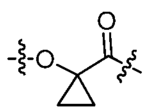, 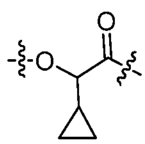, 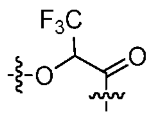, 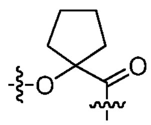, 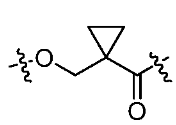, 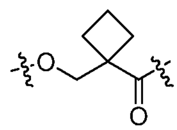

and

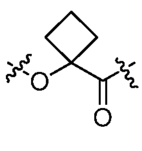;

wherein an O-terminus of Y is connected to the linker unit L.

In some embodiments, in the antibody-drug conjugate of general formula (Pc-L-Y-D) or the pharmaceutically acceptable salt thereof as described above, the linker unit -L- is $-L^1-L^2-L^3-L^4-$, wherein

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-cycloalkyl and linear heteroalkyl of

1 to 8 atoms, the heteroalkyl comprising 1 to 3 heteroatoms selected from the group consisting of N, O and S, wherein the $C_{1-8}$ alkyl, $C_{1-8}$ alkyl-cycloalkyl and linear heteroalkyl are each independently and optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids of phenylalanine (F), glycine (G), valine (V), lysine (K), citrulline, serine (S), glutamic acid (E) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$ and a chemical bond, wherein t is an integer from 1 to 6;

$R^3$, $R^4$ and $R^5$ are identical or different and are each independently selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments, in the antibody-drug conjugate of general formula (Pc-L-Y-D) or the pharmaceutically acceptable salt thereof as described above, the linker unit -L- is $-L^1-L^2-L^3-L^4-$, wherein

$L^1$ is

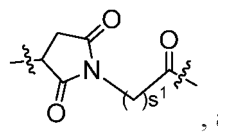

and $s^1$ is an integer from 2 to 8;

$L^2$ is a chemical bond;

$L^3$ is a tetrapeptide residue; preferably, $L^3$ is a tetrapeptide residue of GGFG (SEQ ID NO: 13) (glycine-glycine-phenylalanine-glycine);

$L^4$ is $-NR^5(CR^6R^7)t-$, wherein $R^5$, $R^6$ and $R^7$ are identical or different and are each independently hydrogen or alkyl, and t is 1 or 2;

wherein the $L^1$ terminus is connected to Pc, and the $L^4$ terminus is connected to Y.

In some embodiments, in the antibody-drug conjugate of general formula (Pc-L-Y-D) or the pharmaceutically acceptable salt thereof as described above, -L- is:

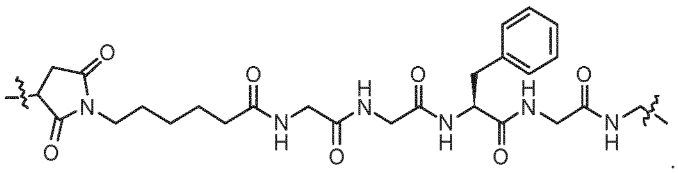

In some embodiments, in the antibody-drug conjugate of general formula (Pc-L-Y-D) or the pharmaceutically acceptable salt thereof as described above, -L-Y- is optionally selected from the group consisting of:

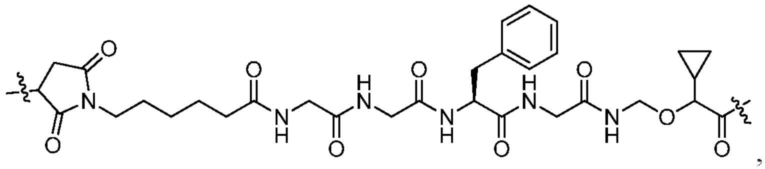

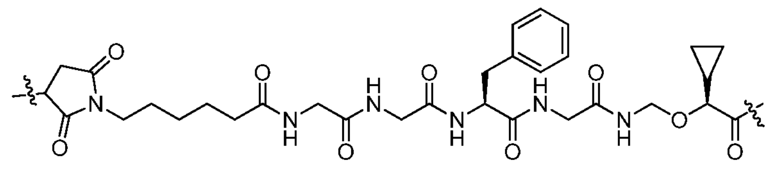

and

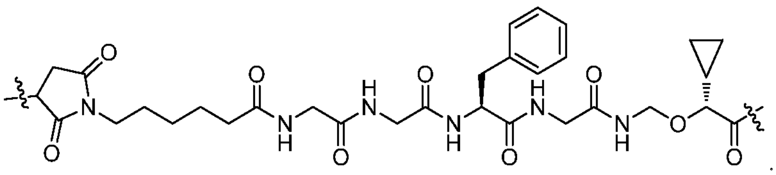

.

In some embodiments, the antibody-drug conjugate of general formula (Pc-L-Y-D) or the pharmaceutically acceptable salt thereof as described above is an antibody-drug conjugate of general formula (Pc-$L_a$-Y-D) or a pharmaceutically acceptable salt thereof:

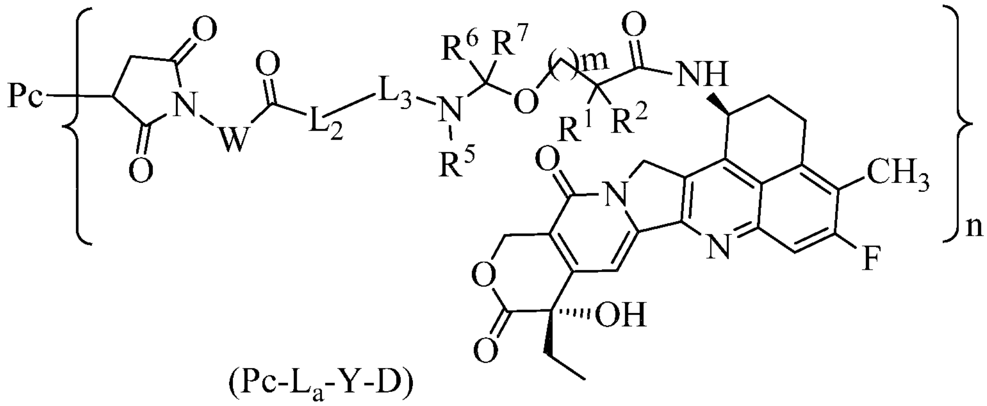

(Pc-$L_a$-Y-D)

wherein:

W, $L^2$, $L^3$, $R^5$, $R^6$ and $R^7$ are as defined in the linker unit -L- as described above;

Pc, n, $R^1$, $R^2$ and m are as defined in general formula (Pc-L-Y-D);

specifically, Pc is an anti-PSMA antibody or an antigen-binding fragment thereof;

m is an integer from 0 to 4; for example, a non-limiting example is that m is selected from the group consisting of 0, 1, 2, 3 and 4;

n is a decimal or an integer from 1 to 10; preferably, n is from 1 to 8, more preferably from 3 to 8, even more preferably from 3 to 7, and most preferably from 6 to 7;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;

W is selected from the group consisting of $C_{1-8}$ alkyl, $C_{1-8}$ alkyl-cycloalkyl and linear heteroalkyl of 1 to 8 atoms, the heteroalkyl comprising 1 to 3 heteroatoms selected from the group consisting of N, O and S, wherein the $C_{1-8}$ alkyl, cycloalkyl and linear heteroalkyl are each independently and optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids of phenylalanine (F), glycine (G), valine (V), lysine (K), citrulline, serine (S), glutamic acid (E) and aspartic acid (D), and are optionally further substituted with one or more substituents selected from the group consisting of halogen, hydroxy, cyano, amino, alkyl, chloroalkyl,

deuterated alkyl, alkoxy and cycloalkyl;

$R^5$ is selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments, the antibody-drug conjugate of general formula (Pc-L-Y-D) or the pharmaceutically acceptable salt thereof as described in any one of the above embodiments is an antibody-drug conjugate of general formula (Pc-$L_b$-Y-D) or a pharmaceutically acceptable salt thereof

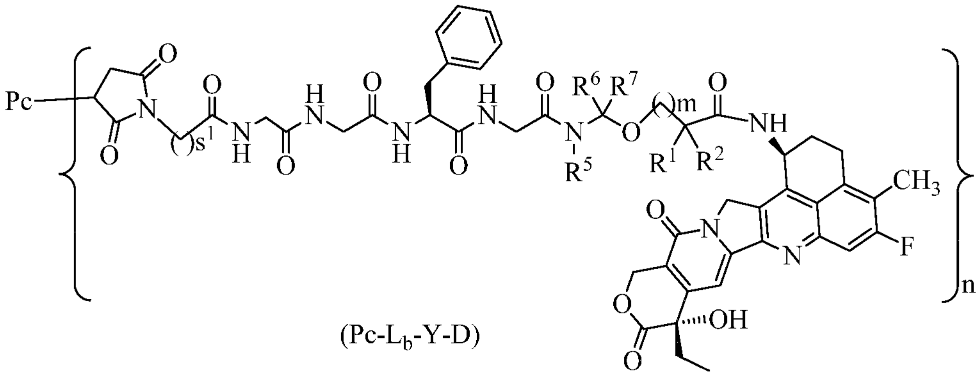

(Pc-$L_b$-Y-D)

wherein:

$s^1$ is an integer from 2 to 8;

Pc, $R^1$, $R^2$, $R^5$, $R^6$, $R^7$, m and n are as defined in general formula (Pc-$L_a$-Y-D).

In some embodiments, in the antibody-drug conjugate of general formula (Pc-L-Y-D) or the pharmaceutically acceptable salt thereof as described in any one of the above embodiments, the antibody-drug conjugate is selected from the group consisting of:

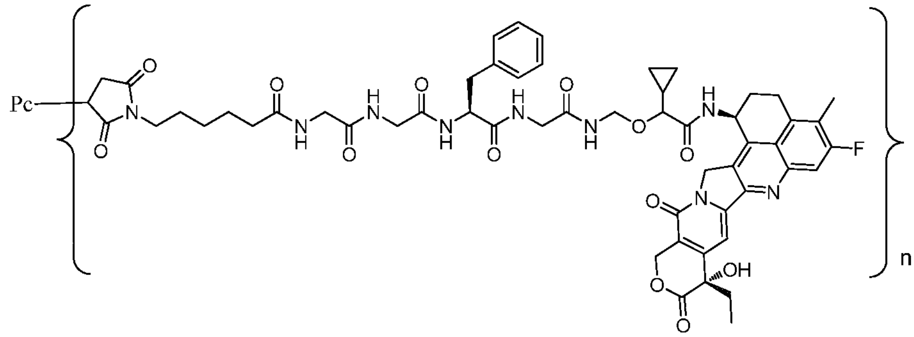

,

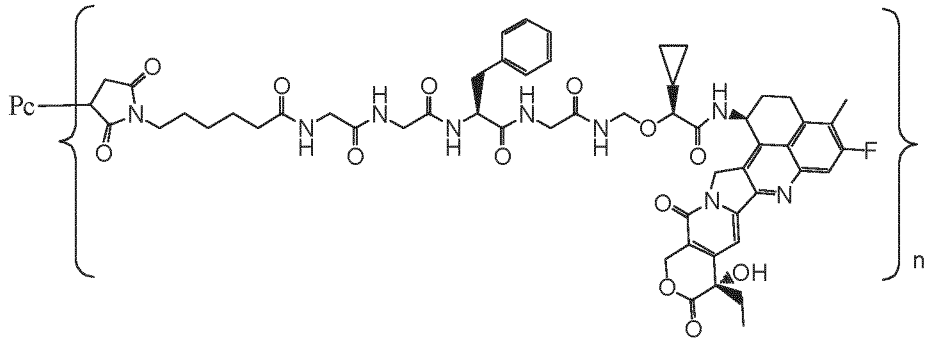

and

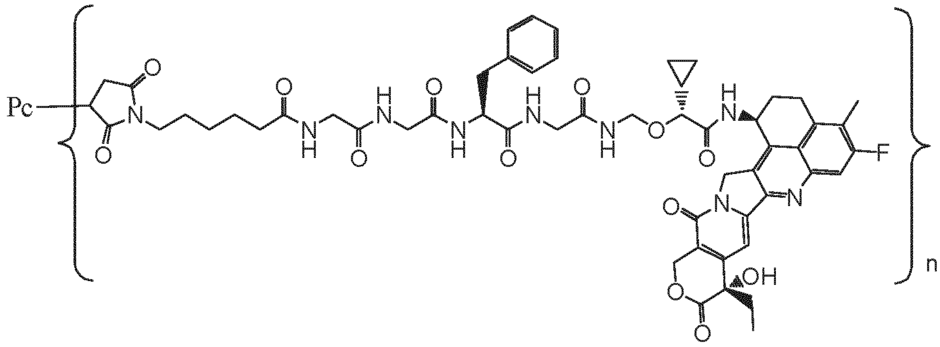

wherein Pc and n are as defined in general formula (Pc-L-Y-D); specifically, Pc is an anti-PSMA antibody or an antigen-binding fragment thereof, or the anti-PSMA antibody as described in any one of the above embodiments; n is a decimal or an integer from 1 to 10; preferably, n is from 1 to 8, more preferably from 3 to 8, most preferably from 3 to 7; most preferably from 6 to 7.

In some embodiments, in the antibody-drug conjugate of general formula (Pc-L-Y-D) or the pharmaceutically acceptable salt thereof as described in any one of the above embodiments, the antibody-drug conjugate is:

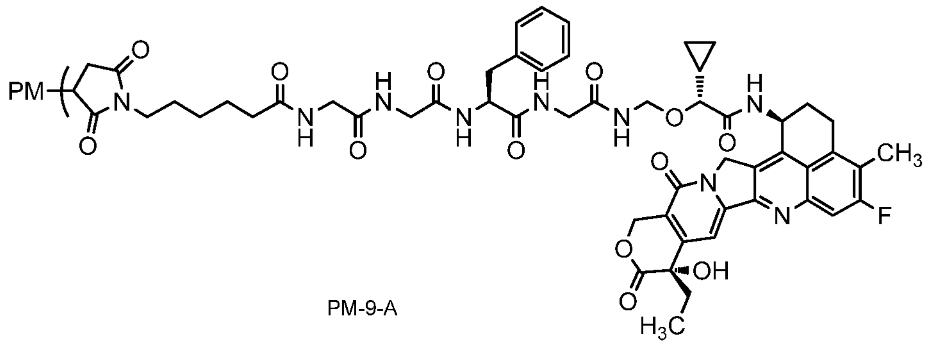

PM-9-A

wherein:

n is a decimal or an integer from 1 to 8, preferably from 3 to 8;
PM is an anti-PSMA antibody comprising a heavy chain set forth in SEQ ID NO: 9 and a light chain set forth in SEQ ID NO: 10.

The present disclosure further provides a method for preparing an antibody-drug conjugate of general formula (Pc-$L_a$-Y-D) or a pharmaceutically acceptable salt thereof, which comprises the following step:

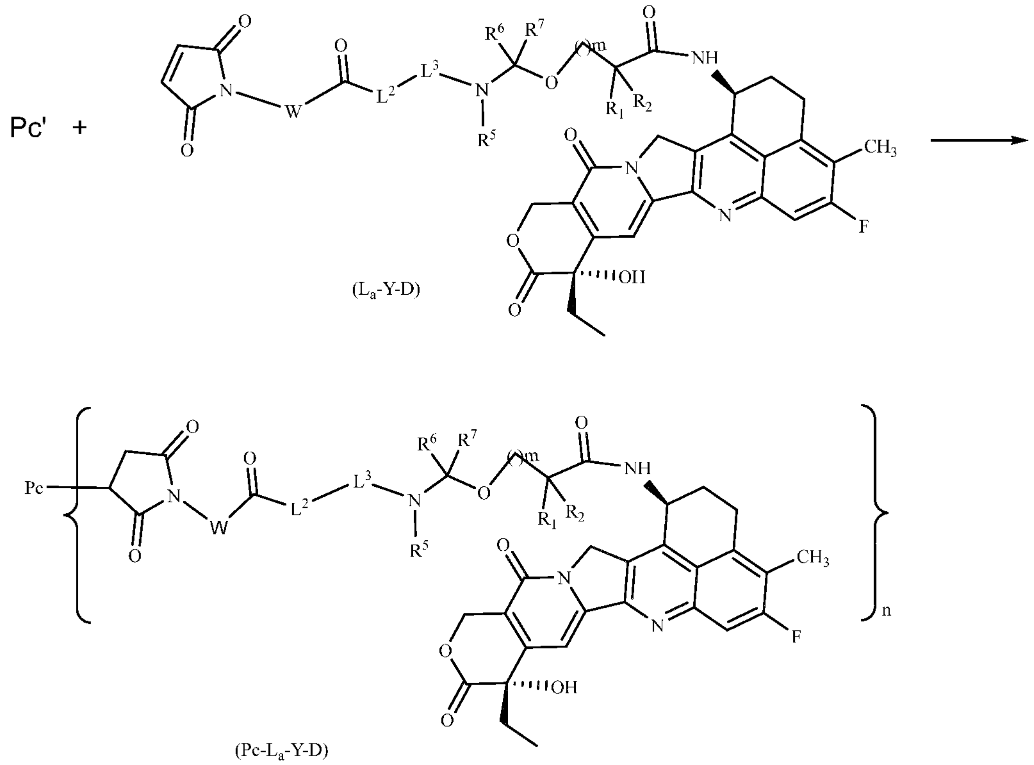

$(L_a\text{-}Y\text{-}D)$

$(Pc\text{-}L_a\text{-}Y\text{-}D)$

subjecting Pc', i.e., reduced Pc, and a compound of general formula ($L_a$-Y-D) to a coupling reaction to give a compound of general formula (Pc-$L_a$-Y-D);
wherein:

Pc is an anti-PSMA antibody or an antigen-binding fragment thereof;
n, m, W, $L^2$, $L^3$, $R^1$, $R^2$, $R^5$, $R^6$ and $R^7$ are as defined in the general formula (Pc-$L_a$-Y-D) as described above.

The present disclosure further provides a method for preparing an antibody-drug conjugate of general formula (Pc-L'-D), which comprises the following step:

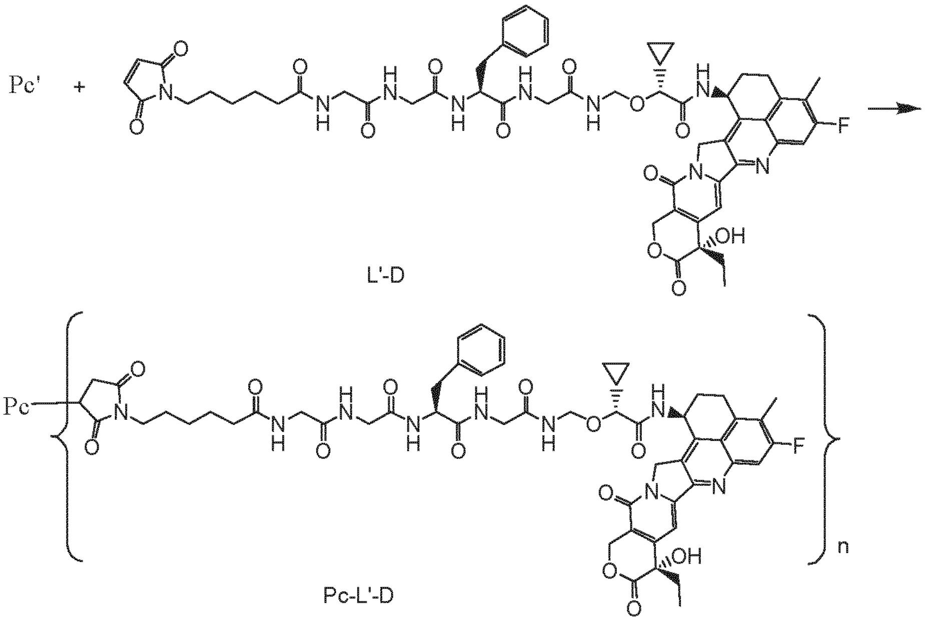

L'-D

Pc-L'-D

in the above step, subjecting Pc' and a compound of general formula (L'-D) to a coupling reaction to give a compound

of general formula (Pc-L'-D); wherein:

Pc is an anti-PSMA antibody or an antigen-binding fragment thereof as described above; Pc' is reduced Pc,
n is as defined in general formula (Pc-L-Y-D).

The present disclosure further provides a method of preparing an antibody-drug conjugate of formula PM-9-A, which comprises the following step:

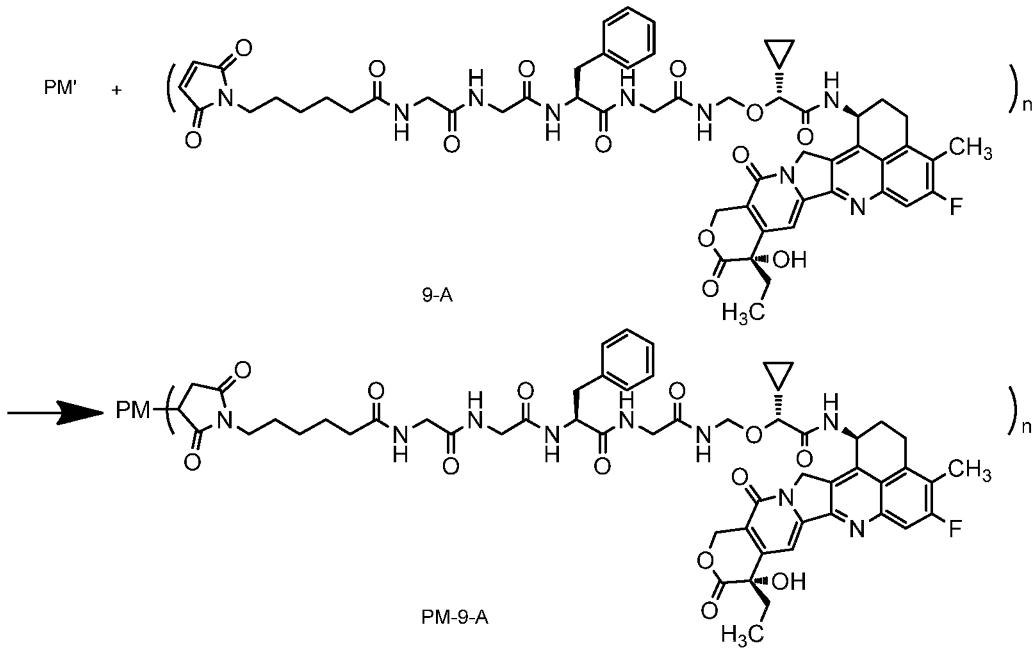

subjecting PM' and a compound of formula 9-A to a coupling reaction to give a compound of general formula (PM-9-A); wherein:

n is a decimal or an integer from 1 to 8, preferably from 3 to 8;
PM is an anti-PSMA antibody comprising a heavy chain with a sequence set forth in SEQ ID NO: 9 and a light chain with a sequence set forth in SEQ ID NO: 10;
PM' is obtained by reduction of PM.

In some embodiments, n represents the drug loading of the antibody-drug conjugate, and may also be referred to as a DAR value, which may be determined by conventional methods such as UV/visible spectroscopy, mass spectrometry, ELISA assay, and HPLC; in some embodiments, n is an average value of 0 to 10, preferably 1 to 10, and more preferably 1 to 8, or 2 to 8, or 2 to 7, or 2 to 4, or 3 to 8, or 3 to 7, or 3 to 6, or 4 to 7, or 4 to 6, or 4 to 5; in some embodiments, n is an average value of 1, 2, 3, 4, 5, 6, 7, 8, 9 and 10.

In another aspect, the present disclosure provides a pharmaceutical composition comprising the antibody-drug conjugate or the pharmaceutically acceptable salt thereof according to the present disclosure, and one or more pharmaceutically acceptable excipients, diluents or carriers. In some embodiments, the pharmaceutical composition in a unit dose comprises 0.1-3000 mg or 1-1000 mg of the anti-PSMA antibody as described above or the antibody-drug conjugate as described above.

In another aspect, the present disclosure provides use of the antibody-drug conjugate or the pharmaceutically acceptable salt thereof according to the present disclosure or the pharmaceutical composition comprising the same as a medicament.

In another aspect, the present disclosure provides use of the antibody-drug conjugate or the pharmaceutically acceptable salt thereof, or the pharmaceutical composition comprising the same according to the present disclosure in preparing a medicament for the treatment of a PSMA-mediated disease or disorder, wherein the PSMA-mediated disease or disorder is preferably a cancer with high, moderate or low expression of PSMA.

In another aspect, the present disclosure provides use of the antibody-drug conjugate or the pharmaceutically acceptable salt thereof, or the pharmaceutical composition comprising the same according to the present disclosure in preparing a medicament for the treatment or prevention of cancer, wherein the cancer is preferably head and neck

squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatoma, hepatocellular carcinoma, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelial carcinoma or Merkel cell carcinoma, preferably prostate cancer; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure further relates to a method for treating and/or preventing a tumor, which comprises administering to a subject in need thereof a therapeutically effective dose of the antibody-drug conjugate or the pharmaceutically acceptable salt thereof, or the pharmaceutical composition comprising the same according to the present disclosure; wherein the tumor is preferably a cancer associated with high, moderate or low expression of PSMA.

In another aspect, the present disclosure further relates to a method for treating or preventing a tumor or cancer, which comprises administering to a subject in need thereof a therapeutically effective dose of the antibody-drug conjugate or the pharmaceutically acceptable salt thereof, or the pharmaceutical composition comprising the same according to the present disclosure, wherein the tumor and cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatoma, hepatocellular carcinoma, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelial carcinoma or Merkel cell carcinoma; preferably prostate cancer; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure further provides use of the anti-PSMA antibody or the antibody-drug conjugate thereof as described above as a medicament, preferably as a medicament for the treatment of cancer or tumor, and more preferably as a medicament for the treatment of PSMA-mediated cancer.

The active compound (e.g., the antibody-drug conjugate or the pharmaceutically acceptable salt thereof according to the present disclosure) may be formulated into a form suitable for administration by any suitable route, preferably in a form of a unit dose, or in a form of a single dose that can be self-administered by a subject. The unit dose of the active compound or composition of the present disclosure may be in a tablet, a capsule, a cachet, a vial, a powder, a granule, a lozenge, a suppository, a powder for reconstitution or a liquid formulation.

The administration dose of the active compound or composition used in the treatment method of the present disclosure will generally vary with the severity of the disease, the weight of the subject, and the efficacy of the active compound. As a general guide, a suitable unit dose may be 0.1-1000 mg.

The pharmaceutical composition of the present disclosure may comprise, in addition to the active compound, one or more auxiliary materials selected from the group consisting of a filler, a diluent, a binder, a wetting agent, a disintegrant, an excipient and the like. Depending on the method of administration, the composition may comprise 0.1 wt.% to 99 wt.% of the active compound.

The PSMA antibody and the antibody-drug conjugate provided by the present disclosure have good affinity for cell surface antigens, good endocytosis efficiency and strong tumor inhibition efficiency, have broader drug application window, higher tumor inhibition effect and therapeutic activity, and better safety, pharmacokinetic property and druggability (such as stability), and are more suitable for clinical drug application.

## BRIEF DESCRIPTION OF THE DRAWINGS

FIG. 1A: *in vitro* binding ability of the ADC or antibody of the present disclosure to cell MDAPCa.
FIG. 1B: *in vitro* binding ability of the ADC or antibody of the present disclosure to cell LNCaP.
FIG. 1C: *in vitro* binding ability of the ADC or antibody of the present disclosure to cell 22Rv 1.
FIG. 1D: *in vitro* binding ability of the ADC or antibody of the present disclosure to cell PC-3.
FIG. 1E: *in vitro* binding ability of the ADC or antibody of the present disclosure to cell DU 145.
FIG. 2A: *in vitro* endocytosis assay of an antibody PM in LNCaP cells; 2K cells, 10% U-L IgG FBS.
FIG. 2B: *in vitro* endocytosis assay of an antibody PM in 22Rv1 cells; 2K cells, 10% U-L IgG FBS.
FIG. 3A: killing effect of different ADCs of the present disclosure on LNCaP cells; 2K cells, 4.5% FBS.
FIG. 3B: killing effect of the toxin (compound 2-B) of the present disclosure on LNCaP cells; 2K cells, 4.5% FBS.
FIG. 3C: killing effect of different ADCs of the present disclosure on 22Rv1 cells; 4K cells, 4.5% FBS.
FIG. 3D: killing effect of the toxin (compound 2-B) of the present disclosure on 22Rv1 cells; 4K cells, 4.5% FBS.
FIG. 3E: killing effect of different ADCs of the present disclosure on PC-3 cells; 4K cells, 4.5% FBS.
FIG. 3F: killing effect of the toxin (compound 2-B) of the present disclosure on PC-3 cells; 2K cells, 4.5% FBS.
FIG. 4A: inhibitory activity of different doses of ADCs of the present disclosure on human prostate cancer cell 22Rv1-induced xenograft tumor in nude mice.
FIG. 4B: change in body weight of mice in the test of inhibitory activity of different doses of ADCs of the present disclosure on human prostate cancer cell 22Rv1-induced xenograft tumor in nude mice.
FIG. 5A: inhibitory activity of different doses of ADCs of the present disclosure on human prostate cancer cell 22Rv1-induced xenograft tumor in nude mice.
FIG. 5B: change in body weight of mice in the test of inhibitory activity of different doses of ADCs of the present disclosure on human prostate cancer cell 22Rv1-induced xenograft tumor in nude mice.
FIG. 6A: inhibitory activity of different doses of ADCs of the present disclosure on human prostate cancer cell LNCap-induced xenograft tumor in SCID Beighe mice.
FIG. 6B: change in body weight of mice in the test of inhibitory activity of different doses of ADCs of the present disclosure on human prostate cancer cell LNCap-induced xenograft tumor in SCID Beighe mice.
FIG. 7: pharmacokinetic stability of ADC-2 of the present disclosure, wherein the concentration of ADC is 100 μg/mL.
FIG. 8: plasma stability of ADC-5 of the present disclosure, wherein the concentration of ADC is 100 μg/mL.
FIG. 9: efficacy of ADCs on the 22Rv1-induced xenograft tumor in tumor-bearing nude mice after the administration.

## DETAILED DESCRIPTION

### Terms

Unless defined otherwise, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the commercial product under the trade name, and the drug and active drug component of the commercial product under the trade name.

Unless otherwise stated, the terms used in the specification and claims have the following meanings.

The term "drug" or "toxin" refers to a cytotoxic drug that may have a chemical molecule within the tumor cell that is strong enough to disrupt its normal growth. The cytotoxic drug can kill cells in principle at a sufficiently high concentration; however, due to lack of specificity, the cytotoxic drug can cause apoptosis of normal cells while killing tumor cells, resulting in serious side effects. This term includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes.

The term "linker unit", "linker", "linking unit" or "linking fragment" refers to a chemical structural fragment or bond, which is linked to a ligand (an antibody, in the present disclosure) at one end and linked to a drug at the other end, and also may be linked to an additional linker and then linked to the ligand or the drug.

The linker may comprise one or more linker elements. Exemplary linker elements include 6-maleimidocaproyl ("MC"), maleimidopropionyl ("MP"), valine-citrulline ("val-cit" or "vc"), alanine-phenylalanine ("ala-phe"), *p*-aminobenzyloxycarbonyl ("PAB"), *N*-succinimidyl 4-(2-pyridylthio)pentanoate ("SPP"), *N*-succinimidyl 4-(*N*-maleimidomethyl)cyclohexane-1 carboxylate ("SMCC", also referred to herein as "MCC"), and *N*-succinimidyl(4-iodo-acetyl)aminobenzoate

("SIAB"). The linker may comprise one or more of the following elements, or a combination thereof: a stretcher unit, a spacer unit and an amino acid unit, and may be synthesized by methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research, 52: 127-131(1992); U.S. Patent No. 5,208,020).

Linker elements include, but are not limited to:

MC = 6-maleimidocaproyl, with a structure as follows:

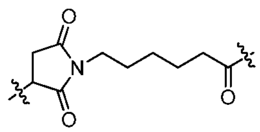

Val-Cit or "vc" = valine-citrulline (an exemplary dipeptide in a protease cleavable linker);
citrulline = 2-amino-5-ureidopentanoic acid;
PAB = *p*-aminobenzyloxycarbonyl (an example of "self-immolative" linker elements);
Me-Val-Cit = *N*-methyl-valine-citrulline (where the linker peptide bond has been modified to prevent it from being cleaved by cathepsin B);
MC(PEG)6-OH = maleimidocaproyl-polyethylene glycol (attachable to antibody cysteine);
SPP = *N*-succinimidyl 4-(2-pyridylthio)valerate;
SPDP = *N*-succinimidyl 3-(2-pyridyldithio)propionate;
SMCC = succinimidyl 4-(*N*-maleimidomethyl)cyclohexane-1-carboxylate;
IT = iminothiolane.
IT = iminothiolane.

The term "antibody-drug conjugate" means that an antibody is linked to a biologically active drug via a linking unit. In the present disclosure, "antibody-drug conjugate" (ADC) means that a monoclonal antibody or an antibody fragment is linked to a biologically active toxic drug via a linking unit. The antibody may be conjugated to the drug directly or via a linker. n is an average number of drug modules conjugated to each antibody, may be an integral or a decimal, and may range, for example, from about 0 to about 20 drug modules; in certain embodiments, from 1 to about 10 drug modules; and in certain embodiments, from 1 to about 8 drug modules, such as 2, 3, 4, 5, 6, 7 or 8 drug modules. In the composition of the mixture of the antibody-drug conjugate of the present disclosure, the mean drug loading of each antibody is about 1 to about 10, including but not limited to about 3 to about 7, about 3 to about 6, about 3 to about 5, about 1 to about 9, about 7 or about 4.

The three-letter and single-letter codes for amino acids used in the present disclosure are as described in J. biol. chem, 243, p3558 (1968).

The term "antibody" refers to an immunoglobulin, and an intact antibody is of a tetrapeptide chain structure formed by connection between two identical heavy chains and two identical light chains by interchain disulfide bonds. According to differences in the amino acid composition and the order of arrangement of the heavy chain constant regions of immunoglobulins, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into $\kappa$ or $\lambda$ chains by the differences in the constant regions. Each of the five classes of Ig may have a $\kappa$ chain or $\lambda$ chain.

In the heavy and light chains of full-length antibodies, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also referred to as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The terms "fully human-derived antibody", "fully human antibody", "human antibody" or "completely human antibody", also referred to as "fully human-derived monoclonal antibody", has both human-derived variable region and

constant region so as to eliminate immunogenicity and toxic side effects. Major relevant technologies for the preparation of fully human-derived antibodies include: human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to specifically bind to an antigen. A fragment of a full-length antibody can be used to perform the antigen-binding function of the antibody. The binding fragment included in the "antigen-binding fragment" is selected from the group consisting of Fab, Fab', $F(ab')_2$, single-chain antibody (scFv), dimerized V region (diabody), disulfide-stabilized V region (dsFv) and antigen-binding fragments of peptides comprising CDRs; examples include (i) Fab fragments, monovalent fragments consisting of VL, VH, CL and CH1 domains; (ii) $F(ab')_2$ fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. Furthermore, although the two domains of the Fv fragment, VL and VH, are encoded by separate genes, they can be linked by a synthetic linker by recombination, so that it is capable of producing a single protein chain in which the VL and VH regions pair to form a monovalent molecule (referred to as single-chain Fv (scFv); see, e.g., Bird et al. (1988) Science 242: 423-426; and Huston et al. (1988) Proc. Natl. Acad. Sci USA 85: 5879-5883). Such single-chain antibodies are also intended to be included in the term "antigen-binding fragment" of an antibody. Such antibody fragments are obtained using conventional techniques known to those skilled in the art, and screened for utility in the same manner as for intact antibodies. Antigen-binding moieties may be produced using recombinant DNA technology or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

In general, Fab is an antibody fragment having a molecular weight of about 50,000 and having antigen-binding activity, among fragments obtained by treating an IgG antibody molecule with a protease papain (e.g., cleaving the amino acid residue at position 224 of H chain), in which a portion on the N-terminal side of H chain is combined with L chain by a disulfide bond.

In general, $F(ab')_2$ is an antibody fragment having a molecular weight of about 100,000 and having antigen-binding activity, and comprising two Fab regions linked at the hinge position, which is obtained by digesting a portion below the disulfide bond in the IgG hinge region with the enzyme pepsin.

In general, Fab' is an antibody fragment having a molecular weight of about 50,000 and having antigen-binding activity, which is obtained by cleaving the disulfide bond in the hinge region of the $F(ab')_2$ as described above.

In addition, Fab' may be produced by inserting DNA encoding the Fab' fragment into a prokaryotic or eukaryotic expression vector and introducing the vector into a prokaryote or a eukaryote to express the Fab'.

The term "single-chain antibody", "single-chain Fv" or "scFv" means a molecule comprising an antibody heavy chain variable domain (or VH) and an antibody light chain variable domain (or VL) linked via a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. In general, there are three CDRs (HCDR1, HCDR2 and HCDR3) in each heavy chain variable region and three CDRs (LCDR1, LCDR2 and LCDR3) in each light chain variable region. Any one of a variety of well-known schemes can be used to determine the amino acid sequence boundaries of the CDRs, including the "Kabat" numbering scheme (see Kabat et al., (1991), "Sequences of Proteins of Immunological Interest", 5th edition, Public Health Service, National Institutes of Health, Bethesda, MD), the "Chothia" numbering scheme (see Al-Lazikani et al., (1997) JMB 273: 927-948), the ImMunoGenTics (IMGT) numbering scheme (Lefranc M. P., Immunologist, 7, 132-136 (1999); Lefranc, M. P., et al., Dev. Comp. Immunol., 27, 55-77 (2003)), etc. For example, for the classical format, according to the Kabat scheme, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2) and 89-97 (LCDR3). According to the Chothia scheme, the CDR amino acids in VH are numbered as 26-32 (HCDR1), 52-56 (HCDR2) and 95-102 (HCDR3); and amino acid residues in VL are numbered as 26-32 (LCDR1), 50-52 (LCDR2) and 91-96 (LCDR3). According to the CDR definitions by combining both the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are

roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). According to the IMGT scheme, the CDRs of the antibody can be determined using the program IMGT/DomainGap Align. Unless otherwise stated, the 6 CDRs in the present disclosure are all obtained according to the Kabat numbering scheme. (Kabat E.A. et al, (1991) Sequences of proteins of immunological interest. NIH Publication 91-3242). According to the Kabat scheme, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2) and 89-97 (LCDR3). Other numbering schemes in the art include Chothia, IMGT, etc.

The term "antibody framework region" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody binds. Epitopes typically comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to" and "specifically bind to" refer to the binding of an antibody or an antigen-binding fragment thereof to an epitope on a predetermined antigen. In general, the antibody or the antigen-binding fragment thereof binds with an affinity (KD) of less than about $10^{-7}$ M, e.g., less than about $10^{-8}$ M, $10^{-9}$ M, or $10^{-10}$ M or less.

The term "KD" refers to the dissociation equilibrium constant for an antibody-antigen interaction. In general, the antibody or the antigen-binding fragment of the present disclosure binds to PSMA or an epitope thereof with a dissociation equilibrium constant (KD) of less than about $10^{-7}$ M, e.g., less than about $10^{-8}$ M or $10^{-9}$ M; for example, the KD value is determined by the FACS method for the affinity of the antibody of the present disclosure for a cell surface antigen.

The term "nucleic acid molecule" refers to a DNA molecule or an RNA molecule. The nucleic acid molecule may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues shared by a first sequence and a second sequence, wherein in aligning the amino acid sequences, gaps are introduced, when necessary, to achieve maximum percent sequence identity, and any conservative substitution is not considered as part of the sequence identity. For the purpose of determining percent amino acid sequence identity, alignments can be achieved in a variety of ways that are within the skill in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences. The term "expression vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

The term "host cell" refers to a cell into which an expression vector has been introduced. Host cells may include microbial (e.g., bacterial), plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or the antigen-binding fragment of the present disclosure can be prepared and purified using conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Positive clones are expanded in a serum-free medium of a bioreactor to produce antibodies. The culture with the secreted antibody can be purified using conventional techniques. For example, purification is carried out on an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The term "peptide" refers to a molecule formed by connecting 2 or more amino acid molecules by peptide bonds, and is a structural and functional fragment of the protein.

The term "alkyl" refers to a saturated aliphatic hydrocarbon group that is a linear or branched group containing 1 to 20 carbon atoms, preferably alkyl containing 1 to 12 (such as 1, 2, 3, 4, 5, 6, 7, 8, 9, 10, 11 or 12) carbon atoms, more preferably alkyl containing 1 to 10 carbon atoms, and most preferably alkyl containing 1 to 6 carbon atoms (containing 1, 2, 3, 4, 5 or 6 carbon atoms). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1.1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl. 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various side-chain isomers thereof, and the like. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl may be substituted or unsubstituted, and when it is substituted, the substitution with a substituent may be performed at any accessible connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms (e.g., 1, 2, 3, 4, 5, 6, 7, 8, 9, 10, 11 or 12 carbon atoms), and more preferably alkylene containing 1 to 6 carbon atoms (containing 1, 2, 3, 4, 5 or 6 carbon atoms). Non-limiting examples of alkylene include, but are not limited to, methylene ($-CH_2-$), 1,1-ethylidene ($-CH(CH_3)-$), 1,2-ethylidene ($-CH_2CH_2)-$, 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$), 1,5-butylidene ($-CH_2CH_2CH_2CH_2CH_2-$), and the like. The alkylene may be substituted or unsubstituted, and when it is substituted, the substitution with a substituent may be performed at any accessible connection site, wherein the substituent is preferably independently and optionally selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. Alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 8 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The cycloalkyl ring includes those in which the cycloalkyl described above (including monocyclic, spiro, fused and bridged rings) is fused to an aryl, heteroaryl or heterocycloalkyl ring, wherein the ring connected to the parent structure is cycloalkyl. Non-limiting examples include indanyl, tetrahydronaphthyl, benzocycloheptanyl, and the like, preferably benzocyclopentyl and tetrahydronaphthyl.

The cycloalkyl may be substituted or unsubstituted, and when it is substituted, the substitution with a substituent may be performed at any accessible connection site, wherein the substituent is preferably independently and optionally selected from the group consisting of hydrogen, halogen, alkyl, alkoxy, haloalkyl, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic hydrocarbon substituent containing 3 to 20 ring atoms, wherein one or more of the ring atoms are heteroatoms selected from the

group consisting of nitrogen, oxygen and $S(O)_p$ (where p is an integer from 0 to 2), excluding a cyclic portion of -O-O-, -O-S- or -S-S-, and the remaining ring atoms are carbon atoms. The heterocyclyl preferably contains 3 to 12 ring atoms, of which 1 to 4 are heteroatoms; more preferably 3 to 8 ring atoms, of which 1 to 3 are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2.3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl. Non-limiting examples include:

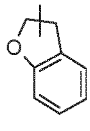, 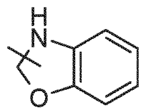, 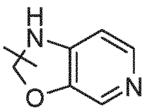, and 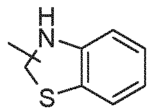.

The heterocyclyl may be substituted or unsubstituted, and when it is substituted, the substitution with a substituent may be performed at any accessible connection site, wherein the substituent is preferably independently and optionally selected from the group consisting of hydrogen, halogen, alkyl, alkoxy, haloalkyl, hydroxy, hydroxyalkyl, cyano, amino, nitro, cycloalkyl, heterocyclyl, aryl and heteroaryl.

The term "aryl" refers to a 6- to 14-membered, preferably 6- to 10-membered all-carbon monocyclic or fused polycyclic (i.e., rings that share a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is an aryl ring. Non-limiting examples include:

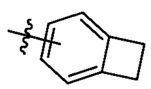, 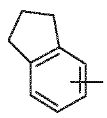, 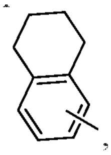, 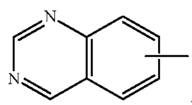, 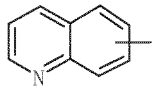, 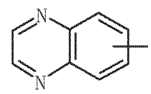,

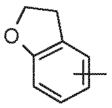, 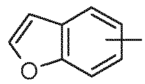, 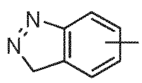, 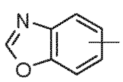, 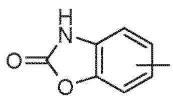, 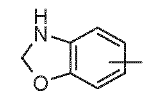,

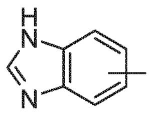, 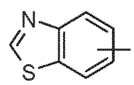, 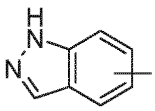, 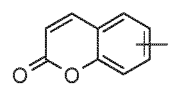 and 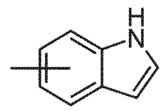.

The aryl may be substituted or unsubstituted, and when it is substituted, the substitution with a substituent may be performed at any accessible connection site, wherein the substituent is preferably independently and optionally selected from the group consisting of hydrogen, halogen, alkyl, alkoxy, haloalkyl, hydroxy, hydroxyalkyl, cyano, amino, nitro, cycloalkyl, heterocyclyl, aryl and heteroaryl.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered and more preferably 5-membered or 6-membered, e.g., furanyl, thienyl, pyridinyl, pyrrolyl, N-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring connected to the parent structure is a heteroaryl ring. Non-limiting examples include:

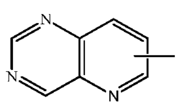 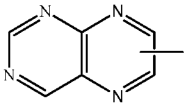 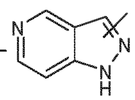 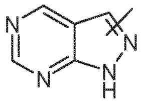 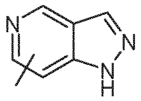 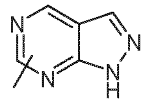

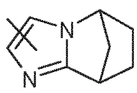 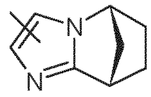 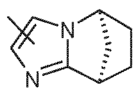 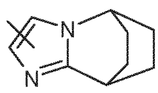 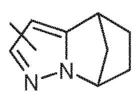 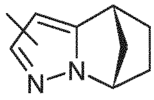

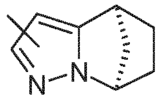 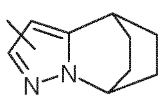 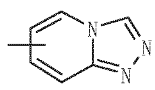 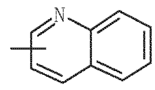 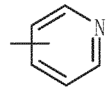 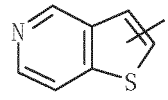

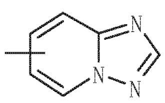 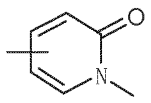 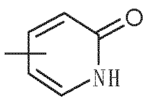 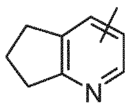 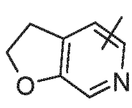 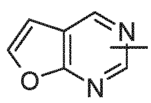

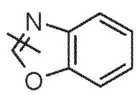 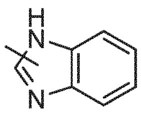 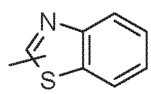 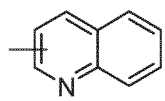 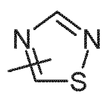 and 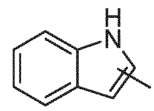 .

The heteroaryl may be substituted or unsubstituted, and when it is substituted, the substitution with a substituent may be performed at any accessible connection site, wherein the substituent is preferably independently and optionally selected from the group consisting of hydrogen, halogen, alkyl, alkoxy, haloalkyl, hydroxy, hydroxyalkyl, cyano, amino, nitro, cycloalkyl, heterocyclyl, aryl and heteroaryl.

The term "haloalkyl" refers to an alkyl group in which the hydrogen is substituted with one or more halogens, wherein the alkyl is as defined above.

The term "deuterated alkyl" refers to an alkyl group in which the hydrogen is substituted with one or more deuterium atoms, wherein the alkyl is as defined above. The term "hydroxyalkyl" refers to an alkyl group in which the hydrogen is substituted with one or more hydroxy groups, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

The term "cyano" refers to -CN.

The term "optional" or "optionally" means that the event or circumstance subsequently described may, but not necessarily, occur, and that the description includes instances where the event or circumstance occurs or does not occur. For example, "heterocyclyl group optionally substituted with alkyl" means that alkyl may be, but not necessarily, present, and that the description includes instances where the heterocyclyl group is or is not substituted with alkyl.

The term "substituted" means that one or more, preferably up to 5, more preferably 1, 2 or 3 hydrogen atoms in the group are independently substituted with a substituent. A substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond. The term "pharmaceutical composition" refers to a mixture containing one or more of the compounds described herein or a physiologically/pharmaceutically acceptable salt or prodrug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities. The term "pharmaceutically acceptable salt" refers to salts of the antibody-drug conjugate of the present disclosure. Such salts are safe and effective when used in a subject and possess the required biological activity. The antibody-drug conjugate of the present disclosure at least comprises one amino group and thus may form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and p-toluenesulfonate.

The term "drug loading" or "mean drug loading" is also referred to as the drug-to-antibody ratio (DAR), which is the average number of drugs conjugated to each antibody in the ADC. It may range, for example, from about 1 to about 10 drugs conjugated to each antibody, and in certain embodiments, from about 1 to about 8 drugs conjugated to each antibody, preferably selected from the group consisting of 2 to 8, 2 to 7, 2 to 6, 2 to 5, 2 to 4, 3 to 4, 3 to 5, 5 to 6, 5 to 7, 5 to 8 and 6 to 8 drugs conjugated to each antibody. Illustratively, the drug loading may be an average number of 1, 2, 3, 4, 5, 6, 7, 8, 9 and 10. The ADC general formulas of the present disclosure include a collection of antibody-drug conjugates in a range of drug loadings as described above. In embodiments of the present disclosure, the drug loading is represented as n, which may also be referred to as a DAR value, and illustratively, may be an average number of 1, 2, 3, 4, 5, 6, 7, 8, 9 and 10. The drug loading can be determined by conventional methods such as UV/visible spectroscopy, mass spectrometry, ELISA assay and HPLC. The loading of the antibody-drug conjugate can be controlled by the following non-limiting methods, including:

(1) controlling a molar ratio of a linking reagent to a monoclonal antibody,
(2) controlling reaction time and temperature, and
(3) selecting different reaction reagents.

Referring to *Chinese Pharmacopoeia* for preparation of conventional pharmaceutical compositions.

The term "carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants, lubricants in tablets; the matrix part in semisolid ointment and cream preparations; preservatives, antioxidants, corrigents, fragrances, cosolvents, emulsifiers, solubilizers, tonicity adjusting agents, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug’s compression moldability and the like. When the drug in the tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.

The pharmaceutical composition may be in the form of a sterile injectable aqueous solution. Available and acceptable vehicles or solvents include water, Ringer’s solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer solutions and microemulsions in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections.

**Synthesis Method**

For the synthesis purpose, the following technical schemes for synthesis are adopted. Provided is a method for preparing the compound of general formula (PM-9-A), which comprises the following step:

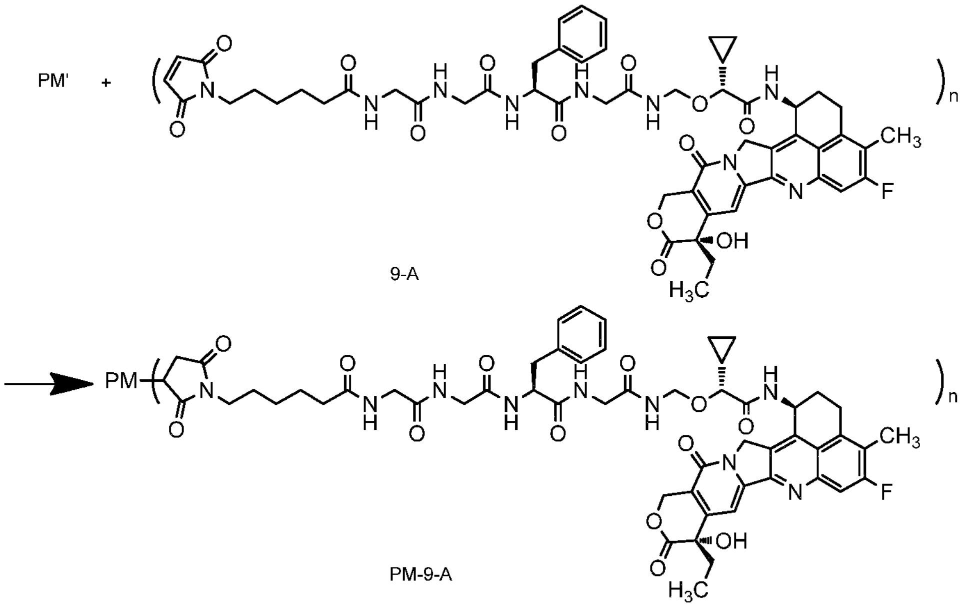

9-A

PM-9-A

subjecting PM', i.e., reduced PM, and a compound of general formula (9-A) to a coupling reaction to give a compound of general formula (PM-9-A), wherein the reducing agent is preferably TCEP; particularly, the disulfide bonds in the antibody are preferably reduced;
wherein:

PM is an anti-PSMA antibody or an antigen-binding fragment thereof;
n is a decimal or an integer from 1 to 10.

One or more embodiments of the present disclosure are described in detail in the specification above. Although any methods and materials similar or identical to those described herein can also be used to implement or test the present disclosure, preferred methods and materials are described below. Other features, purposes and advantages of the present disclosure will be apparent from the description and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure that is defined by the claims.

Experimental procedures without specific conditions indicated in the examples or test examples are generally conducted according to conventional conditions, or according to conditions recommended by the manufacturer of the starting materials or commercial products, see Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

**Example 1. Preparation of antibodies**

**(I) Preparation of PSMA antibody**

**1.1 Antibody sequence**

The antibodies of the present disclosure were prepared with reference to WO2003034903A2, in which the amino acid sequences of variable regions of AB-PG1-XG1-006 were as follows:

Heavy chain variable region of AB-PG1-XG1-006:

QVQLVESGGGVVQPGRSLRLSCAASGFAFSRYGMHWVRQAPGKGLEWVAVIWYDGSNKYYADSVKGRFTISRDNSKNTQYLQMNSLRAEDTAVYYCARGGDFLYYYYYGMDVWGQGTTVTVSS

SEQ ID NO: 1

Light chain variable region of AB-PG1-XG1-006:

DIQMTQSPSSLSASVGDRVTITCRASQGISNYLAWYQQKTGKVPKFLIYEASTLQSGVPSRFSGGGSGTDFTLTISSLQPEDVATYYCQNYNSAPFTFGPGTKVDIK

SEQ ID NO: 2

Note: the underlined portions are CDR regions determined according to the Kabat numbering scheme.

Table 1. CDR regions of the AB-PG1-XG1-006 antibody

| **Antibody** | AB-PG1-XG1-006 |
|---|---|
| **Heavy chain CDR1** | RYGMH (SEQ ID NO:3) |
| **Heavy chain CDR2** | VIWYDGSNKYYADSVKG (SEQ ID NO:4) |
| **Heavy chain CDR3** | GGDFLYYYYYGMDV (SEQ ID NO:5) |
| **Light chain CDR1** | RASQGISNYLA (SEQ ID NO:6) |
| **Light chain CDR2** | EASTLQS (SEQ ID NO:7) |
| **Light chain CDR3** | QNYNSAPFT (SEQ ID NO:8) |

**1.2 Construction of full-length antibody**

The VH/VK gene fragment was constructed by designing a primer PCR according to the above sequences to obtain variable regions.

The variable regions of the antibody were subjected to homologous recombination with a constant region gene (CH1-Fc/CL) fragment to construct an intact antibody VH-CH1-Fc/VK-CL.

The sequences of the intact full-length antibody PM constructed were as follows:

Heavy chain (IgG1) amino acid sequence:

QVQLVESGGGVVQPGRSLRLSCAASGFAFSRYGMHWVRQAPGKGLEWVAVIW
YDGSNKYYADSVKGRFTISRDNSKNTQYLQMNSLRAEDTAVYYCARGGDFLYY
YYYGMDVWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGN
VFSCSVMHEALHNHYTQKSLSLSPGK

SEQ ID NO: 9

Light chain (Kappa) amino acid sequence:

DIQMTQSPSSLSASVGDRVTITCRASQGISNYLAWYQQKTGKVPKFLIYEASTLQ
SGVPSRFSGGGSGTDFTLTISSLQPEDVATYYCQNYNSAPFTFGPGTKVDIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 10

**1.3 Expression and purification of full-length antibody**

HEK293E cells were transfected with plasmids expressing light and heavy chains of the antibody, respectively, and after 6 days, expression supernatants were collected, centrifuged at high speed to remove impurities, and purified using a Protein A column. The column was washed with PBS until $A_{280}$ reading dropped to baseline. The target protein was eluted with acidic eluent of pH 3.0-3.5, and neutralized with 1 M Tris-HCl of pH 8.0-9.0. The eluted sample was appropriately concentrated and further purified by gel chromatography Superdex200 (GE) equilibrated with PBS to remove aggregates, and the eluate with monomer peak was collected and aliquoted for later use.

**(II) Preparation of control antibody Lmab**

The control antibody, labeuzumab (abbreviated as Lmab), was prepared with reference to WHO Drug Information Vol. 30, No. 1, 2016, in which the amino acid sequences of heavy and light chains were as follows:

>Antibody heavy chain sequence Lmab-HC

EVQLVESGGGVVQPGRSLRLSCSASGFDFTTYWMSWVRQAPGKGLEWIGEIHP
DSSTINYAPSLKDRFTISRDNAKNTLFLQMDSLRPEDTGVYFCASLYFGFPWFAY
WGQGTPVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK SEQ ID NO: 11

>Antibody light chain sequence Lmab-LC

DIQLTQSPSSLSASVGDRVTITCKASQDVGTSVAWYQQKPGKAPKLLIYWTSTR
HTGVPSRFSGSGSGTDFTFTISSLQPEDIATYYCQQYSLYRSFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 12

**Example 2. Preparation of compounds**

Experimental procedures without conditions specified in the examples of the present disclosure are generally conducted according to conventional conditions, or according to conditions recommended by the manufacturer of the starting materials or commercial products. Reagents without specific origins indicated are commercially available conventional reagents.

The structure of the compounds was determined by nuclear magnetic resonance (NMR) or mass spectrometry (MS). NMR spectra were measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as determination solvents and tetramethylsilane (TMS) as internal standard. Chemical shifts were given in unit of $10^{-6}$ (ppm).

MS analysis was performed using a FINNIGAN LCQAd (ESI) mass spectrometer (manufacturer: Thermo, model: Finnigan LCQ advantage MAX).

UPLC analysis was performed using a Waters Acquity UPLC SQD liquid chromatography-mass spectrometry system.

HPLC analysis was performed using an Agilent 1200DAD high pressure liquid chromatograph (Sunfire C18 150 × 4.6 mm chromatography column) and a Waters 2695-2996 high pressure liquid chromatograph (Gimini C18 150 × 4.6 mm chromatography column).

UV-HPLC analysis was performed using a Thermo nanodrop2000 ultraviolet spectrophotometer.

Proliferation inhibition rates and $IC_{50}$ values were measured using a PHERA starFS microplate reader (BMG, Germany).

Yantai Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Huanghai silica gel of 200-300 mesh is generally used as a carrier in column chromatography.

Known starting materials of the present disclosure may be synthesized using or according to methods known in the art, or may be purchased from ABCR GmbH & Co.KG, Acros Organics, Aldrich Chemical Company, Accela ChemBio Inc, Shanghai Darui Finechemical Co., Ltd., and the like.

In the Examples, the reactions were performed under argon atmosphere or nitrogen atmosphere unless otherwise stated.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involved 3 cycles of vacuumization and hydrogen purge.

A CEM Discover-S 908860 microwave reactor was used in the microwave reactions.

In the Examples, the solution in the reaction refers to an aqueous solution unless otherwise stated.

In the Examples, the reaction temperature is room temperature unless otherwise stated.

The room temperature is the optimum reaction temperature, ranging from 20 °C to 30 °C.

Preparation of a PBS buffer at pH 6.5 in Examples: 8.5 g of $KH_2PO_4$, 8.56 g of $K_2HPO_4.3H_2O$, 5.85 g of NaCl and 1.5 g of EDTA were added to a flask with the volume brought to 2 L, and the mixture was ultrasonically dissolved and well mixed by shaking to give the desired buffer.

The eluent system for column chromatography and the developing solvent system for thin layer chromatography used for compound purification included: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure were characterized by Q-TOF LC/MS. Q-TOF LC/MS analysis was performed using an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

Y-D drug portion of the antibody-drug conjugates of the present disclosure is found in PCT/CN2019/107873, and the synthesis and tests of relevant compounds are incorporated herein by reference. Non-limiting Examples of synthesis are incorporated by reference as follows:

**Example 2-1**

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycycloprop ane-1-carboxamide 1

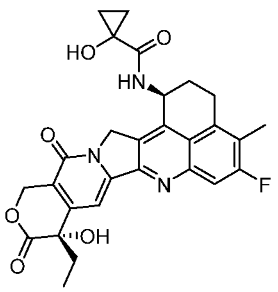

1

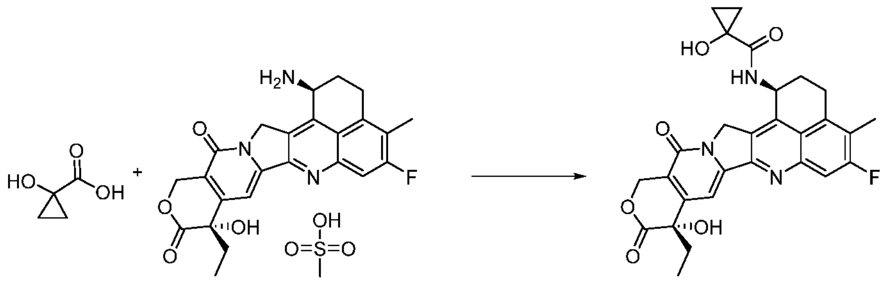

1a 1b 1

To exatecan mesylate 1b (2.0 mg, 3.76 μmol, prepared according to the method disclosed in Patent Application "EP0737686A1") was added 1 mL of *N,N*-dimethylformamide. The mixture was cooled to 0-5 °C in an ice-water bath,

followed by the addition of a drop of triethylamine. The resulting mixture was stirred until it became clear. To the reaction solution were successively added 1-hydroxycyclopropylcarboxylic acid 1a (1.4 mg, 3.7 μmol, prepared according to the known method "Tetrahedron Letters, 25(12), 1269-72; 1984") and 4-(4,6-dimethoxy-1,3,5-triazin-2-yl)-4-methylmorpholinium chloride (3.8 mg, 13.7 μmol). After the addition, the mixture was stirred at 0-5 °C for 2 h. 5 mL of water was added to the reaction solution to quench the reaction, and the mixture was extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 1 (1.6 mg, 82.1% yield).

MS m/z (ESI): 520.2 [M+ 1].

$^1$H NMR (400 MHz, $CDCl_3$): δ 7.90-7.84 (m, 1H), 7.80-7.68(m, 1H), 5.80-5.70 (m, 1H), 5.62-5.54 (m, 2H), 5.44-5.32 (m, 2H), 5.28-5.10 (m, 2H), 3.40-3.15 (m, 3H), 2.44 (s, 3H), 2.23 (t, 1H), 2.06-1.75 (m, 2H), 1.68-1.56 (m, 1H), 1.22-1.18 (m, 2H), 1.04-0.98 (m, 2H), 0.89 (t, 3H).

**Example 2-2**

(*S*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-A**

(*R*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-B**

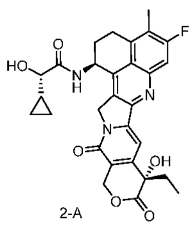

2-A

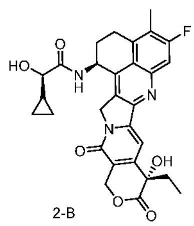

2-B

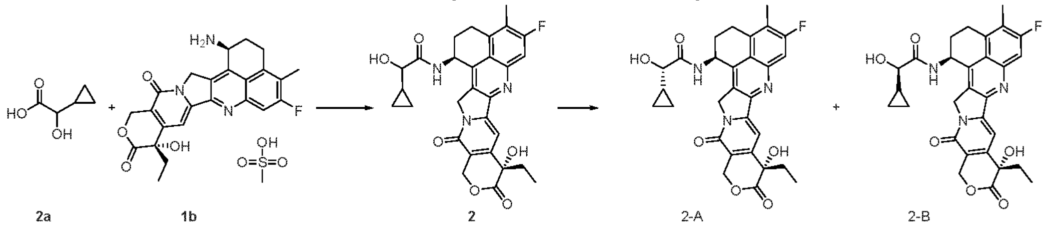

2a 1b 2 2-A 2-B

To 1b (4 mg, 7.53 μmol) were added 2 mL of ethanol and 0.4 mL of *N,N*-dimethylformamide. The mixture was purged with argon three times, and cooled to 0-5 °C in an ice-water bath, followed by the dropwise addition of 0.3 mL of *N*-methylmorpholine. The resulting mixture was stirred until it became clear. To the reaction solution were successively added 2-cyclopropyl-2-hydroxyacetic acid 2a (2.3 mg, 19.8 μmol, prepared according to the method disclosed in Patent Application "WO2013106717"), 1-hydroxybenzotriazole (3 mg, 22.4 μmol) and 1-(3-dimethylaminopropyl)-3-ethylcarbodiimide hydrochloride (4.3 mg, 22.4 μmol). After the addition, the resulting mixture was stirred at 0-5 °C for 1 h. The ice-water bath was removed, and the reaction solution was heated to 30 °C, stirred for 2 h, and concentrated under reduced pressure. The resulting crude compound 2 was purified by high performance liquid chromatography (separation conditions: chromatography column: XBridge Prep C18 OBD 5 μm 19 × 250 mm; mobile phase: A-water (10 mmol $NH_4OAc$), B-acetonitrile, gradient elution, flow rate: 18 mL/min), and the corresponding fractions were collected and concentrated under reduced pressure to give the title products (2-A: 1.5 mg, 2-B: 1.5 mg).

MS m/z (ESI): 534.0 [M+1].

Single-configuration compound 2-B (shorter retention time)

UPLC analysis: retention time: 1.06 min; purity: 88% (chromatography column: ACQUITY UPLC BEH C18 1.7 μm 2.1 × 50 mm; mobile phase: A-water (5 mmol $NH_4OAc$), B-acetonitrile).

$^1$H NMR (400 MHz, DMSO-$d_6$): δ 8.37 (d, 1H), 7.76 (d, 1H), 7.30 (s, 1H), 6.51 (s, 1H), 5.58-5.56 (m, 1H), 5.48 (d, 1H), 5.41 (s, 2H), 5.32-5.29 (m, 2H), 3.60 (t, 1H), 3.19-3.13 (m, 1H), 2.38 (s, 3H), 2.20-2.14 (m, 1H), 1.98 (q, 2H),

1.87-1.83 (m, 1H), 1.50-1.40 (m, 1H), 1.34-1.28 (m, 1H), 0.86 (t, 3H), 0.50-0.39 (m, 4H).

Single-configuration compound 2-A (longer retention time)

UPLC analysis: retention time: 1.10 min; purity: 86% (chromatography column: ACQUITY UPLC BEH C18 1.7 μm 2.1 × 50 mm; mobile phase: A-water (5 mmol $NH_4OAc$), B-acetonitrile).
$^1H$ NMR (400 MHz, DMSO-$d_6$): δ 8.35 (d, 1H), 7.78 (d, 1H), 7.31 (s, 1H), 6.52 (s, 1H), 5.58-5.53 (m, 1H), 5.42 (s, 2H), 5.37 (d, 1H), 5.32 (t, 1H), 3.62 (t, 1H), 3.20-3.15 (m, 2H), 2.40 (s, 3H), 2.25-2.16 (m, 1H), 1.98 (q, 2H), 1.87-1.82 (m, 1H), 1.50-1.40 (m, 1H), 1.21-1.14 (m, 1H), 0.87 (t, 3H), 0.47-0.35 (m, 4H).

**Example 2-3**

(*S*)-*N*-((1*S*,9*S*)-9-ethyl-5-fluoro-9-hydroxy-4-methyl-10,13-dioxo-2,3,9,10,13,15-hexah ydro-1*H*,12*H*-benzo[*de*]pyrano[3',4':6,7]indolizino[1,2-*b*]quinolin-1-yl)-3,3,3-trifluoro-2-hydroxypropionamide **3-A**

(*R*)-*N*-((1*S*,9*S*)-9-ethyl-5-fluoro-9-hydroxy-4-methyl-10,13-dioxo-2,3,9,10,13,15-hexah ydro-1*H*,2*H*-benzo[*de*]pyrano[3',4':6,7]indolizino[1,2-*b*]quinolin-1-yl)-3,3,3-trifluoro-2-hydroxypropionamide **3-B**

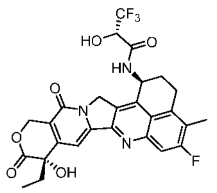
3-A

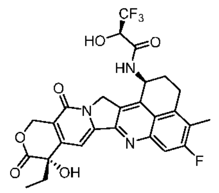
3-B

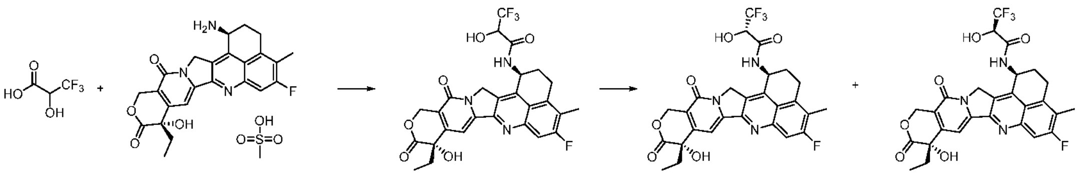
3a 1b 3 3-A 3-B

To **1b** (5.0 mg, 9.41 μmol) were added 2 mL of ethanol and 0.4 mL of *N,N*-dimethylformamide. The mixture was cooled to 0-5 °C in an ice-water bath, followed by the dropwise addition of 0.3 mL of *N*-methylmorpholine. The resulting mixture was stirred until it became clear. To the reaction solution were successively added 3,3,3-trifluoro-2-hydroxypropionic acid **3a** (4.1 mg, 28.4 μmol, supplied by Alfa), 1-hydroxybenzotriazole (3.8 mg, 28.1 μmol) and 1-(3-dimethylaminopropyl)-3-ethylcarbodiimide hydrochloride (5.4 mg, 28.2 μmol). After the addition, the resulting mixture was stirred at 0-5 °C for 10 min. The ice-water bath was removed, and the reaction solution was heated to 30 °C, stirred for 8 h, and concentrated under reduced pressure. The resulting crude compound 3 was purified by high performance liquid chromatography (separation conditions: chromatography column: XBridge Prep C18 OBD 5 μm 19 × 250 mm; mobile phase: A-water (10 mmol $NH_4OAc$), B-acetonitrile, gradient elution, flow rate: 18 mL/min), and the corresponding fractions were collected and concentrated under reduced pressure to give the title products (1.5 mg, 1.5 mg).
MS m/z (ESI): 561.9 [M+1].

Single-configuration compound (shorter retention time)

UPLC analysis: retention time: 1.11 min; purity: 88% (chromatography column: ACQUITY UPLC BEH C18 1.7 μm 2.1 × 50 mm; mobile phase: A-water (5 mmol $NH_4OAc$), B-acetonitrile).
$^1H$ NMR (400 MHz, DMSO-$d_6$): δ 8.94 (d, 1H), 7.80 (d, 1H), 7.32 (s, 1H), 7.20 (d, 1H), 6.53 (s, 1H), 5.61-5.55 (m, 1H), 5.45-5.23 (m, 3H), 5.15-5.06 (m, 1H), 4.66-4.57 (m, 1H), 3.18-3.12 (m, 1H), 2.40 (s, 3H), 2.26-2.20 (m, 1H), 2.16-2.08 (m, 1H), 2.02-1.94 (m, 1H), 1.89-1.82 (m, 1H), 1.50-1.40 (m, 1H), 0.87 (t, 3H).

Single-configuration compound (longer retention time)

UPLC analysis: retention time: 1.19 min; purity: 90% (chromatography column: ACQUITY UPLC BEH C18 1.7

μm 2.1 × 50 mm; mobile phase: A-water (5 mmol $NH_4OAc$), B-acetonitrile).

$^1H$ NMR (400 MHz, DMSO-$d_6$): δ 8.97 (d, 1H), 7.80 (d, 1H), 7.31 (s, 1H), 7.16 (d, 1H), 6.53 (s, 1H), 5.63-5.55 (m, 1H), 5.45-5.20 (m, 3H), 5.16-5.07 (m, 1H), 4.66-4.57 (m, 1H), 3.18-3.12 (m, 1H), 2.40 (s, 3H), 2.22-2.14 (m, 1H), 2.04-1.95 (m, 2H), 1.89-1.82 (m, 1H), 1.50-1.40 (m, 1H), 0.87 (t, 3H).

### Example 2-4

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycyclopent ane-1-carboxamide **4**

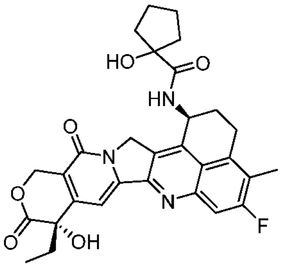

**4**

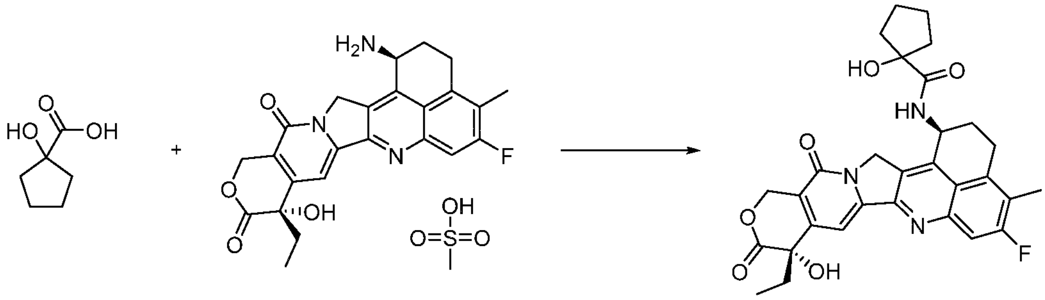

**4a** **1b** **4**

To **1b** (3.0 mg, 5.64 μmol) was added 1 mL of *N,N*-dimethylformamide. The mixture was cooled to 0-5 °C in an ice-water bath, followed by the addition of a drop of triethylamine. The resulting mixture was stirred until it became clear. To the reaction solution were successively added 1-hydroxy-cyclopentanecarboxylic acid **4a** (2.2 mg, 16.9 μmol, prepared according to the method disclosed in Patent Application "WO2013106717") and 4-(4,6-dimethoxy-1,3,5-triazin-2-yl)-4-methylmorpholinium chloride (4.7 mg, 16.9 μmol). After the addition, the resulting mixture was stirred at 0-5 °C for 1 h. 5 mL of water was added to the reaction solution to quench the reaction, and the mixture was extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **4** (2.5 mg, 80.9% yield).

MS m/z (ESI): 548.0 [M+1].

$^1H$ NMR (400 MHz, $CDCl_3$): δ 7.73-7.62 (m, 2H), 5.75-5.62 (m, 1H), 5.46-5.32 (m, 2H), 5.26-5.10 (m, 1H), 3.30-3.10 (m, 1H), 2.43 (s, 3H), 2.28-2.20 (m, 2H), 2.08-1.84 (m, 8H), 1.69-1.58 (m, 2H), 1.04-1.00 (m, 2H), 0.89 (t, 3H).

### Example 2-5

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-(hydroxymethyl)c yclopropane-1-carboxamide **5**

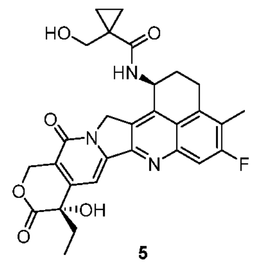

5

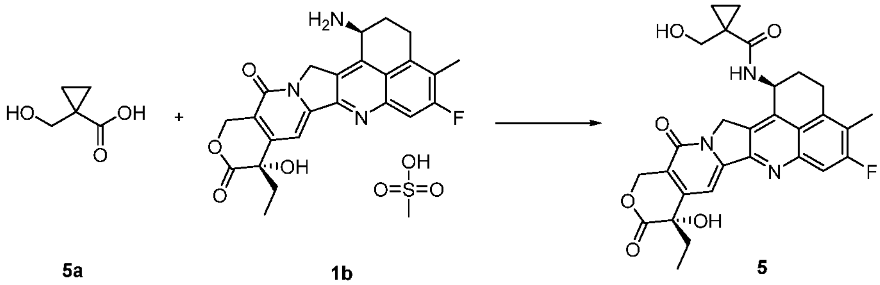

5a 1b 5

To **1b** (2.0 mg, 3.76 μmol) was added 1 mL of *N,N*-dimethylformamide. The mixture was cooled to 0-5 °C in an ice-water bath, followed by the addition of a drop of triethylamine. The resulting mixture was stirred until it became clear. To the reaction solution were successively added 1-(hydroxymethyl)-cyclopentanecarboxylic acid 5a (0.87 mg, 7.5 μmol, prepared according to the method disclosed in Patent Application "WO201396771") and 4-(4,6-dimethoxy-1,3,5-triazin-2-yl)-4-methylmorpholinium chloride (2 mg, 7.24 μmol). After the addition, the resulting mixture was stirred at 0-5 °C for 2 h. 5 mL of water was added to the reaction solution quench the reaction, and the mixture was extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 5 (1.0 mg, 50% yield).

MS m/z (ESI): 533.9 [M+1].

$^1$H NMR (400 MHz, $CDCl_3$): δ 8.07 (s, 1H), 7.23-7.18 (m, 2H), 6.71-6.64 (m, 1H), 6.55-6.51 (m, 1H), 5.36-5.27 (m, 2H), 4.67-4.61 (m, 2H), 3.53-3.48 (m, 1H), 3.30-3.22 (m, 2H), 3.18-3.13 (m, 1H), 2.71-2.61 (m, 2H), 2.35-2.28 (m, 1H), 2.04-1.91 (m, 4H), 1.53-1.40 (m, 3H), 0.91-0.75 (m, 4H).

**Example 2-6**

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-(hydroxymethyl)c yclobutane-1-carboxamide **6**

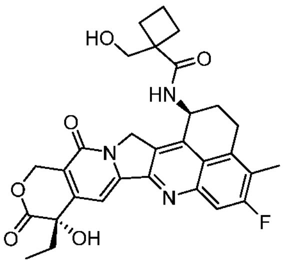

6

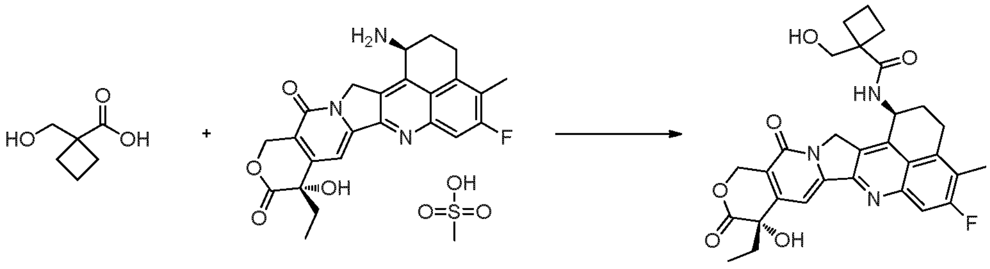

6a 1b 6

To **1b** (3.0 mg, 5.64 μmol) was added 1 mL of *N,N*-dimethylformamide. The mixture was cooled to 0-5 °C in an ice-water bath, followed by the addition of a drop of triethylamine. The resulting mixture was stirred until it became clear. To the reaction solution were successively added 1-(hydroxymethyl)cyclobutane-1-carboxylic acid **6a** (2.2 mg, 16.9 μmol, prepared according to the method disclosed in "Journal of the American Chemical Society, 2014, vol.136, #22, p.8138-8142") and 4-(4,6-dimethoxy-1,3,5-triazin-2-yl)-4-methylmorpholinium chloride (4.7 mg, 16.9 μmol). After the addition, the resulting mixture was stirred at 0-5 °C for 1 h. 5 mL of water was added to the reaction solution to quench the reaction, and the mixture was extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 6 (2.1 mg, 67.9% yield).

MS m/z (ESI): 548.0 [M+1].

$^1$H NMR (400 MHz, DMSO-$d_6$): δ 7.85-7.62 (m, 1H), 6.88 (br, 1H), 5.87-5.48 (m, 2H), 5.47-5.33 (m, 1H), 5.31-5.06 (m, 1H), 4.25-3.91 (m, 2H), 3.25 (br, 1H), 2.60-2.32 (m, 3H), 2.23 (t, 1H), 2.15-1.95 (m, 3H), 1.70-1.56 (m, 2H), 1.41-1.17 (m, 9H), 1.03 (s, 1H), 0.95-0.80 (m, 2H).

### Example 2-7

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycyclobuta ne-1-carboxamide **7**

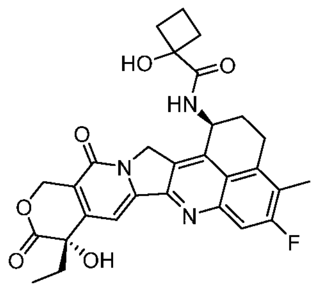

7

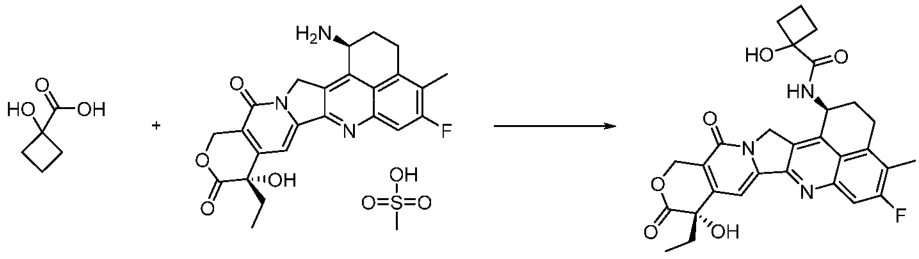

7a 1b 7

To **1b** (3.0 mg, 5.64 μmol) were added 2 mL of ethanol and 0.4 mL of *N,N*-dimethylformamide. The mixture was cooled to 0-5 °C in an ice-water bath, followed by the dropwise addition of 0.3 mL of *N*-methylmorpholine. The resulting mixture was stirred until it became clear. To the reaction solution were successively added 1-hydroxycyclobutanecarboxylic acid **7a** (2.0 mg, 17.22 μmol, supplied by PharmaBlock), 1-hydroxybenzotriazole (2.3 mg, 17.0 μmol) and 1-(3-dimethylaminopropyl)-3-ethylcarbodiimide hydrochloride (3.2 mg, 16.7 μmol). After the addition, the resulting mixture was stirred at 0-5 °C for 10 min. The ice-water bath was removed, and the reaction solution was stirred at room temperature for 2 h, and concentrated under reduced pressure. The resulting residue was purified by thin layer chromatography with developing solvent system B to give the title product 7 (2.5 mg, 83.1% yield).

MS m/z (ESI): 534.0 [M+1].

$^1$H NMR (400 MHz, DMSO-$d_6$): δ 8.28 (d, 1H), 7.75 (d, 1H), 7.29 (s, 1H), 6.51 (s, 1H), 6.12 (s, 1H), 5.59-5.51 (m, 1H), 5.41 (s, 2H), 5.20-5.01 (m, 2H), 3.27-3.17 (m, 1H), 3.15-3.05 (m, 1H), 2.71-2.63 (m, 1H), 2.37 (s, 3H), 2.12-2.05 (m, 1H), 2.03-1.94 (m, 2H), 1.92-1.78 (m, 4H), 1.50-1.42 (m, 1H), 0.90-0.83 (m, 4H).

## Example 2-8

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quino lin-1-yl)cyclopropane-1-carboxamide **8**

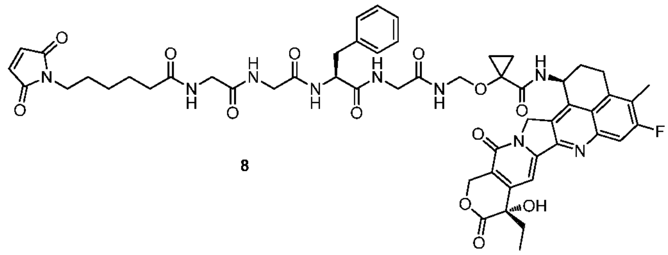

8

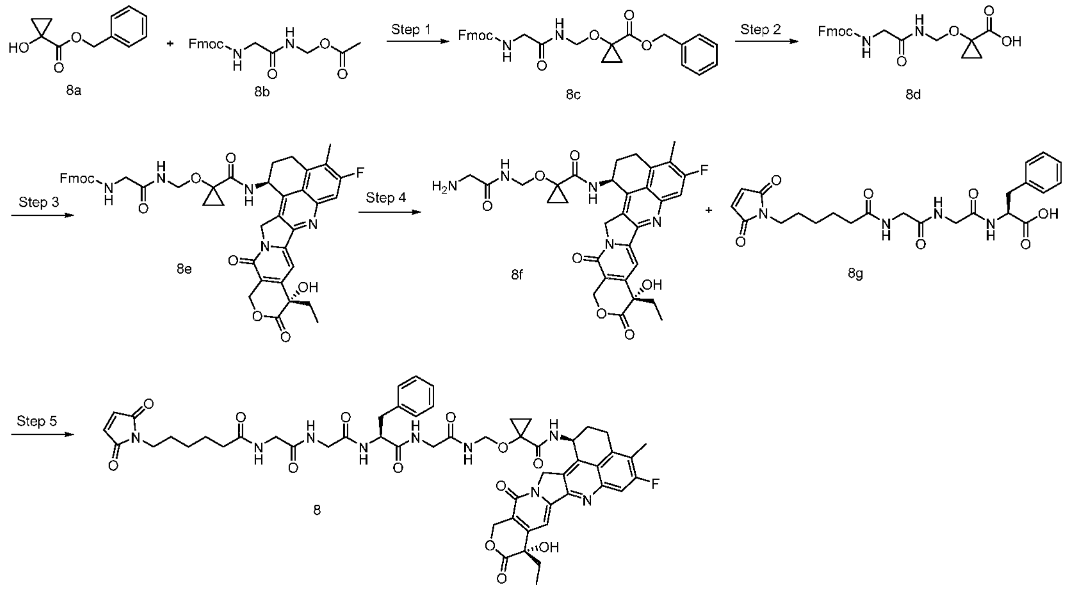


8a, 8b, 8c, 8d, 8e, 8f, 8g, 8

Step 1

Benzyl 1-((2-((((9*H*-fluoren-9-yl)methoxy)carbonyl)amino)acetylamino)methoxy)cyclopropane -1-carboxylate **8c**

To a reaction flask were added benzyl 1-hydroxycyclopropane-1-carboxylate **8a** (104 mg, 0.54 mmol; prepared according to the method disclosed in Patent Application "US2005/20645") and 2-((((9*H*-fluoren-9-yl)methoxy)carbonyl)amino)acetylamino)methyl acetate **8b** (100 mg, 0.27 mmol; prepared according to the method disclosed in Patent Application "CN105829346A"), followed by the addition of 5 mL of tetrahydrofuran. The mixture was purged with argon three times, and cooled to 0-5 °C in an ice-water bath, followed by the addition of potassium *tert*-butoxide (61 mg, 0.54 mmol). The ice bath was removed, and the resulting mixture was heated to room temperature and stirred for 10 min. 20 mL of ice water was added to the reaction solution, and the mixture was extracted with ethyl acetate (5 mL × 2) and chloroform (5 mL × 5). The organic phases were combined and concentrated. The resulting residue was dissolved in 3 mL of 1,4-dioxane, and 0.6 mL of water, sodium bicarbonate (27 mg, 0.32 mmol) and 9-fluorenylmethyl chloroformate (70 mg, 0.27 mmol) were added. The resulting mixture was stirred at room temperature for 1 h. 20 mL of water was added to the reaction solution, and the mixture was extracted with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product **8c** (100 mg, 73.6% yield).

MS m/z (ESI): 501.0 [M+1].

Step 2

1-((2-((((9*H*-fluoren-9-yl)methoxy)carbonyl)amino)acetylamino)methoxy)cyclopropane -1-carboxylic acid **8d**

**8c** (50 mg, 0.10 mmol) was dissolved in 3 mL of a solvent mixture of tetrahydrofuran and ethyl acetate (V:V = 2:1), and palladium on carbon (25 mg, 10% loading) was added. The mixture was purged with hydrogen three times and stirred at room temperature for 1 h. The reaction solution was filtered through celite, and the filter cake was rinsed with tetrahydrofuran. The filtrate was concentrated to give the title product **8d** (41 mg, 100% yield).

MS m/z (ESI): 411.0 [M+1].

Step 3

(9*H*-fluoren-9-yl)methyl(2-(((1-(((1*S*,9*S*)-9-ethyl-5-fluoro-9-hydroxy-4-methyl-10,13-di oxo-2,3,9,10,13,15-hexahydro-1*H*,12*H*-benzo[*de*]pyrano[3'4':6,7]indolizino[1,2-*b*]quin olin-1-yl)aminocarbonyl)cyclopropoxy)methyl)amino)-2-oxoethyl)carbamate **8e**

To a reaction flask was added **1b** (7 mg, 0.013 mmol), followed by the addition of 1 mL of *N,N*-dimethylformamide. The mixture was purged with argon three times, and cooled to 0-5 °C in an ice-water bath, followed by the addition of a drop of triethylamine, a solution of **8d** (7 mg, 0.017 mmol) in 0.5 mL of*N,N*-dimethylformamide, and 4-(4,6-dimethoxy-1,3,5-triazin-2-yl)-4-methylmorpholinium chloride (7 mg, 0.026 mmol). The resulting mixture was stirred in an ice bath for 35 min. 10 mL of water was added to the reaction solution, and the mixture was extracted with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **8e** (8.5 mg, 78.0% yield).

MS m/z (ESI): 828.0 [M+1].

Step 4

1-((2-Aminoacetylamino)methoxy)-*N*-((1*S*,9*S*)-9-ethyl-5-fluoro-9-hydroxy-4-methyl-10 ,13-dioxo-2,3,9,10,13,15-hexahydro-1*H*,12*H*-benzo[*de*]pyrano[3',4':6,7]indolizino[1,2-*b* ]quinolin-1-yl)cyclopropane-1-carboxamide **8f**

**8e** (4 mg, 4.84 μmol) was dissolved in 0.2 mL of dichloromethane, and 0.1 mL of diethylamine was added. The mixture was stirred at room temperature for 2 h, and concentrated under reduced pressure. 2 mL of toluene was added, and the mixture was concentrated under reduced pressure; the procedures were repeated twice. The residue was slurried with 3 mL of n-hexane, and the upper *n*-hexane layer was removed; the procedures were repeated three times. The slurry was concentrated under reduced pressure to give the crude title product **8f** (2.9 mg), which was directly used in the next step without purification.

MS m/z (ESI): 606.0 [M+1].

Step 5

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quino lin-1-yl)cyclopropane-1-carboxamide **8**

Crude **8f** (2.9 mg, 4.84 μmol) was dissolved in 0.5 mL of *N,N*-dimethylformamide. The mixture was purged with argon three times, and cooled to 0-5 °C in an ice-water bath, followed by the addition of a solution of (*S*)-2(-2-(-2-(6-(2,5-dioxo-1*H*-pyrrol-1-yl)hexanamido)acetylamino)acetylamino)-3-phe nylpropionic acid **8g** (2.7 mg, 5.80 μmol, prepared according to the method disclosed in Patent Application "EP2907824") in 0.3 mL of *N,N*-dimethylformamide and 4-(4,6-dimethoxy-1,3,5-triazin-2-yl)-4-methylmorpholinium chloride (2.7 mg, 9.67 μmol). The resulting mixture was stirred in an ice bath for 30 min. The ice bath was removed, and the reaction solution was heated to room temperature, stirred for 15 min, and purified by high performance liquid chromatography (separation conditions: chromatography column: XBridge Prep C18 OBD 5 μm 19 × 250 mm; mobile phase: A-water (10 mmol $NH_4OAc$), B-acetonitrile, gradient elution, flow rate: 18 mL/min). The corresponding fractions were collected and concentrated under reduced pressure to give the title product **8** (2 mg, 39.0% yield).

MS m/z (ESI): 1060.0 [M+1].

$^1$H NMR (400 MHz, DMSO-$d_6$): δ 9.01 (d, 1H), 8.77 (t, 1H), 8.21 (t, 1H), 8.08-7.92 (m, 2H), 7.73 (d, 1H), 7.28 (s, 1H), 7.24-7.07 (m, 4H), 6.98 (s, 1H), 6.50 (s, 1H), 5.61 (q, 1H), 5.40 (s, 2H), 5.32 (t, 1H), 5.12 (q, 2H), 4.62 (t, 1H), 4.52 (t, 1H), 4.40-4.32 (m, 1H), 3.73-3.47 (m, 8H), 3.16-3.04 (m, 2H), 2.89 (dd, 1H), 2.69-2.55 (m, 2H), 2.37-2.23 (m, 4H), 2.12-1.93 (m, 4H), 1.90-1.74 (m, 2H), 1.52-1.38 (m, 4H), 1.33-1.11 (m, 5H), 0.91-0.81 (m, 4H).

**Example 2-9**

*N*-((2*R,*10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3'4':6,7]indolizino[1, 2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-B**

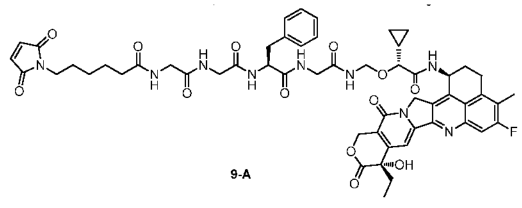

9-A

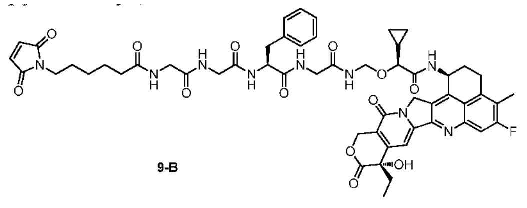

9-B

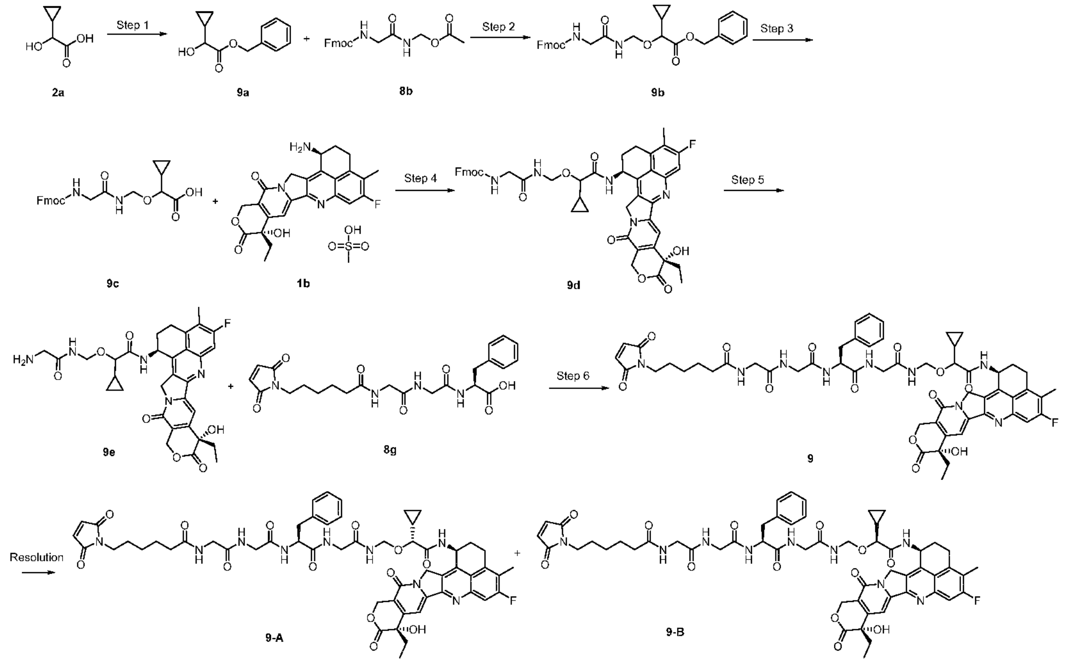


Step 1

Benzyl 2-cyclopropyl-2-hydroxyacetate **9a**

**2a** (1.3 g, 11.2 mmol; prepared according to the method disclosed in Patent Application "WO2013/106717") was dissolved in 50 mL of acetonitrile, and potassium carbonate (6.18 g, 44.8 mmol), benzyl bromide (1.33 mL, 11.2 mmol) and tetrabutylammonium iodide (413 mg, 1.1 mmol) were successively added. The mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **9a** (2 g, 86.9% yield).

Step 2

Benzyl 10-cyclopropyL-1-(9*H*-fluoren-9-yl)-3,6-dioxo-2,9-dioxa-4,7-diazaundecan-11-oate **9b**

To a reaction flask were added **9a** (120.9 mg, 0.586 mmol) and **8b** (180 mg, 0.489 mmol), followed by the addition of 4 mL of tetrahydrofuran. The reaction solution was purged with argon three times, and cooled to 0-5 °C in an ice-water bath, followed by the addition of potassium *tert*-butoxide (109 mg, 0.98 mmol). The ice bath was removed, the resulting mixture was heated to room temperature and stirred for 40 min. 10 mL of ice water was added to the reaction solution, and the mixture was extracted with ethyl acetate (20 mL × 2) and chloroform (10 mL × 5). The organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The resulting mixture was stirred at room temperature for 2 h. 20 mL of water was added to the reaction solution, and the mixture was extracted with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, 19% yield).
MS m/z (ESI): 515.0 [M+1].

Step 3

10-cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry) was added. The mixture was purged with hydrogen three times and stirred at room temperature for 1 h. The reaction solution was filtered through celite, and the filter cake was rinsed with ethyl acetate. The filtrate was concentrated to give the crude title product **9c** (13 mg), which was directly used in the next step without purification. MS m/z (ESI): 424.9 [M+1].

Step 4

(9*H*-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3'4':6,7]indoli zino[1,2-*b*]quinolin-1-yl)amino)-2-oxoethoxy)methyl)amino)-2-oxoethyl)carbamate **9d**

To a reaction flask was added **1b** (10 mg, 18.8 μmol), followed by the addition of 1 mL of *N,N*-dimethylformamide. The mixture was purged with argon three times, and cooled to 0-5 °C in an ice-water bath, followed by the addition of a drop of triethylamine, crude product **9c** (13 mg, 30.6 μmol), and 4-(4,6-dimethoxy-1,3,5-triazin-2-yl)-4-methylmorpholinium chloride (16.9 mg, 61.2 μmol). The resulting mixture was stirred in an ice bath for 40 min. 10 mL of water was added to the reaction solution, and the mixture was extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **9d** (19 mg, 73.6% yield).
MS m/z (ESI): 842.1 [M+1].

Step 5

2-((2-Aminoacetylamino)methoxy)-2-cyclopropyl-*N*-((1*S*,9*S*)-9-ethyl-5-fluoro-9-hydro xy-4-methyl-10,13-dioxo-2,3,9,10,13,15-hexahydro-1*H*,12*H*-benzo[*de*]pyrano[3'4':6,7] indolizino[1,2-*b*]quinolin-1-yl)acetamide **9e**

**9d** (19 mg, 22.6 μmol) was dissolved in 2 mL of dichloromethane, and 1 mL of diethylamine was added. The mixture was stirred at room temperature for 2 h, and concentrated under reduced pressure. 1 mL of toluene was added, and the resulting mixture was concentrated under reduced pressure; the procedures were repeated twice.
The residue was slurried with 3 mL of n-hexane and left to stand. Then, the supernatant was removed, and the solid was kept. The solid residue was concentrated under reduced pressure and dried using an oil pump to give the crude title product **9e** (17 mg), which was directly used in the next step without purification.
MS m/z (ESI): 638.0 [M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3'4':6,7]indolizino[1, 2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3'4':6,7]indolizino[1, 2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-B**

Crude **9e** (13.9 mg, 22.4 μmol) was dissolved in 0.6 mL of *N*,*N*-dimethylformamide. The mixture was purged with argon three times, and cooled to 0-5 °C in an ice-water bath, followed by the addition of a solution of **8g** (21.2 mg, 44.8 μmol) in 0.3 mL of *N*,*N*-dimethylformamide and 4-(4,6-dimethoxy-1,3,5-triazin-2-yl)-4-methylmorpholinium chloride (18.5 mg, 67.3 μmol). The resulting mixture was stirred in an ice bath for 10 min. Then, the ice bath was removed, and the reaction solution was heated to room temperature and stirred for 1 h to give compound **9.** The reaction solution was purified by high performance liquid chromatography (separation conditions: chromatography column: XBridge Prep C18 OBD 5 μm 19 × 250 mm; mobile phase: A-water (10 mmol $NH_4OAc$), B-acetonitrile, gradient elution, flow rate: 18 mL/min). The corresponding fractions were collected and concentrated under reduced pressure to give the title product (9-A: 2.4 mg, 9-B: 1.7 mg).

MS m/z (ESI): 1074.4 [M+1].

Single-configuration compound 9-A (shorter retention time):

UPLC analysis: retention time: 1.14 min; purity: 85% (chromatography column: ACQUITY UPLC BEH C18 1.7 μm 2.1 × 50 mm; mobile phase: A-water (5 mmol $NH_4OAc$), B-acetonitrile).

$^1$H NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time):

UPLC analysis: retention time: 1.16 min; purity: 89% (chromatography column: ACQUITY UPLC BEH C18 1.7 μm 2.1 × 50 mm; mobile phase: A-water (5 mmol $NH_4OAc$), B-acetonitrile).

$^1$H NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

**Example 3. Preparation of ADC**

Drug loading analysis of ADC

Experimental objective and principle

The ADC loading was determined by ultraviolet spectrophotometry (UV-Vis). Instrument: Thermo nanodrop2000 ultraviolet spectrophotometer. The principle is that the total absorbance value of the ADC at a certain wavelength is equal to the sum of the absorbance values of the drug and the monoclonal antibody at that wavelength.

Experimental procedures

Cuvettes containing a sodium succinate buffer were separately placed into the reference cell and sample cell, and the absorbance value of the solvent blank was subtracted. Then, a cuvette containing the test solution was placed into the sample cell, and the absorbance values at 280 nm and 370 nm were determined.

Calculation for results:

$$(1)\ A_{280\ nm} = \varepsilon_{mab\text{-}280} b C_{mab} + \varepsilon_{Drug\text{-}280} b C_{Drug}$$

$\varepsilon_{Drug\text{-}280}$: the mean molar extinction coefficient of the drug at 280 nm of 5100;
$C_{Drug}$: the concentration of the drug;
$\varepsilon_{mab\text{-}280}$: the mean molar extinction coefficient of the monoclonal antibody at 280 nm of 214,600;
$C_{mab}$: the concentration of the monoclonal antibody;
b: the optical path length of 1 cm.

Similarly, an equation for the total absorbance value of the sample at 370 nm can be given as:

$$(2)\ A_{370\ nm} = \varepsilon_{mab\text{-}370} b C_{mab} + \varepsilon_{Drug\text{-}370} b C_{Drug}$$

$\varepsilon_{Drug\text{-}370}$: the mean molar extinction coefficient of the drug at 370 nm of 19,000;
$C_{Drug}$: the concentration of the drug;
$\varepsilon_{mab\text{-}370}$: the extinction coefficient of the monoclonal antibody at 370nm of 0;
$C_{mab}$: the concentration of the monoclonal antibody;
b: the optical path length of 1 cm.

The drug loading of ADC can be calculated using both equations (1) and (2) as well as the extinction coefficients of the monoclonal antibody and the drug at both wavelengths and their concentrations.

$$\text{Drug loading} = C_{Drug}/C_{mab}.$$

Preparation examples of PSMA antibody-drug conjugate PM-9-A with different DAR values

The following examples are the preparation processes of related ADCs of the present disclosure. In Examples 3-4, 3-5, 3-7, and 3-11, the antibody **PM** was subjected to a coupling reaction with the drug **9-A** with a linking unit via a mercapto group on cysteine to obtain an antibody-drug conjugate PM-9-A (DAR = about 3-4); in Example 3-1 to Example 3-3 as well as Example 3-6, the antibody **PM** was subjected to a coupling reaction with the drug **9-A** with a linking unit via a mercapto group on cysteine to obtain an PSMA ADC molecule PM-9-A (DAR = about 6-7); in Examples 3-8 to 3-10, the antibody **PM** was subjected to a reaction with the drug VcMMAE with a linking unit (Biochempartner, CAS 646502-53-6) via a mercapto group on cysteine to obtain a conjugate molecule PM-VcMMAE as a control.

By adjusting the ratio of antibody to drug, the reaction scale, and other conditions, the antibody-drug conjugate with different DAR values (n), preferably DAR values of 1 to 8, more preferably 3 to 8, and most preferably 3 to 7, can be obtained.

(I) Preparation of antibody-drug conjugate PM-9-A with different DAR values

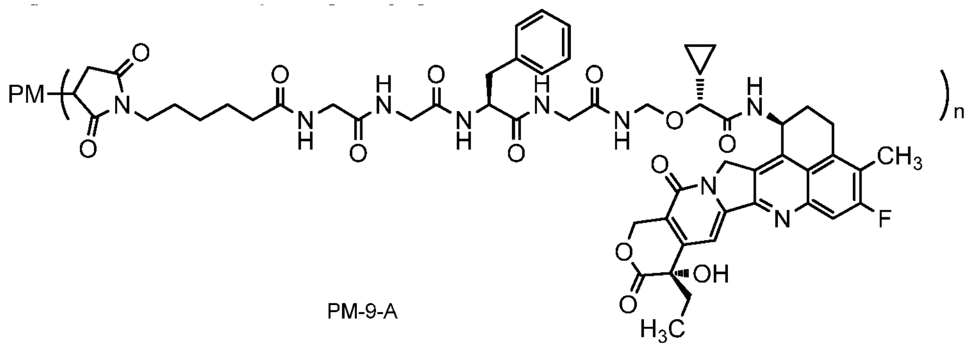

PM-9-A

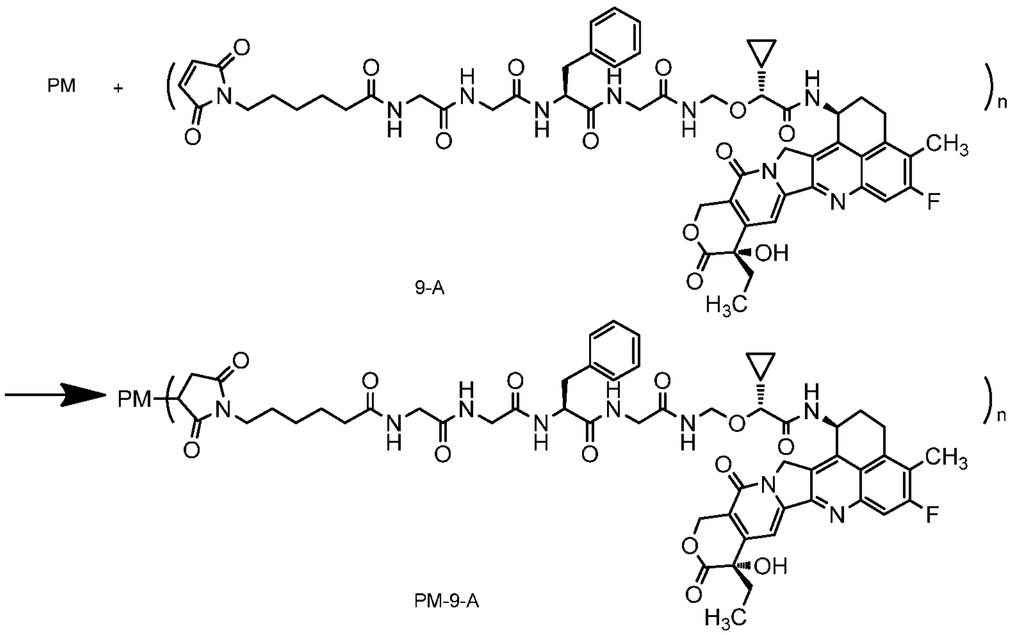

9-A

PM-9-A

### Example 3-1. ADC-1

To antibody PM in aqueous PBS buffer (0.05 M aqueous PBS buffer at pH 6.5; 10.0 mg/mL, 0.27 mL, 18 nmol) was added a prepared aqueous solution of tris(2-carboxyethyl)phosphine (TCEP) (10 mM, 9.8 μL, 98 nmol) at 37 °C. The mixture was shaken on a water bath shaker at 37 °C for 3 h before the reaction was terminated. The reaction solution was cooled to 25 °C in a water bath.

Compound **9-A** (0.3 mg, 277 nmol) was dissolved in 20 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-1** of the conjugate of formula PM-9-A in PBS buffer (0.18 mg/mL, 11 mL), which was then stored at 4 °C.

Calculation of mean value by UV-Vis: n = 6.31.

### Example 3-2. ADC-2

To antibody **PM** in aqueous PBS buffer (0.05 M aqueous PBS buffer at pH 6.5; 10.0 mg/mL, 3.6 mL, 243 nmol) was added a prepared aqueous solution of tris(2-carboxyethyl)phosphine (TCEP) (10 mM, 128.9 μL, 1289 nmol) at 37 °C. The mixture was shaken on a water bath shaker at 37 °C for 3 h before the reaction was terminated. The reaction solution was cooled to 25 °C in a water bath.

Compound **9-A** (3.93 mg, 3649 nmol) was dissolved in 200 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-2** of the conjugate of formula PM-9-A in PBS buffer (1.93 mg/mL, 15.4 mL), which was then stored at 4 °C.

Calculation of mean value by UV-Vis: n = 6.63.

### Example 3-3. ADC-3

To antibody **PM** in aqueous PBS buffer (0.05 M aqueous PBS buffer at pH 6.5; 10.0 mg/mL, 10 mL, 676 nmol) was added a prepared aqueous solution of tris(2-carboxyethyl)phosphine (TCEP) (10 mM, 358.1 μL, 3581 nmol) at 37 °C. The mixture was shaken on a water bath shaker at 37 °C for 3 h before the reaction was terminated. The reaction solution was cooled to 25 °C in a water bath.

Compound **9-A** (10.91 mg, 10135 nmol) was dissolved in 480 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-3**

of the conjugate of formula PM-9-A in PBS buffer (3.47 mg/mL, 25 mL), which was then stored at 4 °C.

Calculation of mean value by UV-Vis: n = 6.9.

**Example 3-4. ADC-4**

To antibody PM in aqueous PBS buffer (0.05 M aqueous PBS buffer at pH 6.5; 10.0 mg/mL, 0.21 mL, 14 nmol) was added a prepared aqueous solution of tris(2-carboxyethyl)phosphine (TCEP) (10 mM, 3.5 μL, 35 nmol) at 37 °C. The mixture was shaken on a water bath shaker at 37 °C for 3 h before the reaction was terminated. The reaction solution was cooled to 25 °C in a water bath.

Compound **9-A** (0.15 mg, 139 nmol) was dissolved in 20 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-4** of the conjugate of formula PM-9-A in PBS buffer (0.28 mg/mL, 4.6 mL), which was then stored at 4 °C.

Calculation of mean value by UV-Vis: n = 3.68.

**Example 3-5. ADC-5**

To antibody PM in aqueous PBS buffer (0.05 M aqueous PBS buffer at pH 6.5; 10.0 mg/mL, 5.23 mL, 353 nmol) was added a prepared aqueous solution of tris(2-carboxyethyl)phosphine (TCEP) (10 mM, 84.8 μL, 848 nmol) at 37 °C. The mixture was shaken on a water bath shaker at 37 °C for 3 h before the reaction was terminated. The reaction solution was cooled to 25 °C in a water bath.

Compound **9-A** (3.8 mg, 3534 nmol) was dissolved in 260 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-5** of the conjugate of formula PM-9-A in PBS buffer (2.48 mg/mL, 18.2 mL), which was then stored at 4 °C.

Calculation of mean value by UV-Vis: n = 3.89.

**Example 3-6. ADC-6**

To antibody PM in aqueous PBS buffer (0.05 M aqueous PBS buffer at pH 6.5; 10.0 mg/mL, 3.5 mL, 236 nmol) was added a prepared aqueous solution of tris(2-carboxyethyl)phosphine (TCEP) (10 mM, 125.3 μL, 1253 nmol) at 37 °C. The mixture was shaken on a water bath shaker at 37 °C for 3 h before the reaction was terminated. The reaction solution was cooled to 25 °C in a water bath.

Compound **9-A** (3.82 mg, 3547 nmol) was dissolved in 150 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-6** of the conjugate of formula PM-9-A in PBS buffer (1.83 mg/mL, 14 mL), which was then stored at 4 °C.

Calculation of mean value by UV-Vis: n = 6.61.

**Example 3-7. ADC-7**

To antibody PM in aqueous PBS buffer (0.05 M aqueous PBS buffer at pH 6.5; 10.0 mg/mL, 14.68 mL, 992 nmol) was added a prepared aqueous solution of tris(2-carboxyethyl)phosphine (TCEP) (10 mM, 238.1 μL, 2381 nmol) at 37 °C. The mixture was shaken on a water bath shaker at 37 °C for 3 h before the reaction was terminated. The reaction solution was cooled to 25 °C in a water bath.

Compound **9-A** (10.67 mg, 9919 nmol) was dissolved in 420 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-7** of the conjugate of formula PM-9-A in PBS buffer (3.61 mg/mL, 37 mL), which was then stored at 4 °C.

Calculation of mean value by UV-Vis: n = 3.93.

(II) Preparation of control antibody-drug conjugate PM-VcMMAE (referring to WO2007002222A2)

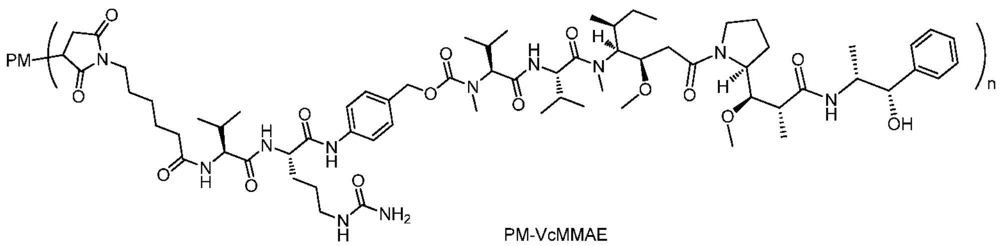

PM-VcMMAE

### Example 3-8. ADC-8

To antibody PM in aqueous PBS buffer (0.05 M aqueous PBS buffer at pH 6.5; 10.0 mg/mL, 2.5 mL, 169 nmol) was added a prepared aqueous solution of tris(2-carboxyethyl)phosphine (TCEP) (10 mM, 42.2 μL, 422 nmol) at 37 °C. The mixture was shaken on a water bath shaker at 37 °C for 3 h before the reaction was terminated. The reaction solution was cooled to 25 °C in a water bath.

Compound **VcMMAE** (2.22 mg, 1689 nmol) was dissolved in 100 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-8** of the conjugate of formula PM-VcMMAE in PBS buffer (1.76 mg/mL, 12 mL), which was then stored at 4 °C. Calculation of mean value by CE-SDS: n = 4.47.

### Example 3-9. ADC-9

To antibody PM in aqueous PBS buffer (0.05 M aqueous PBS buffer at pH 6.5; 10.0 mg/mL, 3.3 mL, 223 nmol) was added a prepared aqueous solution of tris(2-carboxyethyl)phosphine (TCEP) (10 mM, 55.7 μL, 557 nmol) at 37 °C. The mixture was shaken on a water bath shaker at 37 °C for 3 h before the reaction was terminated. The reaction solution was cooled to 25 °C in a water bath.

Compound **VcMMAE** (2.93 mg, 2230 nmol) was dissolved in 200 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-9** of the conjugate of formula PM-VcMMAE in PBS buffer (2.05 mg/mL, 17 mL), which was then stored at 4 °C.

Calculation of mean value by CE-SDS: n = 4.23.

### Example 3-10. ADC-10

To antibody PM in aqueous PBS buffer (0.05 M aqueous PBS buffer at pH 6.5; 10.0 mg/mL, 2.5 mL, 169 nmol) was added a prepared aqueous solution of tris(2-carboxyethyl)phosphine (TCEP) (10 mM, 42.2 μL, 422 nmol) at 37 °C. The mixture was shaken on a water bath shaker at 37 °C for 3 h before the reaction was terminated. The reaction solution was cooled to 25 °C in a water bath.

Compound **VcMMAE** (2.22 mg, 1689 nmol) was dissolved in 150 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-10** of the conjugate of formula PM-VcMMAE in PBS buffer (2.2 mg/mL, 13 mL), which was then stored at 4 °C.

Calculation of mean value by CE-SDS: n = 3.92.

### Example 3-11. ADC-11

To antibody PM in aqueous PBS buffer (0.05 M aqueous PBS buffer at pH 6.5; 10.0 mg/mL, 2.4 mL, 160 nmol) was added a prepared aqueous solution of tris(2-carboxyethyl)phosphine (TCEP) (10 mM, 42.2 μL, 422 nmol) at 37 °C. The mixture was shaken on a water bath shaker at 37 °C for 3 h before the reaction was terminated. The reaction solution was cooled to 25 °C in a water bath.

Compound **9-A** (1.75 mg, 1629 nmol) was dissolved in 100 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give an exemplary product **ADC-11** of the

conjugate of formula PM-9-A in PBS buffer (1.34 mg/mL, 15.5 mL), which was then stored at 4 °C.

Calculation of mean value by UV-Vis: n = 4.19.

**Example 3-12. ADC-12**

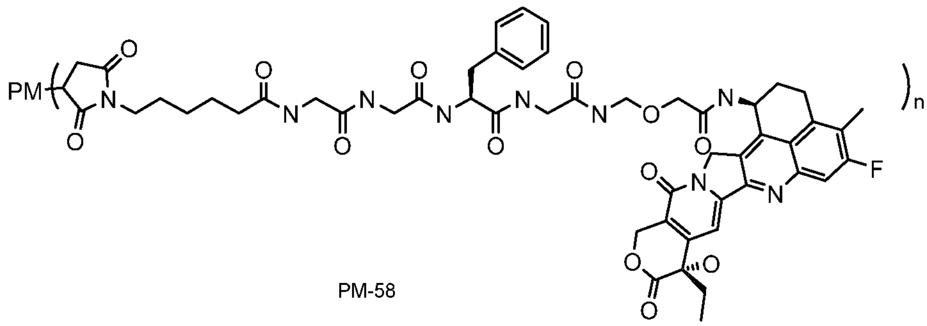

PM-58

To antibody PM in aqueous PBS buffer (0.05 M aqueous PBS buffer at pH 6.5; 10.0 mg/mL, 2.4 mL, 160 nmol) was added a prepared aqueous solution of tris(2-carboxyethyl)phosphine (TCEP) (10 mM, 42.2 μL, 422 nmol) at 37 °C. The mixture was shaken on a water bath shaker at 37 °C for 3 h before the reaction was terminated. The reaction solution was cooled to 25 °C in a water bath.

Compound **58** (prepared by referring to Example 58 on page 163 of Patent CN104755494A, 1.68 mg, 1624 nmol) was dissolved in 100 μL of dimethylsulfoxide, and the resulting solution was added to the above reaction mixture, which was then shaken on a water bath shaker at 25 °C for 3 h before the reaction was terminated. The reaction solution was desalted and purified through a Sephadex G25 gel column (elution phase: 0.05 M PBS buffer at pH 6.5, containing 0.001 M EDTA) to give an exemplary product **ADC-12** of formula PM-58 in PBS buffer (1.45 mg/mL, 14.8 mL), which was then stored at 4 °C.

Calculation of mean value by UV-Vis: n = 4.16.

(III) Preparation of control antibody-drug conjugate Lmab-9-A

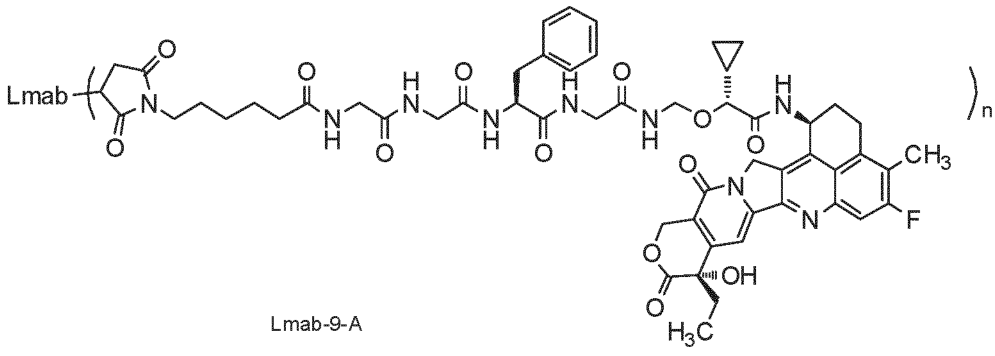

Lmab-9-A

To antibody **Lmab** in aqueous PBS buffer (0.05 M aqueous PBS buffer at pH 6.5; 10.0 mg/mL, 2.15 mL, 145 nmol) was added a prepared aqueous solution of tris(2-carboxyethyl)phosphine (TCEP) (10 mM, 74.1 μL, 741 nmol) at 37 °C. The mixture was shaken on a water bath shaker at 37 °C for 3 h before the reaction was terminated. The reaction solution was cooled to 25 °C in a water bath.

Compound **9-A** (3.20 mg, 2179 nmol) was dissolved in 155 μL of dimethylsulfoxide, and the solution was added to the above reaction solution. The resulting mixture was shaken on a water bath shaker at 25 °C for 3 h before the reaction was terminated. The reaction solution was desalted and purified through a Sephadex G25 gel column (elution phase: 0.05 M aqueous PBS buffer at pH 6.5, containing 0.001 M EDTA) to give the title product Lmab-9-A in PBS buffer (0.99 mg/mL, 15 mL), which was then stored at 4 °C.

Calculation of mean value by UV-Vis: n = 7.07.

### Verification of activity of the antibodies of the present disclosure with biochemical test methods

### Test example 1. *In vitro* cell binding assay

In this experiment, fluorescence signals of antibodies on the cell surface were detected, and the binding of the antibodies was evaluated according to the intensity of the fluorescence signals. The prepared ADC-2, ADC-10, control ADC Lmab-9-A and antibody PM were used together for the *in vitro* binding assay.

The ADC-2, ADC-10 and antibody PM diluted in a gradient were separately incubated with $1\times10^5$ cells (ATCC, MDA PCa 2b/CRL-2422, LNCaP/CRL-1740, 22Rv1/CRL-2505, PC-3/CRL-1435, DU 145/HTB-81) at 4 °C for 60 min, and then excess ADCs or antibodies were washed off. The cells were incubated with an FITC-labeled goat anti-human IgG (H + L) secondary antibody (Jackson Immuno Research, 109-095-003) at 4 °C for 30 min, and then excess antibodies were washed off. The fluorescence signals on the cell surface were read using BD CantoII. The results are shown in Table 2, FIG. 1A, FIG. 1B, FIG. 1C, FIG. 1D, and FIG. 1E.

Table 2. *In vitro* cell binding activity ($EC_{50}$, nM)

| | MDAPCa 2b | LNCaP | 22RV1 | PC-3 | DU145 |
|---|---|---|---|---|---|
| Antibody PM | 1.079 | 1.421 | 0.6761 | No binding | No binding |
| ADC-2 | 0.7183 | 0.9381 | 0.5714 | No binding | No binding |
| ADC-10 | 0.9537 | 1.581 | 0.6894 | No binding | No binding |
| Lmab-9-A | No binding | No binding | No binding | No binding | No binding |

Expression level of antigen PSMA of the cells used for detection: MDA PCa 2b > LNCaP >22Rv1; PC-3 and DU 145 did not express PSMA.

The results show that ADC-2, ADC-10, control ADCLmab-9-A and antibody PM have correspondingly strong or weak binding ability to cells with different expression levels of PSMA antigen. Smaller $EC_{50}$ represents greater binding ability.

### Test example 2. *In vitro* endocytosis assay

DT3C, 70 kd, is a recombinantly expressed fusion protein formed by fusing fragment A of diphtheria toxin (toxin portion only) and fragment 3C of group G streptococcus (IgG binding portion). The protein can have a high affinity for IgG portion of antibody, enter cells together with the IgG portion when the antibody is endocytosed, and release toxic DT under the action of intracellular furin protease. The DT can inhibit the activity of EF2-ADP ribosylation, block the protein translation process and finally cause cell death. DT3C that does not enter the cell has no activity of cell killing. The endocytic activity to the antibodies is evaluated based on cell killing.

Sterile filtered DT3C (70 KD, SB HRS3A, MEI3AU0803) and antibody PM antibody (DT3C, with a molar concentration being 6 times the antibody molar concentration) were well mixed in a volume ratio of 1:1. The mixture was incubated at room temperature for 30 min, diluted in a gradient with a serum-free medium, added to cells (ATCC, LNCaP/CRL-1740, 22Rv1/CRL-2505) (2000 cells/well) cultured in a medium containing 20% low IgG FBS prepared one day in advance, and incubated in a 5% carbon dioxide incubator at 37 °C for three days. CellTiter-Glo (Promega, G7573) was added, and the mixture was incubated at room temperature for 10 min away from the light. The chemiluminescence was read on Victor3. The results are shown in Table 3, FIG. 2A, and FIG. 2B.

Table 3. Endocytic activity of different cells to the antibodies ($IC_{50}$, nM)

| | LNCaP | 22RV1 |
|---|---|---|
| Antibody PM + DT3C | ~ 4.641e+012 | 0.08563 |
| hIgG1 control + DT3C | No endocytosis | No endocytosis |
| DT3C only | No endocytosis | No endocytosis |

The results show that the antibody PM can be endocytosed on cells positively expressing PSMA antigen.

**Test example 3. Cell proliferation inhibition assay**

In this experiment, the content of ATP in the cells was detected, and the inhibitory effect of the PSMA ADC on the proliferation of cells (ATCC, LNCaP/CRL-1740, 22Rv1/CRL-2505 and PC-3/CRL-1435) was evaluated according to the $IC_{50}$ values. Test samples: ADC-2, ADC-4, and control ADC Lmab-9-A

1. Inhibitory effect on proliferation of LNCaP cells

LNCaP cells were cultured in an RPMI-1640 medium containing 10% FBS, and passaged 2-3 times a week in a passage ratio of 1:3 or 1:6. During passaging, the medium was removed by pipetting, and the cell layer was rinsed with 5 mL of 0.25% pancreatin. Then the pancreatin was removed by pipetting, the cells were digested in an incubator for 3-5 min, and then a fresh medium was added to resuspend cells. To a 96-well culture plate were added 180 μL of cell suspension at a density of $2\times10^3$ cells/well and an RPMI-1640 medium containing 4.5% FBS, and to the periphery of the 96-well plate was only added the medium. The plate was incubated in an incubator for 24 h (37 °C, 5% $CO_2$).

The test ADCs (ADC-2, ADC-4, and Lmab-9-A) were prepared into an initial concentration, and diluted in a gradient dilution ratio of 1:6 with PBS to obtain 9 concentration points, and an additional 0 concentration point was set. Then 20 μL of the dilution was added to the above cell plate, and incubated in an incubator (37 °C, 5% $CO_2$) for 5 days. The compound 2-B (i.e., free toxin of 9-A) was diluted into a stock solution with DSMO, which was then prepared into an initial concentration of 200 μM with PBS, and diluted in a gradient dilution ratio of 1:6 with PBS to obtain 9 concentration points, and an additional 0 concentration point was set. Then 1 μL of the dilution was added to 20 μL of RPMI-1640, and the mixture was transferred to a 96-well culture plate, and incubated in an incubator (37 °C, 5% $CO_2$) for 5 days. In the 96-well culture plate, 80 μL of CellTiter-Glo reagent was added to each well, and the plate was incubated at room temperature for 10-15 min away from the light. The chemiluminescence signal values were read on Victor3, and the data were processed using GraphPad software. The results are shown in Table 4, FIG. 3A, and FIG. 3B.

2. Inhibitory effect on proliferation of 22Rv1 cells

22Rv1 cells were cultured in an RPMI-1640 medium containing 10% FBS, and passaged twice a week in a passage ratio of 1:3 or 1:6. During passaging, the medium was removed by pipetting, and the cell layer was rinsed with 5 mL of 0.25% pancreatin. Then the pancreatin was removed by pipetting, the cells were digested in an incubator for 3-5 min, and then a fresh medium was added to resuspend cells. To a 96-well culture plate were added 180 μL of cell suspension at a density of $4\times10^3$ cells/well and an RPMI-1640 medium containing 4.5% FBS, and to the periphery of the 96-well plate was only added the medium. The plate was incubated in an incubator for 24 h (37 °C, 5% $CO_2$). The preparation and experimental methods of the test ADCs and the compound 2-B were the same as above. The results are shown in Table 4, FIG. 3C, and FIG. 3D.

3. Inhibitory effect on proliferation of PC-3 cells

PC-3 cells were cultured in an F-12K medium containing 10% FBS, and passaged 2-3 times a week in a passage ratio of 1:3 or 1:6. During passaging, the medium was removed by pipetting, and the cell layer was rinsed with 5 mL of 0.25% pancreatin. Then the pancreatin was removed by pipetting, the cells were digested in an incubator for 3-5 min, and then a fresh medium was added to resuspend cells. To a 96-well culture plate were added 180 μL of cell suspension at a density of $4\times10^3$ cells/well and an F-12K medium containing 4.5% FBS, and to the periphery of the 96-well plate was only added the medium. The plate was incubated in an incubator for 24 h (37 °C, 5% $CO_2$). The preparation and experimental methods of the test ADCs and the compound 2-B were the same as above. The results are shown in Table 4, FIG. 3E, and FIG. 3F.

Table 4. Effect on cell killing ($IC_{50}$, nM)

| | Compound 2-B | ADC-2 | ADC-4 | Lmab-9-A |
|---|---|---|---|---|
| LNCaP | 1.695 | 0.7988 | 4.67 | ~ 7.751e+006 |
| 22RV 1 | 2.843 | 1.238 | 78.34 | ~ 3.509e+007 |
| PC-3 | 8.328 | ~ 1.256e+006 | ~ 3.140e+006 | ~ 529.9 |

Conclusion: the PSMAADCs have killing effects on LNCaP and 22RV1 cells. Compound 2-B (i.e., the free toxin of 9-A) has a transmembrane killing effect.

**Test example 4. Evaluation of efficacy of ADC drugs on human prostate cancer cell 22Rv1-induced xenograft tumor in nude mice**

I. Test method

Male nu/nu nude mice aged 6-8 weeks (purchased from Beijing Vital River Laboratory Animal Technology Co., Ltd, certificate No. 1908120082) were used in this experiment. Housing environment: SPF grade. Nude mice were inoculated subcutaneously with human prostate cancer cells 22Rv1 (Chinese Academy of Sciences). When the mean tumor volume reached 220 $mm^3$, animals were randomly grouped (D0) with 6 animals in each group, administered by intraperitoneal injection twice a week for a total of 5 times. The tumor volume and body weight were measured twice a week, and the data were recorded.

Tumor volume $V = 1/2 \times a \times b^2$, where a and b represent length and width, respectively. Relative tumor proliferation rate T/C(%) = (T - T0)/(C - C0) × ×100, where T and C are the tumor volume of animals at the end of the experiment in the treatment group and control group, respectively; T0 and C0 are the tumor volume of animals at the beginning of the experiment in the treatment group and control group, respectively.

$$\text{Tumor inhibition rate TGI (\%)} = 1 - \text{T/C (\%)}.$$

II. Test object

ADC-10: 3 mpk, 10 mpk;
ADC-2: 3 mpk, 10 mpk;
Blank control group: PBS buffer at pH 7.4.

III. Tumor inhibition effect of antibody ADCs

On day 17 (D17) after the treatment, it was observed that the tumor inhibition rates of positive ADC-10 at 10 mpk and 3 mpk were 48.9% and 10.0%, respectively, and the tumor inhibition rates of ADC-2 at 10 mpk and 3 mpk were > 100% and 91.5%, respectively. They were significantly superior to the blank control group and the positive ADC group, and exhibited a good dose-dependent relationship (Table 5 and FIG. 4A).

The body weight of the mice was stable in the treatment process, suggesting that each administration dose of ADC-2 has no significant toxic side effects (FIG. 4B).

Table 5. Efficacy of ADCs on the 22Rv1-induced xenograft tumor in tumor-bearing nude mice after the administration

| Group | Mean tumor volume (**mm$^3$**) | | Mean tumor volume (**mm$^3$**) | | Tumor inhibition rate |
|---|---|---|---|---|---|
| | D0 | SEM | D17 | SEM | D17 |
| Blank control | 221.8 | 15.2 | 1790.3 | 246.9 | - |
| ADC-10, 10mpk | 227.6 | 17.7 | 1028.6* | 203.9 | 48.9% |
| ADC-10, 3mpk | 221.9 | 16.7 | 1632.9 | 223 | 10.0% |
| ADC-2, 10mpk | 221.2 | 14.3 | 40.6*** ### | 8.2 | >100% |
| ADC-2, 3mpk | 219.4 | 14.4 | 352.3*** ### | 33.8 | 91.5% |
| vs. blank control group: * p < 0.05, *** p < 0.001; vs. corresponding dose groups of ADC-10: ### p < 0.001 | | | | | |

**Test example 5. Evaluation of efficacy of ADC drugs on human prostate cancer cell 22Rv1-induced xenograft tumor in nude mice**

I. Test method

Male nu/nu nude mice aged 6-8 weeks (purchased from Beijing Vital River Laboratory Animal Technology Co., Ltd, certificate No. 1908120082) were used in this experiment. Housing environment: SPF grade. Nude mice were inoculated subcutaneously with human prostate cancer cells 22Rv1 (Chinese Academy of Sciences). When the mean

tumor volume reached 210 $mm^3$, animals were randomly grouped (D0) with 8 animals in each group, administered by intraperitoneal injection twice a week for a total of 6 times. The tumor volume and body weight were measured twice a week, and the data were recorded.

Tumor volume V = 1/2 × a × $b^2$, where a and b represent length and width, respectively. Relative tumor proliferation rate T/C(%) = (T - T0)/(C - C0) × ×100, where T and C are the tumor volume of animals at the end of the experiment in the treatment group and control group, respectively; T0 and C0 are the tumor volume of animals at the beginning of the experiment in the treatment group and control group, respectively.

$$\text{Tumor inhibition rate TGI } (\%) = 1 - \text{T/C } (\%).$$

II. Test object

ADC-8: 10 mpk;
ADC-6: 3mpk, 6mpk;
ADC-7: 3 mpk, 6 mpk, 10 mpk;
Blank control group: PBS buffer at pH 7.4.

III. Tumor inhibition effect of antibody ADCs

On day 14 (D14) after the treatment, it was observed that the tumor inhibition rate of positive ADC-8 at 10 mpk was 81.6%, the tumor inhibition rates of test ADC-6 at 6 mpk and 3 mpk were > 100% and 97.8%, respectively, and the tumor inhibition rates of another test ADC-7 at 10 mpk, 6 mpk and 3 mpk were > 100%, 88.4% and 80.5%, respectively. All administration groups were significantly superior to the control group, the test ADC-6 was significantly superior to the test ADC-7 at an equal dose, and the two test ADCs both exhibited a good dose-dependent relationship (Table 6 and FIG. 5A).

The body weight of the mice was stable in the treatment process, suggesting that each administration dose of each test antibody has no significant toxic side effects (FIG. 5B).

Table 6. Efficacy of ADCs on the 22Rv1-induced xenograft tumor in tumor-bearing nude mice after the administration

| Group | Mean tumor volume (**mm³**) | | Mean tumor volume (**mm³**) | | Tumor inhibition rate | Mean tumor volume (**mm³**) | |
|---|---|---|---|---|---|---|---|
| | D0 | SEM | D14 | SEM | D14 | D30 | SEM |
| Control | 214.6 | 12.7 | 1931.3 | 232.4 | - | - | - |
| ADC-8,10mpk | 211.4 | 19.8 | 526.6*** | 52.5 | 81.6% | 1596.7 | 147.3 |
| ADC-6, 6mpk | 212.2 | 15.4 | 108.2*** | 26.2 | >100% | 234.0 | 99.5 |
| ADC-6, 3mpk | 210.8 | 19.6 | 245.7*** | 20.2 | 97.8% | 420.4 | 41.1 |
| ADC-7, 10mpk | 215.6 | 22.4 | 183.1*** | 31.4 | >100% | 251.9 | 70.5 |
| ADC-7, 6mpk | 217.5 | 18.4 | 416.4***### | 59.4 | 88.4% | 657.5## | 99.1 |
| ADC-7,3mpk | 211.6 | 13.5 | 545.8***### | 68.1 | 80.5% | 1209.8## | 195.8 |
| vs. control group: *** p < 0.001; vs. corresponding dose groups of ADC-6: ## p < 0.01, ### p < 0.001 | | | | | | | |

**Test example 6. Evaluation of efficacy of ADC drugs on human prostate cancer cell LNCap-induced xenograft tumor in SCID Beighe mice**

I. Test method

Male SCID Beige mice aged 6-8 weeks (purchased from Beijing Vital River Laboratory Animal Technology Co., Ltd, certificate No. 1908120082) were used in this experiment. Housing environment: SPF grade. Mice were inoculated subcutaneously with human prostate cancer cells LNCap (ATCC). When the mean tumor volume reached 160 $mm^3$, animals were randomly grouped (D0) with 7 animals in each group, administered by intraperitoneal injection on D0 and D4 for a total of 2 times. The tumor volume and body weight were measured twice a week, and the data were recorded.

Tumor volume $V = 1/2 \times a \times b^2$, where a and b represent length and width, respectively. Relative tumor proliferation rate T/C(%) = (T - T0)/(C - C0) × ×100, where T and C are the tumor volume of animals at the end of the experiment in the treatment group and control group, respectively; T0 and C0 are the tumor volume of animals at the beginning of the experiment in the treatment group and control group, respectively.

Tumor inhibition rate TGI (%) = 1 - T/C (%).

II. Test object

ADC-9: 10 mpk;
ADC-3: 3 mpk, 10 mpk;
ADC-5: 3 mpk, 6 mpk, 10 mpk;
Blank control group: PBS buffer at pH 7.4.

III. Tumor inhibition effect of antibody ADCs

Tumors in all administration groups started to regress after two administrations on D0 and D4, and the efficacy was maintained until the experiment ended on D18. The test ADC-3 and test ADC-5 both exhibited a certain dose-dependent relationship, but there was no significant difference between them (Table 7 and FIG. 6A).

The body weight of the mice was stable in the treatment process, suggesting that each administration dose of each test antibody has no significant toxic side effects (FIG. 6B).

Table 7. Efficacy of ADCs on the LNCap-induced xenograft tumor in tumor-bearing SCID Beige mice after the administration

| Group | Mean tumor volume (**$mm^3$**) | | Mean tumor volume (**$mm^3$**) | | Tumor inhibition rate |
|---|---|---|---|---|---|
| | D0 | SEM | D18 | SEM | D18 |
| Blank control | 161.8 | 16.4 | 928.9 | 82.4 | >100% |
| ADC-9,10mpk | 161.7 | 14.5 | 17.5*** | 5.0 | >100% |
| ADC-3, 10mpk | 158.7 | 16.8 | 87.3*** | 25.3 | >100% |
| ADC-3, 3mpk | 161.1 | 19.1 | 111.9*** | 15.6 | >100% |
| ADC-5, 10mpk | 159.1 | 14.0 | 52.2*** | 14.7 | >100% |
| ADC-5, 6mpk | 160.2 | 15.9 | 127.5*** | 24.9 | >100% |
| ADC-5, 3mpk | 159.3 | 17.3 | 130.0*** | 23.2 | >100% |
| vs. control group: *** $p < 0.001$ | | | | | |

**Test example 7. Evaluation of $T_{1/2}$ of PMSAADC in SD rat**

Nine SD male rats (purchased from Zhejiang Vital River Laboratory Animal Technology Co., Ltd) were divided into three groups, with 3 animals in each group, and subjected to adaptive feeding in a 12/12 hour light/dark cycle at a constant temperature of 20-26 °C and a humidity of 40%-70%. The rats were given free access to food and water. On the day of experiment, SD male rats were separately injected with test drugs ADC-2, ADC-5 and ADC-10 via the tail vein at an administration dose of 3 mg/kg and an injection volume of 5 mL/kg.

The blood collection schedule was as follows: blood was collected from the fundus vein of rats at 5 min, 8 h, 24 h (day 2), day 3, day 5, day 8, day 11, day 15, day 22 and day 29 after the administration on day 1, 300 μL each time (equivalent to 150 μL of serum), the collected blood samples were left to stand at room temperature for half an hour to agglutinate, then centrifuged at 1000× g for 15 min at 4 °C, and the supernatant (serum) was transferred to an EP tube and immediately stored at -80 °C. The concentration of PSMA antibody ADCs in the serum of SD rats was determined by the ELISA method.

Test method:

Determination of total antibody:

1. 100 μL of 1 μg/mL goat anti-human IgG FC (abcam) was added to each well in an ELISA plate (coming) and incubated at 4 °C overnight.
2. The plate was washed three times with a wash buffer PBST (1× PBS, 0.5% tween-20 (Sangon Biotech)).
3. 200 μL of blocking solution (5% milk, Beyotime) was added to each well and incubated at 37 °C for 1-3 h.
4. The plate was washed three times with a wash buffer PBST.
5. 100 μL of standard sample, quality control sample and test sample was added, and incubated at 37 °C for 1-3 h.
6. The plate was washed three times with a wash buffer.
7. 100 μL of rat pre-adsorbed goat anti-human IgG light/heavy chain HRP (1:5000, abcam) was added and incubated at 37 °C for 1-1.5 h.
8. The plate was washed three times with a wash buffer.
9. 100 μL of TMB (KPL) was added and incubated at normal temperature for 10 min away from the light.
10. 100 μL of 1 M dilute sulfuric acid (SinoPharm) was added to stop the incubation, and the plate was read at a wavelength of 450 nm (molecular device, flexstation 3).

Determination of intact ADC:

1. 100 μL of 1 μg/mL goat anti-mouse IgG Fc was added to each well in an ELISA plate and incubated at 4 °C overnight.
2. The plate was washed three times with a wash buffer PBST.
3. 200 μL of blocking solution was added to each well and incubated at 37 °C for 1-3 h.
4. The plate was washed three times with a wash buffer.
5. 100 μL of 0.2 μg/mL antitoxin antibody or anti-pab-mmae (Immune Biotech, s-497-8) was added to each well and incubated at 37 °C for 1-1.5 h.
6. The plate was washed three times with a wash buffer.
7. 100 μL of standard sample, quality control sample and test sample was added, and incubated at 37 °C for 1-3 h.
8. The plate was washed three times with a wash buffer.
9. 100 μL of rat pre-adsorbed goat anti-human IgG light/heavy chain HRP (1:5000) was added and incubated at 37 °C for 1-1.5 h.
10. The plate was washed three times with a wash buffer.
11. 100 μL of TMB was added and incubated at normal temperature for 10 min away from the light.
12. 100 μL of 1 M dilute sulfuric acid was added to stop the incubation, and the plate was read at a wavelength of 450 nm.

Determination and analysis results:

The concentration of PSMA antibody ADCs in serum was determined by ELISA for PK analysis. The results are shown in Table 8.

Table 8. $T_{1/2}$ of PSMA antibody conjugates in SD rats

| Test drug | Analyte | Route of administration | $T_{1/2}$ (mean ± SD, h) |
|---|---|---|---|
| ADC-10 | Total antibody | IV (3 mg/kg ) | 219.8±18.8 |
| | Intact ADC | | 76.7± 8.7 |

(continued)

| Test drug | Analyte | Route of administration | $T_{1/2}$ (mean ± SD, h) |
|---|---|---|---|
| ADC-2 | Total antibody | IV (3 mg/kg ) | 171.2±15.6 |
| | Intact ADC | | 153.9±14.2 |
| ADC-5 | Total antibody | IV (3 mg/kg ) | 172.5±5.8 |
| | Intact ADC | | 137.9±5.3 |

The results show that after intravenous administration of the test antibody conjugates ADC-10, ADC-2 and ADC-5 at 3 mg/kg to rats, the half-life values of their total antibodies (conjugated antibody in ADC and free antibody in serum) in rats are about 219.8 h (9.2 days), 171.2 h (7.1 days), and 172.5 h (7.2 days), respectively; the half-life values of their intact ADCs in rats are about 76.7 h (3.2 days), 153.9 h (6.4 days), and 137.9 h (5.7 days), respectively. $T_{1/2}$ values of ADC-2 and ADC-5 are superior to that of ADC-10.

**Test example 8. Stability study of PSMAADCs (free toxin detection)**

1. Stability of ADC-2 in rat pharmacokinetic samples

The release amount of compound 2-B (free toxin of 9-A) in male rats over time within 28 days after intravenous administration of the sample was monitored by LC-MS, to reflect the stability of the sample at a specific concentration. ADC-2 showed good stability according to the determination of the content of compound 2-B in rat plasma at different time points (5 min, 8 h, day 1, day 2, day 4, day 7, day 10, day 14, day 21, day 28) within 28 days. The results are shown in Table 9.

The results show that ADC-2 always shows good stability in male rats within 28 days.

Table 9. Stability study of PSMAADCs

| Compound 2-B (ADC-2-rat-i.v.-3mpk, M) | | | |
|---|---|---|---|
| Time | M1 | M2 | M3 |
| 0.0 | 0 | 0 | 0 |
| 5 min | blq | blq | blq |
| 8h | blq | blq | blq |
| Day 1 | blq | blq | blq |
| Day 2 | blq | blq | blq |
| Day 4 | blq | blq | blq |
| Day 7 | blq | blq | blq |
| Day 10 | blq | blq | blq |
| Day 14 | blq | blq | blq |
| Day 21 | blq | blq | blq |
| Day 28 | blq | blq | blq |
| Blq indicates 1 ng/mL; M indicates male rats. | | | |

2. Plasma stability study of PSMA ADCs

The release amount of compound 2-B in the sample ADC-2 (100 μg/mL) over time in plasma (heparin anticoagulation) of five species (human, monkey, dog, rat, and mouse, with 1% BSA-PBS used as a control) within different time periods (incubated in a 37 °C & 5% $CO_2$ incubator for 0 day, 7 days, 14 days and 21 days) was monitored by mass spectrometry, to reflect the stability of the sample at a specific concentration (100 μg/mL). ADC-2 showed good stability according to the determination of the content of compound 2-B at different time points. The results are shown in FIG. 7 and Table 10. The results show that ADC-2 shows good stability in plasma of different species at a certain temperature

within a certain time period.

Note: the test was performed in a sterile laboratory, and the blank plasma was sterilized by filtration through a 0.22 μm microporous filter membrane.

Table 10. Content of compound 2-B (ng/mL)

| | BSA | Rat | Mouse | Human | Dog | Monkey |
|---|---|---|---|---|---|---|
| 0d-1 | BLQ | BLQ | BLQ | BLQ | BLQ | BLQ |
| 0d-2 | BLQ | BLQ | BLQ | BLQ | BLQ | BLQ |
| 7d-1 | 1.83 | 10.7 | 11.7 | 2.15 | 20.2 | 3.67 |
| 7d-2 | BLQ | 9.52 | 12.9 | 3.51 | 18.2 | 3.95 |
| 14d-1 | 1.46 | 9.60 | 15.1 | 2.19 | 21.7 | 3.12 |
| 14d-2 | 3.74 | 9.61 | 19.2 | 3.92 | 23.0 | 3.59 |
| 21d-1 | 3.51 | 19.8 | 18.3 | 5.78 | 26.0 | 6.54 |
| 21d-2 | 4.15 | 17.3 | 16.9 | 4.71 | 27.7 | 5.61 |

The linear range of the compound 2-B is 1-5000 ng/mL; BLQ denotes 1 ng/mL. "-1" and "-2" indicate the first and second repetitions of the experiment.

3. Plasma stability study of PSMA ADCs

The release amount of compound 2-B in the sample ADC-5 over time in plasma (heparin anticoagulation) of five species (human, monkey, dog, rat, and mouse, with 1% BSA-PBS used as a control) within different time periods (incubated in a 37 °C & 5% $CO_2$ incubator for 0 day, 7 days, 14 days and 23 days) was monitored by mass spectrometry, to reflect the stability of the sample at a specific concentration (100 μg/mL). ADC-5 showed good stability according to the determination of the content of compound 2-B at different time points. The results are shown in Table 11 and FIG. 8. The results show that ADC-5 shows good stability in plasma of different species at a certain temperature within a certain time period.

Note: the test was performed in a sterile laboratory, and the blank plasma was sterilized by filtration through a 0.22 μm microporous filter membrane.

Table 11. Content of compound 2-B (ng/mL)-ADC-5 (100 μg/mL) incubated in plasma of different species at 37 °C for 23 days

| | **BSA** | **Rat** | **Mouse** | **Human** | **Dog** | **Monkey** |
|---|---|---|---|---|---|---|
| **0d-1** | BLQ | BLQ | BLQ | BLQ | BLQ | BLQ |
| **0d-2** | BLQ | BLQ | BLQ | BLQ | BLQ | BLQ |
| **7d-1** | BLQ | 3.74 | 4.69 | 2.46 | 7.14 | BLQ |
| **7d-2** | BLQ | 3.82 | 3.88 | BLQ | 5.89 | BLQ |
| **14d-1** | BLQ | 10.1 | 9.32 | BLQ | 14.2 | 5.09 |
| **14d-2** | BLQ | 10.0 | 10.6 | 2.00 | 13.8 | 6.34 |
| **23d-1** | 3.32 | 14.9 | 16.5 | 8.57 | 23.1 | 9.11 |

Only a single well was set for 23d. The linear range of the compound 2-B is 2-5000 ng/mL; BLQ denotes 1 ng/mL.

**Test example 9. Evaluation of efficacy of ADC drugs on human prostate cancer cell 22Rv1-induced xenograft tumor in nude mice**

I. Test method

Male nu/nu nude mice aged 6-8 weeks (purchased from Beijing Vital River Laboratory Animal Technology Co.,

Ltd, certificate No. 1908120082) were used in this experiment. Housing environment: SPF grade. Nude mice were inoculated subcutaneously with human prostate cancer cells 22Rv1 (Chinese Academy of Sciences). When the mean tumor volume reached 190 $mm^3$, animals were randomly grouped (D0) with 8 animals in each group, administered by intraperitoneal injection twice a week for a total of 4 times. The tumor volume and body weight were measured twice a week, and the data were recorded.

The tumor volume (V) was calculated as follows: $V = 1/2 \times a \times b^2$, where a and b represent length and width, respectively.

Relative tumor proliferation rate $T/C(\%) = (T - T_0)/(C - C_0) \times 100$, where T and C are the tumor volume of animals at the end of the experiment in the treatment group and control group, respectively; $T_0$ and $C_0$ are the tumor volume of animals at the beginning of the experiment in the treatment group and control group, respectively.

$$\text{Tumor inhibition rate TGI } (\%) = 1 - T/C\ (\%).$$

II. Test object

ADC-11: 5 mpk
ADC-12: 5 mpk
Blank control group: PBS buffer at pH 7.4

III. Tumor inhibition effect of antibody ADCs

On day 13 (D13) after the treatment, it was observed that the tumor inhibition rates of ADC-11 and ADC-12 at 5 mpk were 87.63% and 79.82%, respectively. They were both significantly superior to the control group. There was no significant difference between ADC-11 and ADC-12 (Table 12 and FIG. 9).

The body weight of the mice was stable in the treatment process, suggesting that each test antibody has no significant toxic side effects.

Table 12. Efficacy of ADCs on the 22Rv1-induced xenograft tumor in tumor-bearing nude mice after the administration

| Group | Mean tumor volume ($mm^3$) | | Mean tumor volume (mm3) | | Tumor inhibition rate |
|---|---|---|---|---|---|
| | D0 | SEM | D13 | SEM | D17 |
| Blank control | 189.4 | 12.8 | 1429 | 125.8 | - |
| ADC-11, 5mpk | 187.3 | 11.5 | 340.7*** | 63.2 | 87.63% |
| ADC-12, 5mpk | 186.7 | 12 | 436.8*** | 68.6 | 79.82% |
| vs. blank control: ** p < 0.01, *** p < 0.001 | | | | | |

**Claims**

**1.** An antibody-drug conjugate of general formula (Pc-L-Y-D) or a pharmaceutically acceptable salt thereof:

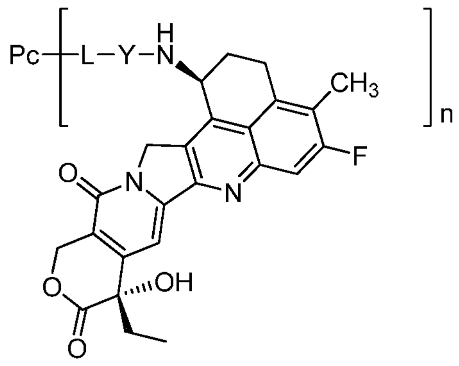

(Pc-L-Y-D)

wherein:

Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$ and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl;

or, $R^a$ and $R^b$, together with carbon atoms connected thereto, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl;

$R^2$ is selected from the group consisting of hydrogen, halogen, haloalkyl, deuterated alkyl, cycloalkyl, cycloalkylalkyl, alkoxyalkyl, heterocyclyl, aryl and heteroaryl;

or, $R^1$ and $R^2$, together with carbon atoms connected thereto, form cycloalkyl or heterocyclyl;

or, $R^a$ and $R^2$, together with carbon atoms connected thereto, form cycloalkyl or heterocyclyl;

m is an integer from 0 to 4;

n is a decimal or an integer from 1 to 10;

L is a linker unit;

Pc is an anti-PSMA antibody or an antigen-binding fragment thereof.

**2.** The antibody-drug conjugate of general formula (Pc-L-Y-D) or the pharmaceutically acceptable salt thereof according to claim 1, wherein the anti-PSMA antibody or the antigen-binding fragment thereof comprises a heavy chain variable region and a light chain variable region, wherein:
the heavy chain variable region comprises an HCDR1, an HCDR2 and an HCDR3 set forth in SEQ ID NO: 3, SEQ ID NO: 4 and SEQ ID NO: 5, respectively, and the light chain variable region comprises an LCDR1, an LCDR2 and an LCDR3 set forth in SEQ ID NO: 6, SEQ ID NO: 7 and SEQ ID NO: 8, respectively.

**3.** The antibody-drug conjugate of general formula (Pc-L-Y-D) or the pharmaceutically acceptable salt thereof according to any one of claim 1 or 2, wherein the anti-PSMA antibody is a murine antibody, a chimeric antibody, a humanized antibody or a human antibody.

**4.** The antibody-drug conjugate of general formula (Pc-L-Y-D) or the pharmaceutically acceptable salt thereof according to any one of claims 1 to 3, wherein the anti-PSMA antibody or the antigen-binding fragment thereof comprises a heavy chain variable region set forth in SEQ ID NO: 1 and a light chain variable region set forth in SEQ ID NO: 2.

**5.** The antibody-drug conjugate of general formula (Pc-L-Y-D) or the pharmaceutically acceptable salt thereof according to any one of claims 1 to 4, wherein the anti-PSMA antibody comprises a heavy chain constant region and a light chain constant region of the antibody; preferably, the heavy chain constant region is selected from the group consisting of constant regions of human IgG1, IgG2, IgG3 and IgG4 and conventional variants thereof, and the light chain constant region is selected from the group consisting of constant regions of human antibody κ and λ chains and conventional variants thereof; more preferably, the anti-PSMA antibody comprises a heavy chain set forth in SEQ ID NO: 9 and a light chain set forth in SEQ ID NO: 10.

**6.** The antibody-drug conjugate of general formula (Pc-L-Y-D) or the pharmaceutically acceptable salt thereof according to any one of claims 1 to 5, wherein n is a decimal or an integer from 1 to 8, preferably from 3 to 8.

7. The antibody-drug conjugate of general formula (Pc-L-Y-D) or the pharmaceutically acceptable salt thereof according to any one of claims 1 to 6, wherein:

Y is -O-$(CR^aR^b)_m$-$CR^1R^2$-C(O)-;
$R^a$ and $R^b$ are identical or different and are each independently selected from the group consisting of hydrogen, deuterium, halogen and $C_{1-6}$ alkyl;
$R^1$ is haloalkyl or $C_{3-6}$ cycloalkyl;
$R^2$ is selected from the group consisting of hydrogen, haloalkyl and $C_{3-6}$ cycloalkyl;
or, $R^1$ and $R^2$, together with carbon atoms connected thereto, form $C_{3-6}$ cycloalkyl;
m is 0 or 1.

8. The antibody-drug conjugate of general formula (Pc-L-Y-D) or the pharmaceutically acceptable salt thereof according to any one of claims 1 to 7, wherein Y is selected from the group consisting of:

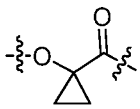, 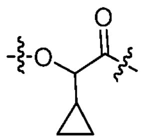, 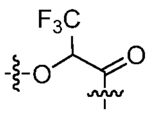, 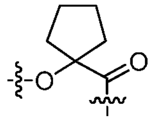, 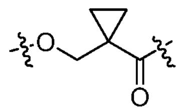, 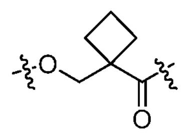

and

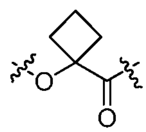;

wherein an O-terminus of Y is connected to the linker unit L.

9. The antibody-drug conjugate of general formula (Pc-L-Y-D) or the pharmaceutically acceptable salt thereof according to any one of claims 1 to 8, wherein the linker unit -L-is -$L^1$-$L^2$-$L^3$-$L^4$-. wherein

$L^1$ is selected from the group consisting of -(succinimidyl-3-yl-*N*)-W-C(O)-, -$CH_2$-C(O)-$NR^3$-W-C(O)- and -C(O)-W-C(O)-, wherein W is selected from the group consisting of $C_{1-8}$ alkyl, $C_{1-8}$ alkyl-cycloalkyl and linear heteroalkyl of 1 to 8 atoms, the heteroalkyl comprising 1 to 3 heteroatoms selected from the group consisting of N, O and S, wherein the $C_{1-8}$ alkyl, Ci-s alkyl-cycloalkyl and linear heteroalkyl are each independently and optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of -$NR^4(CH_2CH_2O)p^1CH_2CH_2C(O)$-, -$NR^4(CH_2CH_2O)p^1CH_2C(O)$-, -$S(CH_2)p^1C(O)$- and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids of phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$L^4$ is selected from the group consisting of -$NR^5(CR^6R^7)_t$-, -C(O)$NR^5$, -C(O)$NR^5(CH_2)_t$- and a chemical bond, wherein t is an integer from 1 to 6;
$R^3$, $R^4$ and $R^5$ are identical or different and are each independently selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxyalkyl.

10. The antibody-drug conjugate of general formula (Pc-L-Y-D) or the pharmaceutically acceptable salt thereof according to any one of claims 1 to 9, wherein the linker unit -L- is -$L^1$-$L^2$-$L^3$-$L^4$-, wherein

$L^1$ is

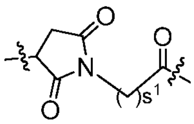

,

and $s^1$ is an integer from 2 to 8;
$L^2$ is a chemical bond;
$L^3$ is a tetrapeptide residue; preferably $L^3$ is a tetrapeptide residue of GGFG;
$L^4$ is $-NR^5(CR^6R^7)t-$, wherein $R^5$, $R^6$ and $R^7$ are identical or different and are each independently hydrogen or alkyl, and t is 1 or 2;
wherein the $L^1$ terminus is connected to Pc, and the $L^4$ terminus is connected to Y.

**11.** The antibody-drug conjugate of general formula (Pc-L-Y-D) or the pharmaceutically acceptable salt thereof according to any one of claims 1 to 10, wherein -L- is:

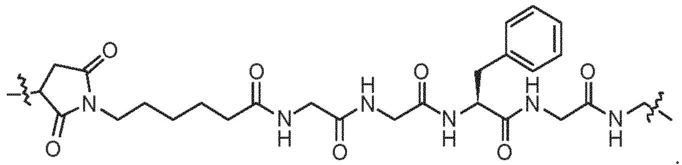

.

**12.** The antibody-drug conjugate of general formula (Pc-L-Y-D) or the pharmaceutically acceptable salt thereof according to any one of claims 1 to 11, wherein -L-Y- is optionally selected from the group consisting of:

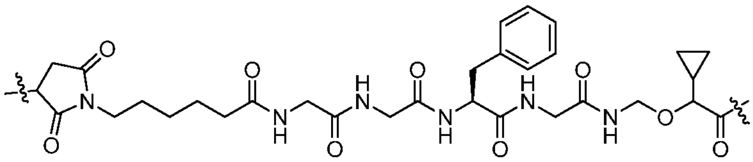

,

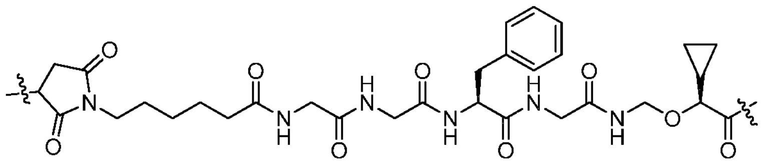

and

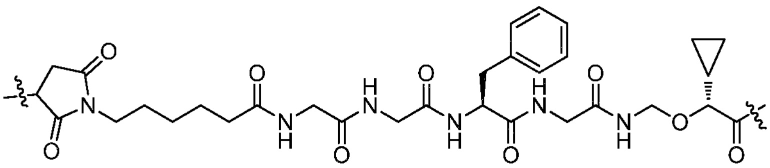

.

**13.** The antibody-drug conjugate of general formula (Pc-L-Y-D) or the pharmaceutically acceptable salt thereof according to any one of claims 1 to 10, being an antibody-drug conjugate of general formula ($Pc-L_a-Y-D$) or a pharmaceutically acceptable salt thereof:

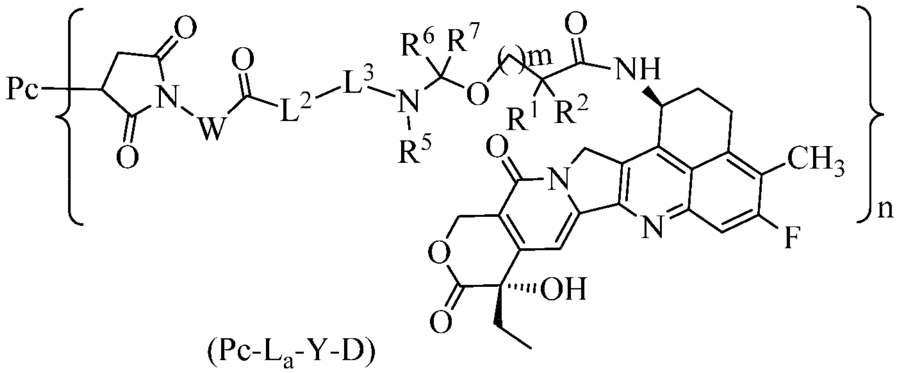

(Pc-$L_a$-Y-D)

wherein,

Pc is an anti-PSMA antibody or an antigen-binding fragment thereof;
m is an integer from 0 to 4;
n is a decimal or an integer from 1 to 10;
$R^1$ is selected from the group consisting of halogen, haloalkyl, deuterated alkyl, cycloalkyl, cycloalkylalkyl, alkoxyalkyl, heterocyclyl, aryl and heteroaryl;
$R^2$ is selected from the group consisting of hydrogen, halogen, haloalkyl, deuterated alkyl, cycloalkyl, cycloalkylalkyl, alkoxyalkyl, heterocyclyl, aryl and heteroaryl;
or, $R^1$ and $R^2$, together with carbon atoms connected thereto, form cycloalkyl or heterocyclyl;
W is selected from the group consisting of $C_{1-8}$ alkyl, Ci-s alkyl-cycloalkyl and linear heteroalkyl of 1 to 8 atoms, the heteroalkyl comprising 1 to 3 heteroatoms selected from the group consisting of N, O and S, wherein the $C_{1-8}$ alkyl, Ci-s alkyl-cycloalkyl and linear heteroalkyl are each independently and optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids of phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$R^5$ is selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxy alkyl.

**14.** The antibody-drug conjugate of general formula (Pc-L-Y-D) or the pharmaceutically acceptable salt thereof according to any one of claims 1 to 10 and claim 13, being an antibody-drug conjugate of general formula (Pc-$L_b$-Y-D) or a pharmaceutically acceptable salt thereof:

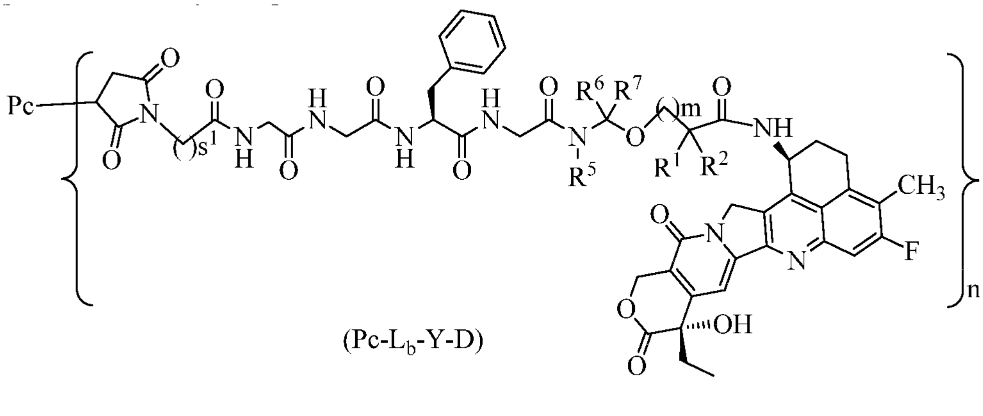

(Pc-$L_b$-Y-D)

wherein:

$s^1$ is an integer from 2 to 8;
Pc, $R^1$, $R^2$, $R^5$, $R^6$, $R^7$, m and n are as defined in claim 13.

**15.** The antibody-drug conjugate of general formula (Pc-L-Y-D) or the pharmaceutically acceptable salt thereof according to any one of claims 1 to 14, wherein the antibody-drug conjugate is selected from the group consisting of:

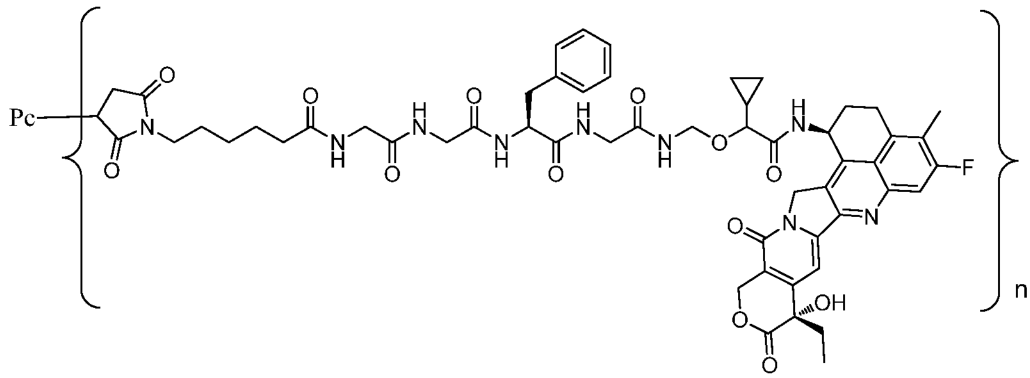

,

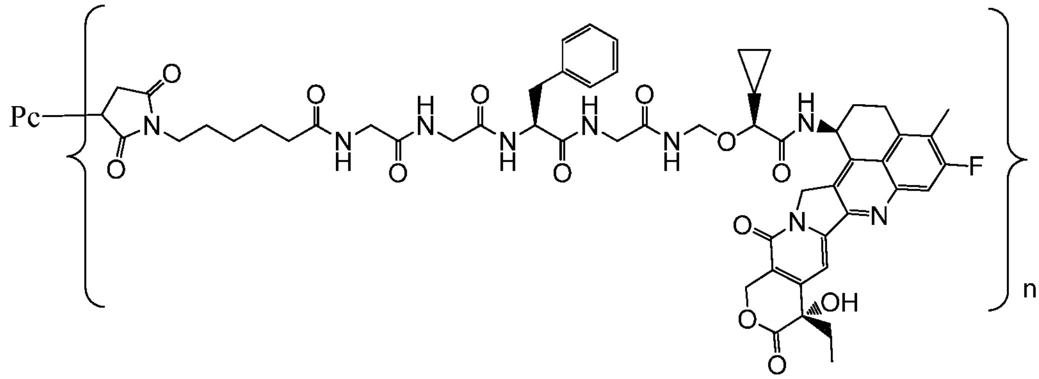

and

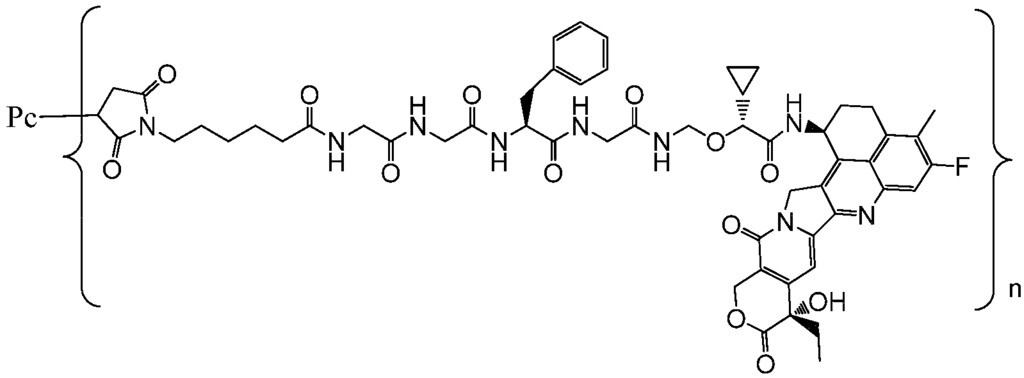

wherein,

Pc is an anti-PSMA antibody or an antigen-binding fragment thereof;
n is a decimal or an integer from 1 to 10.

**16.** The antibody-drug conjugate of general formula (Pc-L-Y-D) or the pharmaceutically acceptable salt thereof according to any one of claims 1 to 15, wherein the antibody-drug conjugate is:

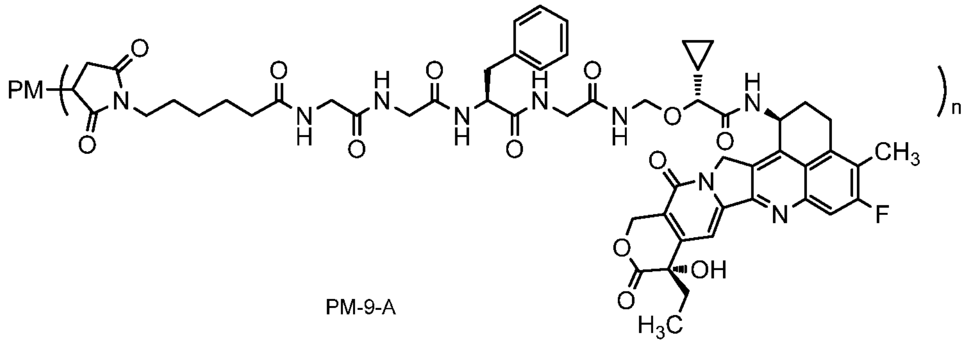

PM-9-A

wherein:

n is a decimal or an integer from 1 to 8, preferably from 3 to 8;
PM is an anti-PSMA antibody comprising a heavy chain set forth in SEQ ID NO: 9 and a light chain set forth in SEQ ID NO: 10.

**17.** A method for preparing an antibody-drug conjugate of general formula (Pc-$L_a$-Y-D) or a pharmaceutically acceptable salt thereof, comprising the following steps:

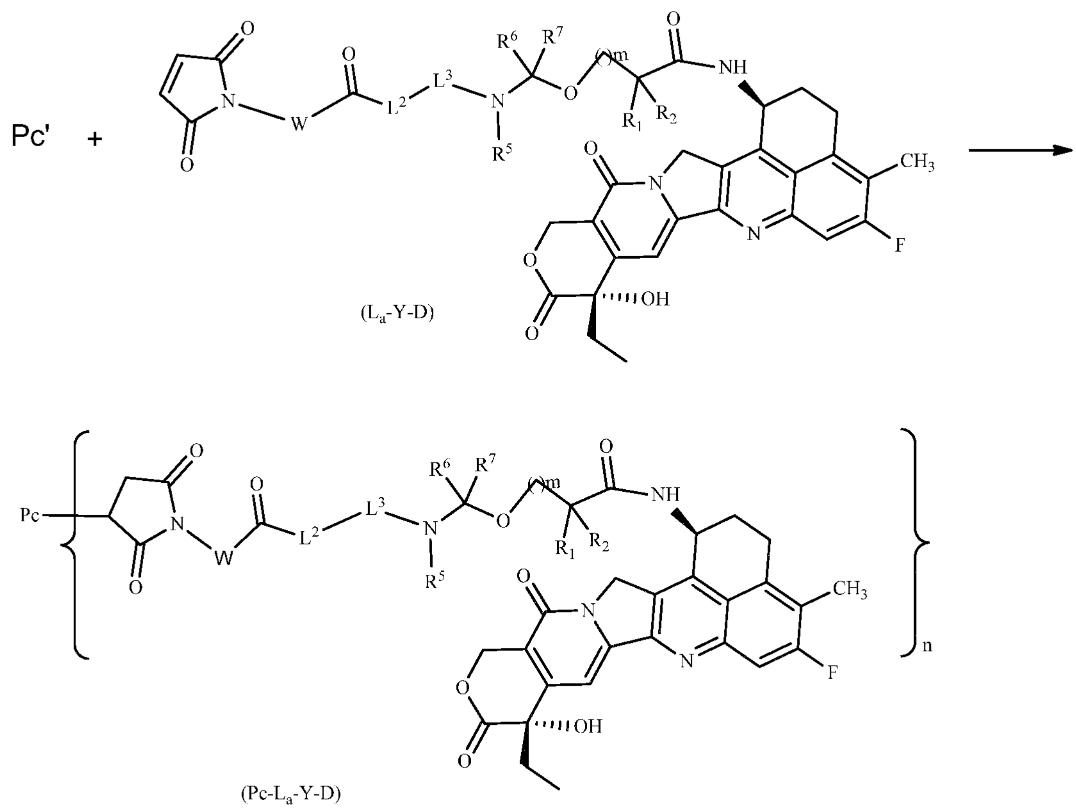

($L_a$-Y-D)

(Pc-$L_a$-Y-D)

subjecting Pc' and a compound of general formula ($L_a$-Y-D) to a coupling reaction to give a compound of general formula (Pc-$L_a$-Y-D);
wherein:

Pc is an anti-PSMA antibody or an antigen-binding fragment thereof; preferably, Pc is the anti-PSMA antibody or the antigen-binding fragment thereof according to any one of claims 2 to 5; Pc' is obtained by reduction of Pc;
n, m, W, $L^2$, $L^3$, $R^1$, $R^2$, $R^5$, $R^6$ and $R^7$ are as defined in claim 13.

**18.** A pharmaceutical composition comprising the antibody-drug conjugate or the pharmaceutically acceptable salt thereof according to any one of claims 1 to 16, and one or more pharmaceutically acceptable excipients, diluents or carriers.

**19.** Use of the antibody-drug conjugate or the pharmaceutically acceptable salt thereof according to any one of claims 1 to 16, or the pharmaceutical composition according to claim 18, in preparing a medicament for the treatment of a PSMA-mediated disease or disorder.

**20.** Use of the antibody-drug conjugate or the pharmaceutically acceptable salt thereof according to any one of claims 1 to 16, or the pharmaceutical composition according to claim 18, in preparing a medicament for the treatment and/or prevention of tumor and cancer, wherein the tumor and cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatoma, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelial carcinoma or Merkel cell carcinoma; preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma; the lung cancer is selected from the group consisting of non-small cell lung cancer and small cell lung cancer; the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

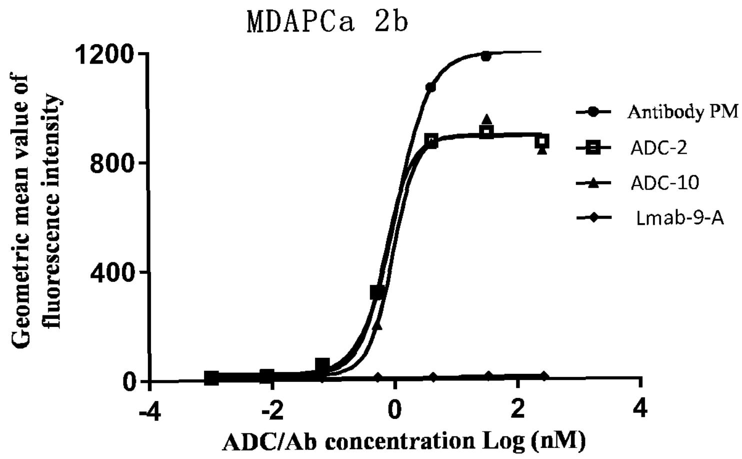


FIG. 1A

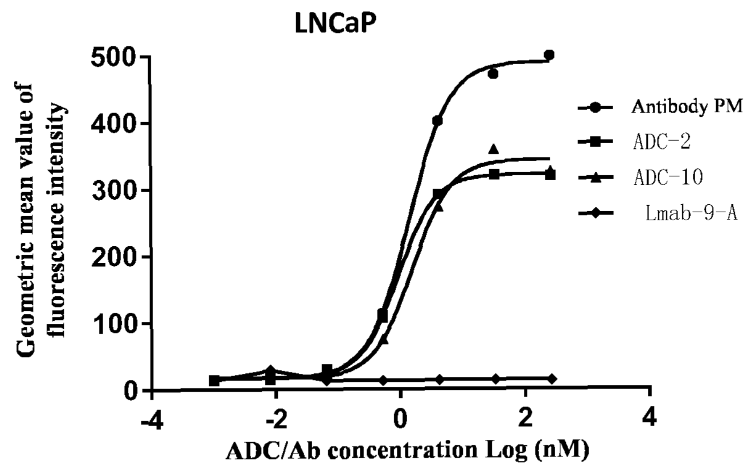


FIG. 1B

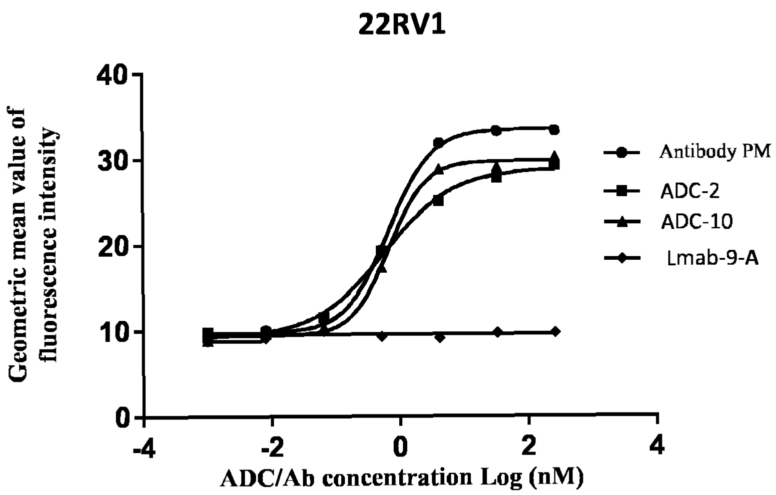


FIG. 1C

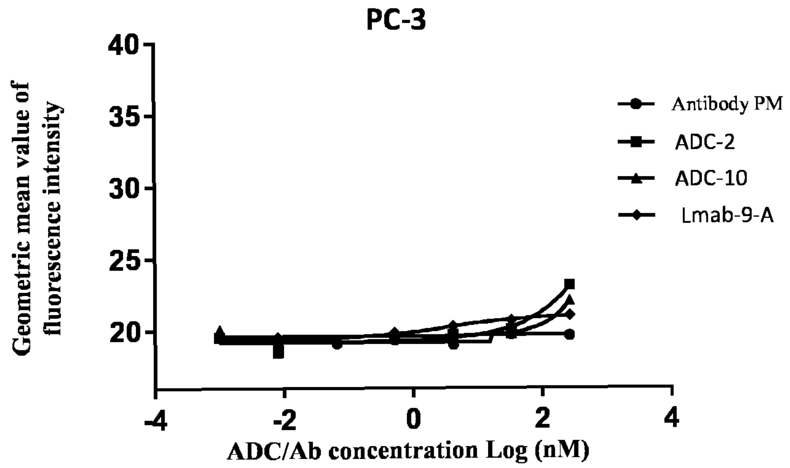


FIG. 1D

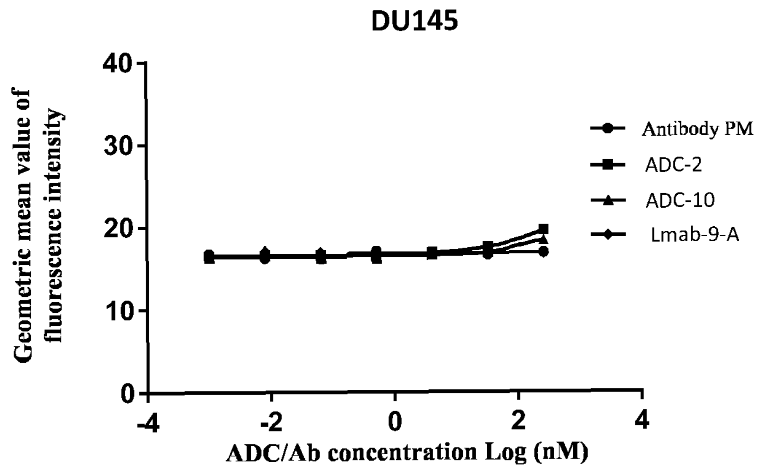


FIG. 1E

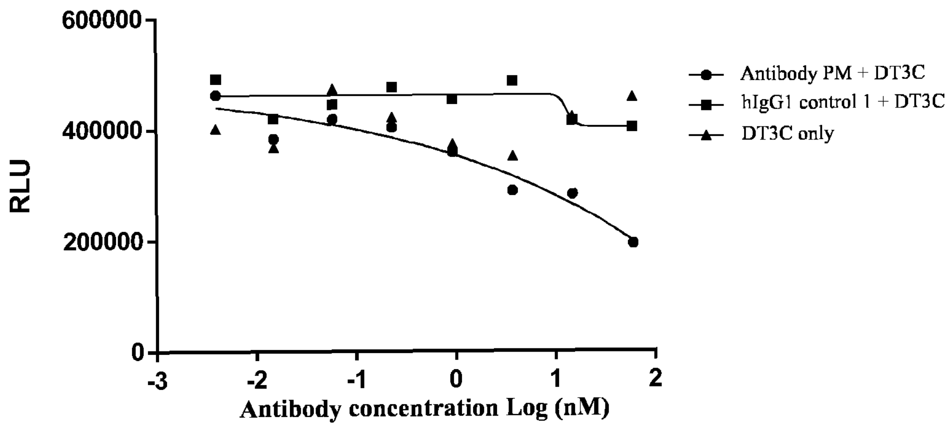


FIG. 2A

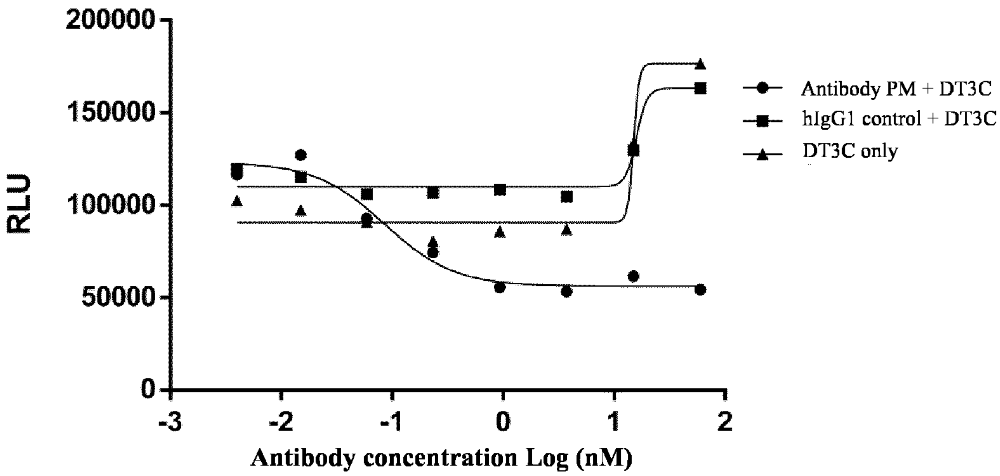


FIG. 2B

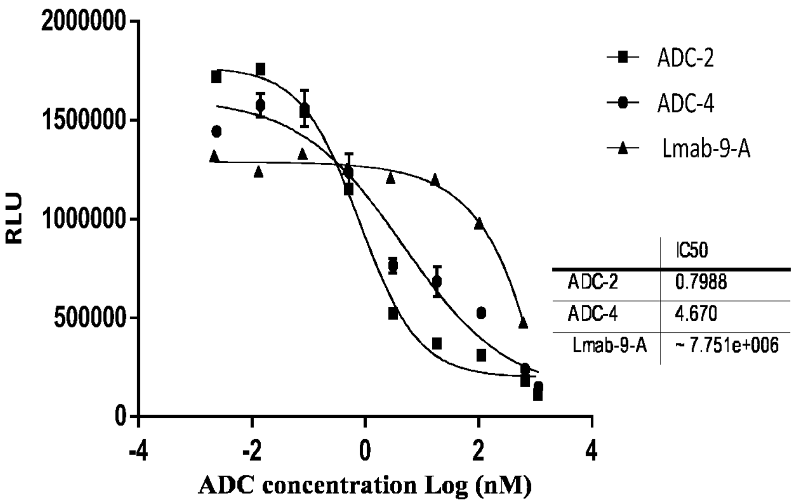


FIG. 3A

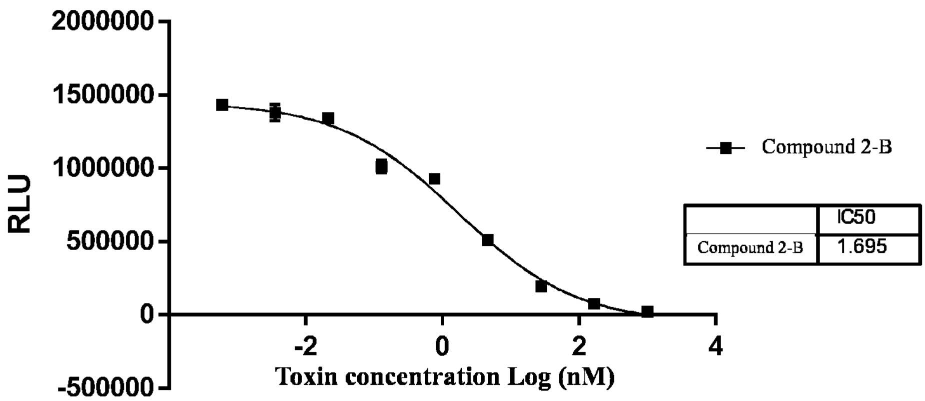


FIG. 3B

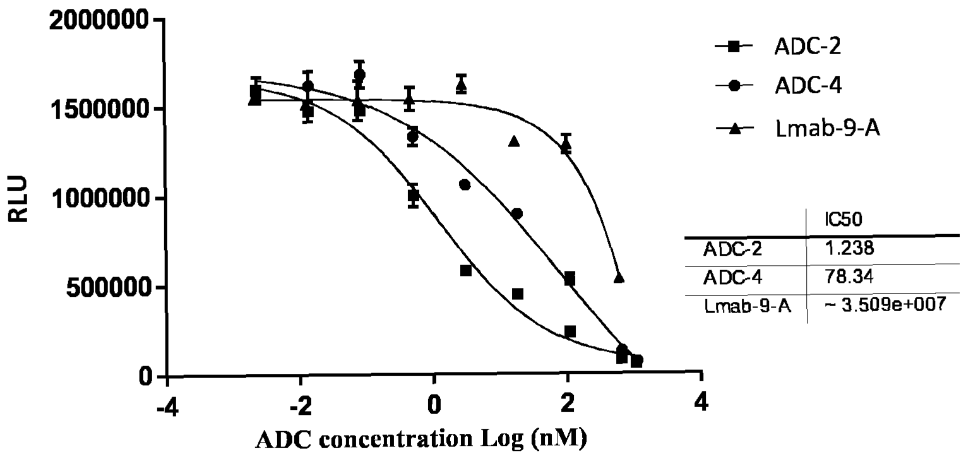


FIG. 3C

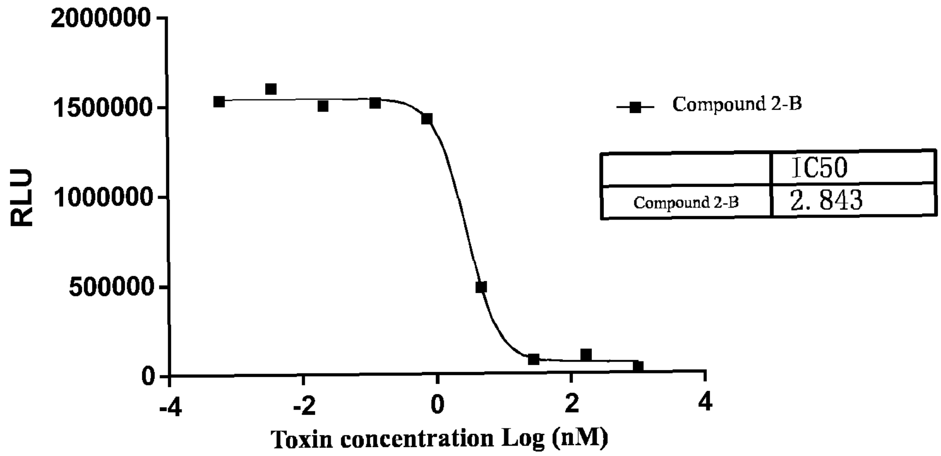


FIG. 3D

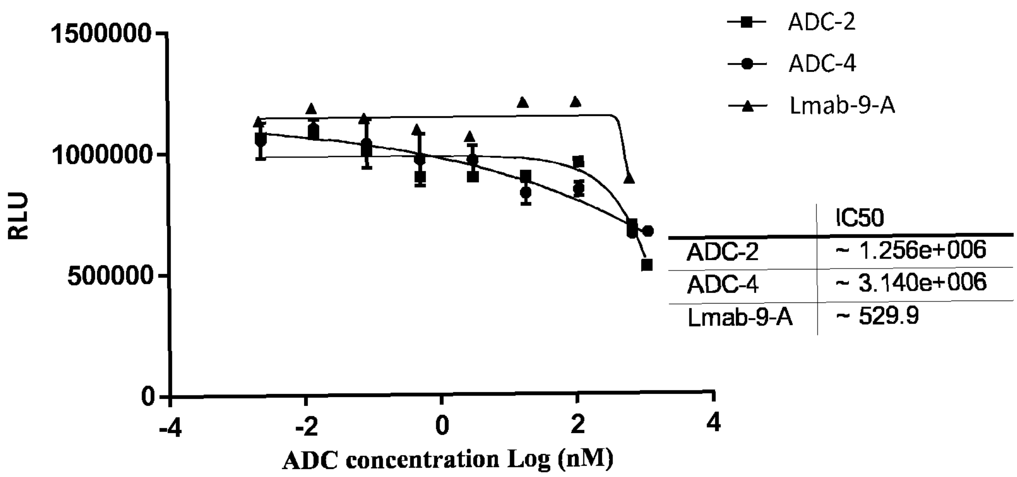


FIG. 3E

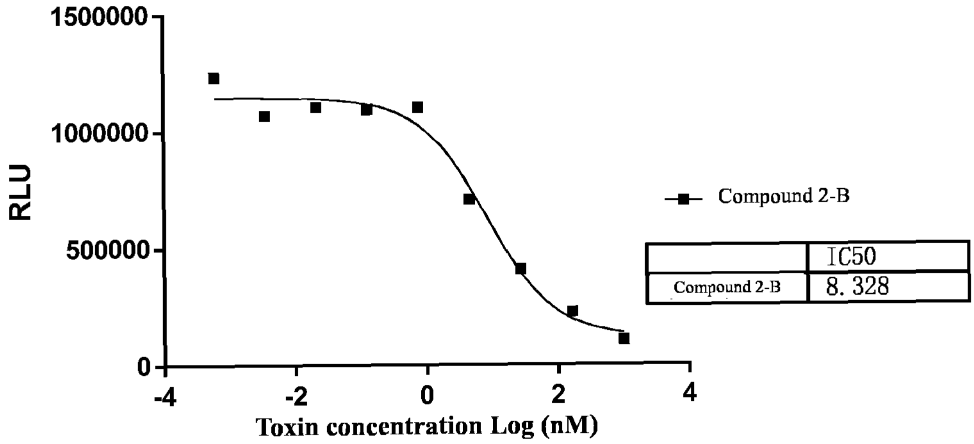


FIG. 3F

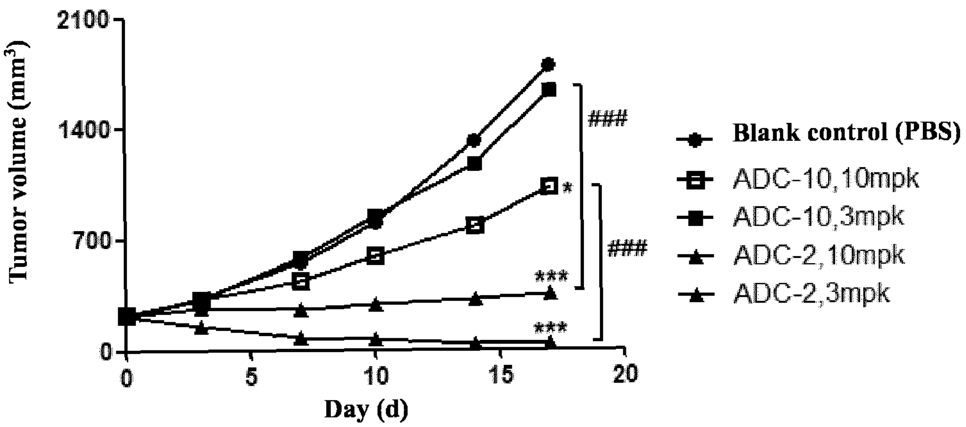


FIG. 4A

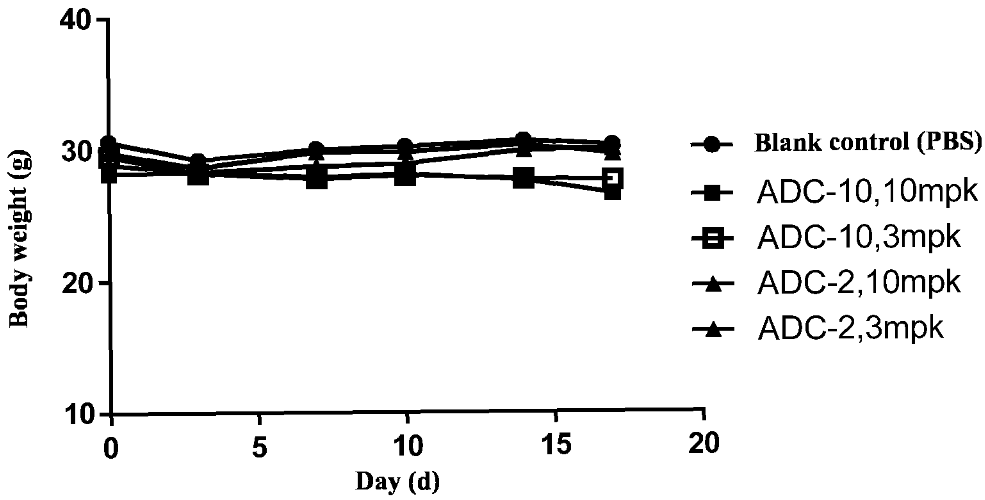


FIG. 4B

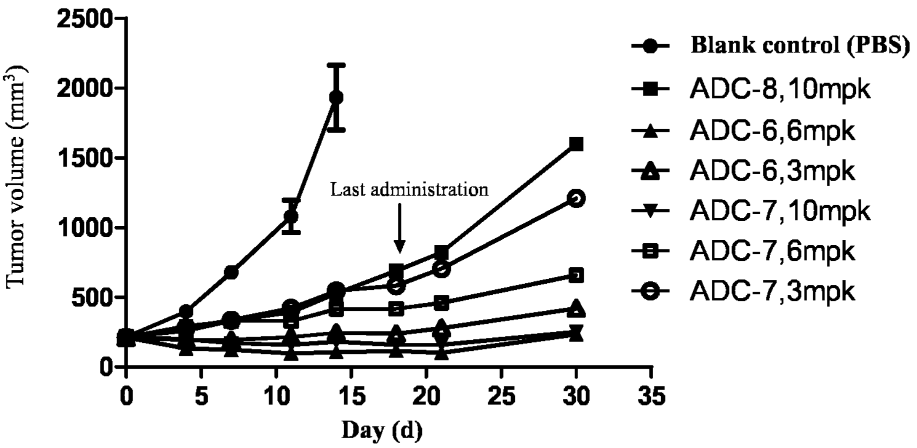


FIG. 5A

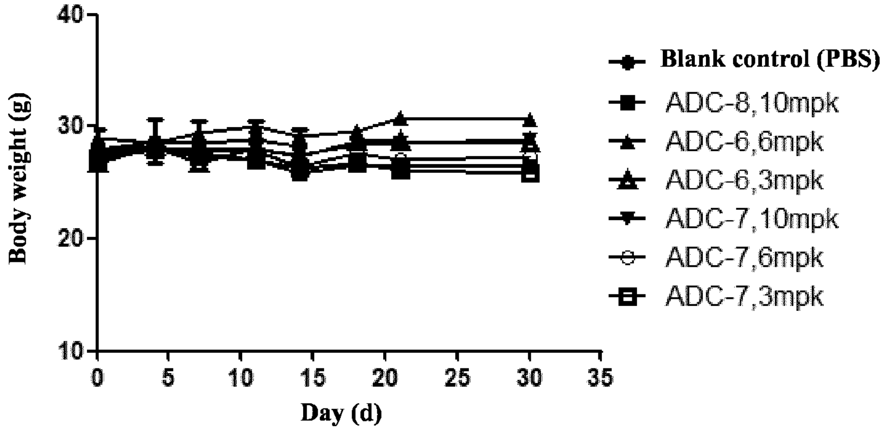


FIG. 5B

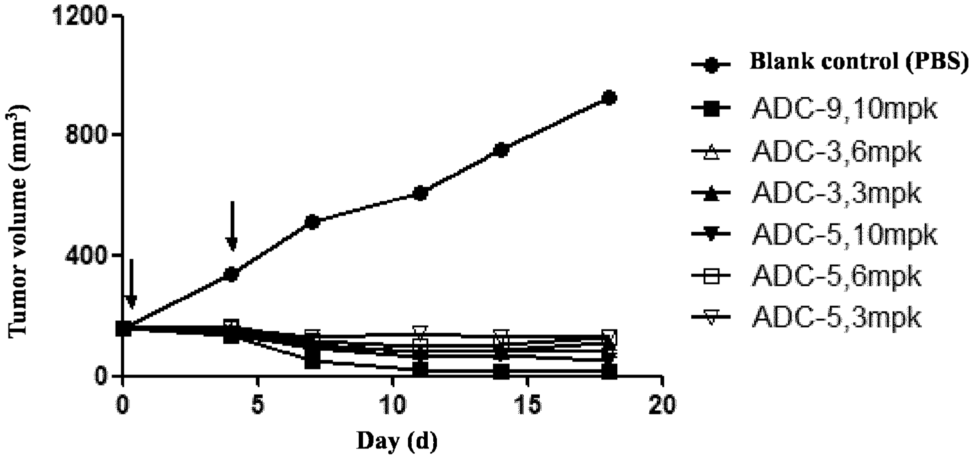


FIG. 6A

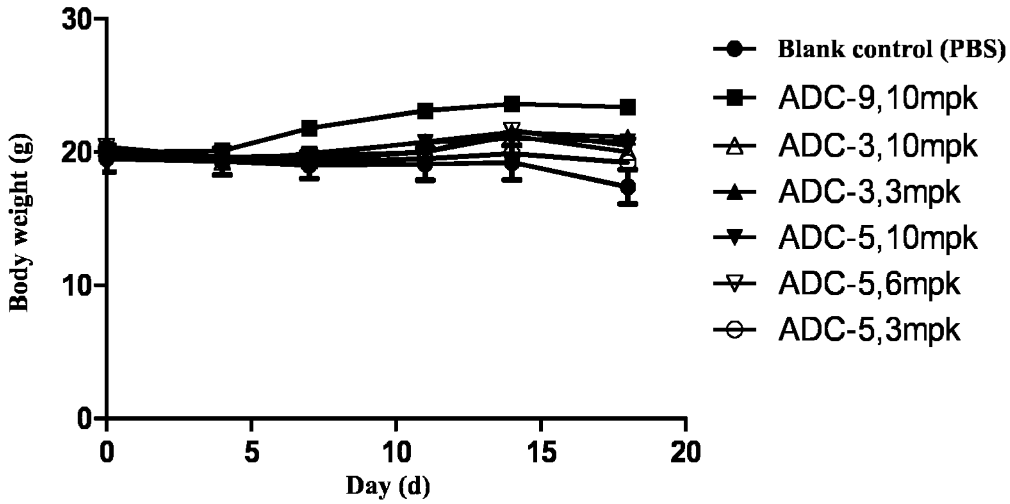
30
20
10
0
Body weight (g)
0
5
10
15
20
Day (d)
Blank control (PBS)
ADC-9,10mpk
ADC-3,10mpk
ADC-3,3mpk
ADC-5,10mpk
ADC-5,6mpk
ADC-5,3mpk

FIG. 6B

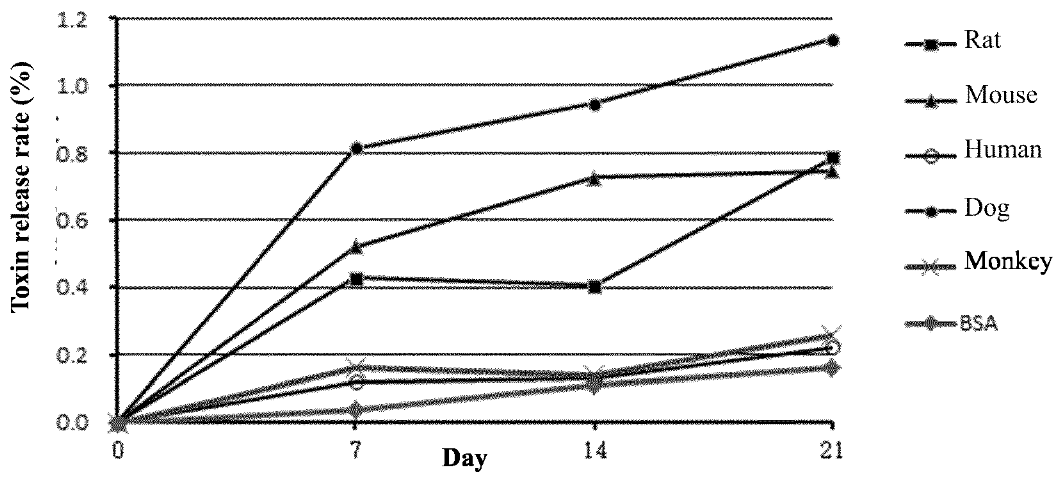
1.2
1.0
0.8
0.6
0.4
0.2
0.0
Toxin release rate (%)
0
7
14
21
Day
Rat
Mouse
Human
Dog
Monkey
BSA

FIG. 7

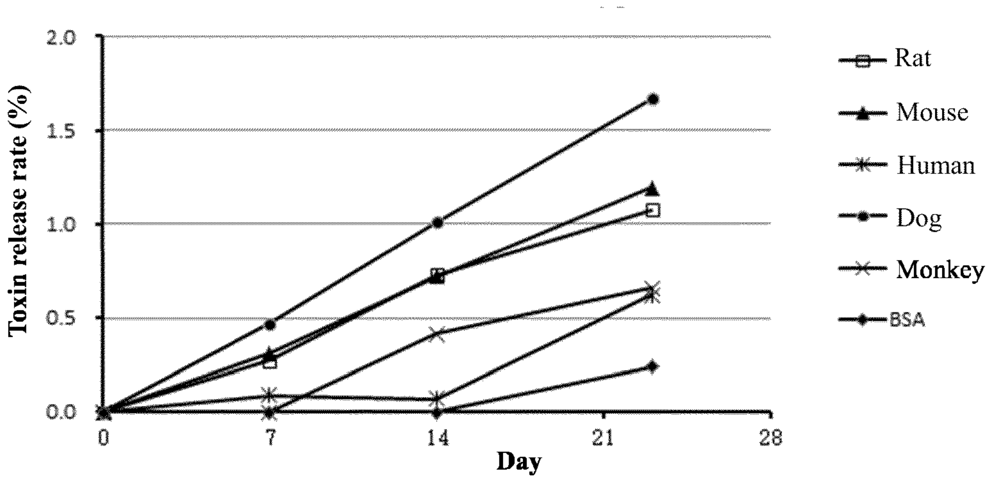
Toxin release rate (%)
2.0
1.5
1.0
0.5
0.0
0
7
14
21
28
Day
Rat
Mouse
Human
Dog
Monkey
BSA

FIG. 8

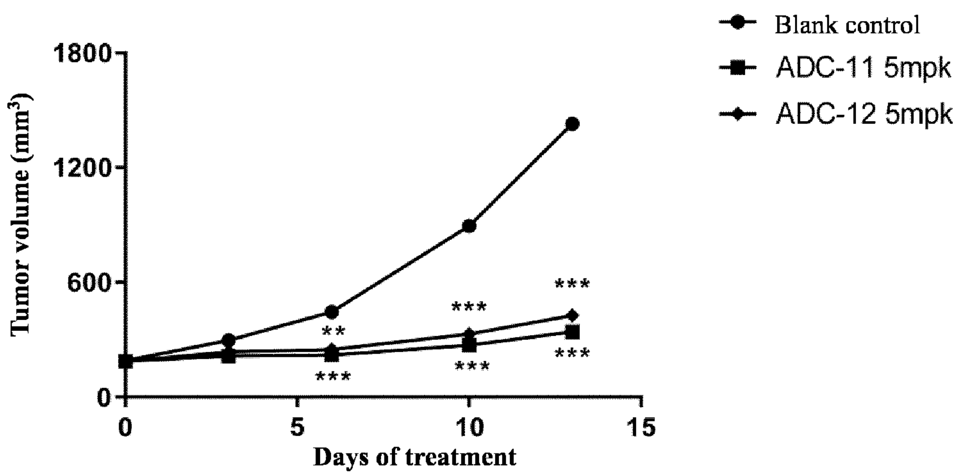
Blank control
ADC-11 5mpk
ADC-12 5mpk
Tumor volume (mm3)
1800
1200
600
0
0
5
10
15
Days of treatment
**
***

FIG. 9

**INTERNATIONAL SEARCH REPORT**

International application No.
**PCT/CN2021/082857**

**A. CLASSIFICATION OF SUBJECT MATTER**

C07D 491/22(2006.01)i; A61K 31/506(2006.01)i; A61K 47/68(2017.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNABS, CNTXT, DWPI, SIPOABS, CATXT, USTXT, EPTXT, WOTXT, CPEA, CNKI, PUBMED, WEB OF SCIENCE, ELSEVIER, 百度学术, GenBank, EMBL, 中国专利生物序列检索系统: PSMA, 抗体, anti-PSMA, 依喜替康, 依沙替康, 伊立替康, SN-38, 缀合物, 偶联物, 癌症, 肿瘤, exatecan, irinotecan, dx-8951f, cancer, tumor, carcinoma, conjugate, search based on sequences SEQ ID NOs: 1-10

**C. DOCUMENTS CONSIDERED TO BE RELEVANT**

| Category* | Citation of document, with indication, where appropriate, of the relevant passages | Relevant to claim No. |
|---|---|---|
| A | Ogitani Y et al. "DS-8201a, Anovel HER2-Targeting ADC with a Novel DNA Topoisomerase I Inhibitor, Demonstrates a Promising Antitumor Efficacy with Differentiation from T-DM1" *Clinical Cancer Research,* Vol. 22, No. 20, 29 March 2016 (2016-03-29), pp. 5097-5108 | 1-20 |
| A | Agatsuma T. "Development of New ADC Technology with Topoisomerase I Inhibitor" *YAKUGAKU ZASSHI: JOURNAL OF THE PHARMACEUTICAL SOCIETY OF JAPAN,* Vol. vol. 137, No. 5, 01 January 2017 (2017-01-01), pp. 545-550 | 1-20 |
| A | CN 107735090 A (IMMUNOMEDICS INC.) 23 February 2018 (2018-02-23) see entire document | 1-20 |
| A | US 10195175 B2 (IMMUNOMEDICS INC.) 05 February 2019 (2019-02-05) see entire document | 1-20 |
| A | CN 106999517 A (IMMUNOMEDICS INC.) 01 August 2017 (2017-08-01) see entire document | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2021** | **30 June 2021** |

Name and mailing address of the ISA/CN
**China National Intellectual Property Administration (ISA/CN)**
**No. 6, Xitucheng Road, Jimenqiao, Haidian District, Beijing 100088**
**China**
Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2021/082857**

**C. DOCUMENTS CONSIDERED TO BE RELEVANT**

| Category* | Citation of document, with indication, where appropriate, of the relevant passages | Relevant to claim No. |
|---|---|---|
| A | CN 104755494 A (DAIICHI SANKYO COMPANY, LIMITED) 01 July 2015 (2015-07-01) see entire document | 1-20 |
| PX | WO 2020063676 A1 (JIANGSU HENGRUI MEDICINE CO., LTD. et al.) 02 April 2020 (2020-04-02) see claims 1-46 | 1-20 |
| PX | CN 112125915 A (SICHUAN BAILI PHARMACEUTICAL CO., LTD.) 25 December 2020 (2020-12-25) see description paragraphs [0076]-[0154] claims 1-22 | 1-20 |

Form PCT/ISA/210 (second sheet) (January 2015)

**INTERNATIONAL SEARCH REPORT**

International application No.
**PCT/CN2021/082857**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2021/082857**

| Patent document cited in search report | | | Publication date (day/month/year) | Patent family member(s) | | | Publication date (day/month/year) |
|---|---|---|---|---|---|---|---|
| CN | 107735090 | A | 23 February 2018 | US | 2018085469 | A1 | 29 March 2018 |
| | | | | CA | 2983600 | A1 | 05 January 2017 |
| | | | | US | 9855344 | B2 | 02 January 2018 |
| | | | | KR | 20180023915 | A | 07 March 2018 |
| | | | | US | 2016256562 | A1 | 08 September 2016 |
| | | | | US | 2015306243 | A1 | 29 October 2015 |
| | | | | JP | 2018520148 | A | 26 July 2018 |
| | | | | WO | 2015012904 | A2 | 29 January 2015 |
| | | | | US | 9375489 | B2 | 28 June 2016 |
| | | | | US | 2014227180 | A1 | 14 August 2014 |
| | | | | US | 10034950 | B2 | 31 July 2018 |
| | | | | WO | 2015012904 | A3 | 07 May 2015 |
| | | | | US | 2017165370 | A1 | 15 June 2017 |
| | | | | US | 9107960 | B2 | 18 August 2015 |
| | | | | JP | 6828950 | B2 | 10 February 2021 |
| | | | | AU | 2016285853 | A1 | 16 November 2017 |
| | | | | US | 9629926 | B2 | 25 April 2017 |
| US | 10195175 | B2 | 05 February 2019 | US | 2017209594 | A1 | 27 July 2017 |
| | | | | US | 2019083451 | A1 | 21 March 2019 |
| CN | 106999517 | A | 01 August 2017 | US | 2016095939 | A1 | 07 April 2016 |
| | | | | JP | 2017536340 | A | 07 December 2017 |
| | | | | JP | 2020105187 | A | 09 July 2020 |
| | | | | AU | 2015328370 | A1 | 13 April 2017 |
| | | | | JP | 6678941 | B2 | 15 April 2020 |
| | | | | EP | 3204018 | A4 | 23 May 2018 |
| | | | | CA | 2961774 | A1 | 14 April 2016 |
| | | | | WO | 2016057398 | A1 | 14 April 2016 |
| | | | | EP | 3204018 | A1 | 16 August 2017 |
| | | | | US | 2018296689 | A1 | 18 October 2018 |
| CN | 104755494 | A | 01 July 2015 | TW | 202033499 | A | 16 September 2020 |
| | | | | HR | P20200353 | T1 | 12 June 2020 |
| | | | | LT | 2907824 | T | 11 June 2018 |
| | | | | KR | 20200026323 | A | 10 March 2020 |
| | | | | SG | 10201804788 X | A | 30 July 2018 |
| | | | | KR | 101841818 | B1 | 26 March 2018 |
| | | | | AU | 2013328111 | A1 | 12 March 2015 |
| | | | | TW | 201811746 | A | 01 April 2018 |
| | | | | TW | 201420117 | A | 01 June 2014 |
| | | | | HU | E039000 | T2 | 28 December 2018 |
| | | | | PH | 12015500667 | B1 | 18 May 2015 |
| | | | | JP | 6030267 | B1 | 24 November 2016 |
| | | | | JP | 2020180124 | A | 05 November 2020 |
| | | | | EP | 3342785 | A1 | 04 July 2018 |
| | | | | JP | 2018188455 | A | 29 November 2018 |
| | | | | CA | 2885800 | A1 | 17 April 2014 |
| | | | | HR | P20180870 | T1 | 13 July 2018 |
| | | | | MX | 2019004502 | A | 21 August 2019 |
| | | | | JP | 6715888 | B2 | 01 July 2020 |
| | | | | PL | 3342785 | T3 | 07 September 2020 |
| | | | | KR | 102087017 | B1 | 10 March 2020 |

Form PCT/ISA/210 (patent family annex) (January 2015)

**INTERNATIONAL SEARCH REPORT**
**Information on patent family members**

International application No.
**PCT/CN2021/082857**

| Patent document cited in search report | Publication date (day/month/year) | Patent family member(s) | Publication date (day/month/year) |
|---|---|---|---|
| | | ES 2773710 T3 | 14 July 2020 |
| | | LT 3342785 T | 10 February 2020 |
| | | JP 2016196484 A | 24 November 2016 |
| | | HU E048748 T2 | 28 August 2020 |
| | | MX 2015003903 A | 17 July 2015 |
| | | NZ 705394 A | 26 October 2018 |
| | | CY 1120363 T1 | 10 July 2019 |
| | | TW 201920098 A | 01 June 2019 |
| | | PL 2907824 T3 | 31 August 2018 |
| | | JP 5953378 B2 | 20 July 2016 |
| | | MX 364484 B | 29 April 2019 |
| | | AU 2013328111 B2 | 02 November 2017 |
| | | CN 104755494 B | 07 September 2018 |
| | | TW I615152 B | 21 February 2018 |
| | | DK 2907824 T3 | 23 July 2018 |
| | | EP 2907824 B1 | 11 April 2018 |
| | | KR 20190135559 A | 06 December 2019 |
| | | IL 271760 D0 | 27 February 2020 |
| | | JP 6371452 B2 | 08 August 2018 |
| | | WO 2014057687 A1 | 17 April 2014 |
| | | CA 3021435 A1 | 17 April 2014 |
| | | US 2016279259 A1 | 29 September 2016 |
| | | RU 2018128384 A | 02 October 2018 |
| | | JP 2018008982 A | 18 January 2018 |
| | | JP WO2014057687 A1 | 05 September 2016 |
| | | US 2019008981 A1 | 10 January 2019 |
| | | NZ 746440 A | 29 November 2019 |
| | | US 10195288 B2 | 05 February 2019 |
| WO 2020063676 A1 | 02 April 2020 | TW 202027795 A | 01 August 2020 |
| | | CN 112512591 A | 16 March 2021 |
| CN 112125915 A | 25 December 2020 | WO 2021052402 A1 | 25 March 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010218297
- US 20050238649 A1
- US 5208020 A
- WO 2003034903 A2
- CN 2019107873 W
- EP 0737686 A1
- WO 2013106717 A
- WO 201396771 A
- US 200520645 B
- CN 105829346 A
- EP 2907824 A
- CN 104755494 A


### Non-patent literature cited in the description


- *CA CANCER J CLIN,* 2019, vol. 69, 7-34
- *European Urology,* vol. 66 (6), 1190-1193
- *Journal of Nuclear Medicine,* vol. 59 (2), 177-182
- *Clinical Cancer Research,* January 1997, vol. 3, 81-85
- *Urologic Oncology: Seminars and Original Investigations,* vol. 1 (1), 18-28
- *Clin Cancer Res.,* 15 November 2010, vol. 16 (22), 5414-5423
- *J Nucl Med,* 2017, vol. 58, 81-84
- *Curr Med Chem.,* 2012, vol. 19 (6), 856-870
- *The EMBO Journal,* 2006, vol. 25, 1375-1384
- *Current Medicinal Chemistry,* 2012, vol. 19, 1316-1322
- *Frontiers in Bioscience, Landmark,* 01 March 2019, vol. 24, 648-687
- **CHARI et al.** *Cancer Research,* 1992, vol. 52, 127-131
- *J. biol. chem,* 1968, vol. 243, 3558
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M. P.** *Immunologist,* 1999, vol. 7, 132-136
- **LEFRANC, M. P. et al.** *Dev. Comp. Immunol.,* 2003, vol. 27, 55-77
- **KABAT E.A. et al.** Sequences of proteins of immunological interest. NIH Publication, 1991
- Epitope Mapping Protocols in Methods in Molecular Biology. 1996, vol. 66
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience
- *Drug Information,* 2016, vol. 30 (1
- *Tetrahedron Letters,* 1984, vol. 25 (12), 1269-72
- *Journal of the American Chemical Society,* 2014, vol. 136 (22), 8138-8142
- *CHEMICAL ABSTRACTS,* 646502-53-6